# EUROPÄISCHE PATENTSCHRIFT

(11) **EP 3 003 042 B1**
(45) Veröffentlichungstag und Bekanntmachung des Hinweises auf die Patenterteilung: **21.06.2017**
(21) Anmeldenummer: 14726141.6
(22) Anmeldetag: 23.05.2014
(51) Int. Cl.: A01N 43/90, C07D 487/04, A01P 7/04, A01P 5/00

(54) **HETEROCYCLISCHE VERBINDUNGEN ALS SCHÄDLINGSBEKÄMPFUNGSMITTEL**
HETEROCYCLIC COMPOUNDS AS PEST CONTROLLERS
COMPOSÉS HÉTÉROCYCLIQUES EN TANT QUE MOYEN DE LUTTE CONTRE LES PARASITES

(30) Priorität: 28.05.2013 EP 13169415
(43) Veröffentlichungstag der Anmeldung: 13.04.2016
(73) Patentinhaber: Bayer CropScience Aktiengesellschaft, 40789 Monheim am Rhein (DE)
(72) Erfinder: CEREZO-GALVEZ, Silvia, 40764 Langenfeld (DE); BRETSCHNEIDER, Thomas, 53797 Lohmar (DE); FISCHER, Reiner, 40789 Monheim (DE); FÜßLEIN, Martin, 40225 Düsseldorf (DE); GRONDAL, Christoph, 50937 Köln (DE); JESCHKE, Peter, 51467 Bergisch Gladbach (DE); REINISCH, Peter, 40764 Langenfeld (DE); GUECLUE, Mehmet, 51063 Köln (DE); ILG, Kerstin, 50670 Köln (DE); LÖSEL, Peter, 51371 Leverkusen (DE); MALSAM, Olga, 51503 Rösrath (DE); VOERSTE, Arnd, 50674 Köln (DE)
(74) Vertreter: BIP Patents
(86) Internationale Anmeldenummer: PCT/EP2014/060596
(87) Internationale Veröffentlichungsnummer: WO 2014/191301

(56) Entgegenhaltungen:
- WO-A1-2004/099211
- WO-A1-2010/100189

## Beschreibung

Die vorliegende Anmeldung betrifft die nicht therapeutische Verwendung von bekannten und neuen heterocyclischen Verbindungen zur Bekämpfung von tierischen Schädlingen, neue heterocyclische Verbindungen, Verfahren zu Ihrer Herstellung und ihre Verwendung zur Bekämpfung von tierischen Schädlingen. In WO 2012/102387 A1 sind heterocyclische Verbindungen beschrieben, die insbesondere als Insektizide und Akarizide verwendet werden können.

Heterocyclische Verbindungen für pharmazeutische Anwendungen werden in WO 2004/009597 A2, WO 2008/107418 A1, WO 2009/068617 A1 und US 2004/242596 A1 offenbart.

In WO 2004/099211 A1 werden 6-Cyclylmethyl- und 6-Alkylmethyl-substituierte Pyrazolopyrimidine für pharmazeutische Anwendungen beschrieben.

In Bioorganic & Medicinal Chemistry Letters (2007), 17(15), 4303-4307 wird über Synthese und pharmakologische Eigenschaften bestimmter Pyrazolo[3,4-d]pyrimidin-4-one berichtet.

Moderne Pflanzenschutzmittel müssen vielen Anforderungen genügen, beispielsweise in Bezug auf Höhe, Dauer und Breite ihrer Wirkung und möglichen Verwendung. Es spielen Fragen der Toxizität, der Kombinierbarkeit mit anderen Wirkstoffen oder Formulierhilfsmitteln eine Rolle sowie die Frage des Aufwands, der für die Synthese eines Wirkstoffs betrieben werden muss. Ferner können Resistenzen auftreten. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten verbreitert wird.

Gelöst wird die Aufgabe, sowie weitere nicht explizit genannte Aufgaben, die aus den hierin diskutierten Zusammenhängen ableitbar oder erschließbar sind, durch die Verwendung von Verbindungen der Formel (I) 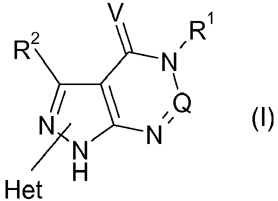 in welcher Het für einen Rest aus der Reihe 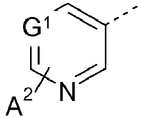 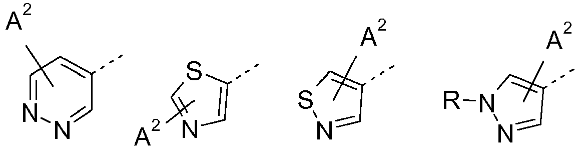 steht, worin die gestrichelte Linie die Bindung zu einem der Stickstoffatome im Pyrazolring darstellt und
- G¹: für N oder C-A¹ steht,
- A¹: für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
- A²: für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
- R: für Wasserstoff, Alkyl, Halogenalkyl oder gegebenenfalls substituiertes Cycloalkyl steht,
- R¹: für einen Rest aus der Reihe Wasserstoff, Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylsulfanylalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, Dialkylaminosulfanylalkyl, Dialkylaminosulfinylalkyl, Dialkylaminosulfonylalkyl, gegebenenfalls durch Halogen substituiertes Alkoxycarbonylalkyl, gegebenenfalls durch Halogen substituiertes Alkinyloxy, gegebenenfalls durch Halogen substituiertes Alkinyloxycarbonyl, Dialkylaminocarbonyl, N-Alkyl-N-Cycloalkylaminocarbonyl, Dialkylaminocarbonylalkyl, N-Alkyl-N-Cycloalkylaminocarbonylalkyl, Heterocyclylcarbonylalkyl, Alkylsulfanyl, Halogenalkylsulfanyl, Alkylsulfinyl, Halogenalkylsulfinyl, Alkylsulfonyl, Halogenalkylsulfonyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylcarbonyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylalkyl, gegebenenfalls substituiertes Heterocyclyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfonyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Heterocyclylalkyl, gegebenenfalls durch Halogen, Cyano, Nitro, Alkyl, Halogenalkyl, Cycloalkyl (welches substituiert sein kann), Alkoxy oder Halogenalkoxy substituiertes Aryl, gegebenenfalls durch Halogen, Cyano, Alkyl, Halogenalkyl, Cycloalkyl (welches substituiert sein kann), Alkoxy oder Halogenalkoxy substituiertes Arylalkyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfonyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetaryl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetarylalkyl steht,
- R²: für Wasserstoff oder Alkyl steht,
- Q: für Stickstoff oder C-R³ steht, worin
- R³: für einen Rest aus der Reihe Wasserstoff, Alkyl, Halogenalkyl, OH, Alkoxy, Halogenalkoxy, SH, Alkylsulfanyl, Alkylsulfinyl, Alkylsulfonyl, NH₂, Alkylamino und Dialkylamino steht,
- V: für einen Rest aus der Reihe Sauerstoff, Schwefel und NR⁴ steht und
- R⁴: für einen Rest aus der Reihe Wasserstoff, Cyano, Alkyl, Halogenalkyl und Cycloalkyl steht,
zur Bekämpfung von tierischen Schädlingen.

Je nachdem mit welchem Stickstoffatom der Rest Het verknüpft ist, lassen sich die erfindungsgemäßen Verbindungen der Formel (I) durch die Formeln (Ia) und (Ib) darstellen.

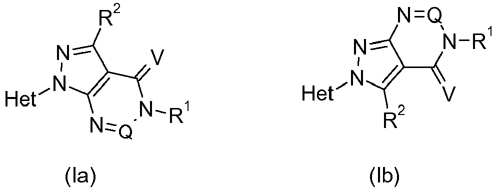

Wenn nichts anderes erwähnt ist, sind daher immer die Verbindungen der Formeln (Ia) und (Ib) gemeint, wenn von Verbindungen der Formel (I) die Rede ist.

Bevorzugte Substituenten bzw. Bereiche der in den Verbindungen der Formel (I) aufgeführten Reste werden im Folgenden erläutert.

Het steht für einen Rest aus der Reihe 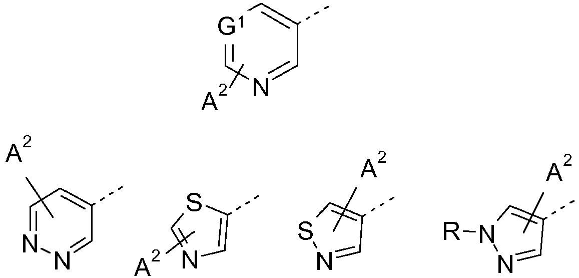 worin die gestrichelte Linie die Bindung zu einem der Stickstoffatome im Pyrazolring darstellt.
- G¹: steht für N oder C-A¹.
- A¹: steht für einen Rest aus der Reihe Wasserstoff, Halogen, C₁-C₆-Alkyl und C₁-C₄-Halogenalkyl.
- A²: steht für einen Rest aus der Reihe Wasserstoff, Halogen, C₁-C₆-Alkyl und C₁-C₄-Halogenalkyl.
- R: steht für einen Rest aus der Reihe Wasserstoff, C₁-C₆-Alkyl, C₁-C₆-Halogenalkyl und gegebenenfalls einfach bis dreifach durch Halogen, Cyano, Nitro, C₁-C₃-Alkyl, C₁-C₃-Alkoxy und einfach durch C₃-C₆-Cycloalkyl substituiertes C₃-C₆-Cycloalkyl.
- R¹: steht für einen Rest aus der Reihe Wasserstoff, C₁-C₆-Alkyl, C₂-C₆-Halogenalkyl, Cyano-C₁-C₆-alkyl, C₂-C₄-Alkenyl, C₂-C₄-Halogenalkenyl, C₂-C₄-Alkinyl, C₂-C₄-Halogenalkinyl, C₁-C₆-Alkoxy, C₁-C₆-Halogenalkoxy, gegebenenfalls durch Halogen substituiertes C₁-C₆-Alkoxy-C₁-C₆-alkyl, gegebenenfalls durch Halogen substituiertes Bis(C₁-C₆-alkoxy)-C₁-C₆-alkyl, gegebenenfalls durch Halogen substituiertes C₁-C₆-Alkylsulfanyl-C₁-C₆-alkyl, gegebenenfalls durch Halogen substituiertes C₁-C₄-Alkylcarbonyl-C₁-C₄-alkyl, gegebenenfalls durch Halogen substituiertes C₁-C₆-Alkylsulfinyl-C₁-C₆-alkyl, gegebenenfalls durch Halogen substituiertes C₁-C₆-Alkylsulfonyl-C₁-C₆-alkyl, Di-(C₁-C₆-alkyl)-aminosulfanyl-C₁-C₆-alkyl, Di-(C₁-C₆)-alkylaminosulfinyl-C₁-C₆-alkyl, Di-(C₁-C₆-alkyl)-aminosulfonyl-C₁-C₆-alkyl, gegebenenfalls durch Halogen substituiertes C₁-C₆-Alkoxycarbonyl-C₁-C₆-alkyl, gegebenenfalls durch Halogen substituiertes C₂-C₄-Alkinyloxy, gegebenenfalls durch Halogen substituiertes C₂-C₄-Alkinyloxycarbonyl, Di-(C₁-C₆-alkyl)-aminocarbonyl, N-C₁-C₆-Alkyl-N-C₃-C₆-Cycloalkylaminocarbonyl, Di-(C₁-C₆-alkyl)-aminocarbonyl-(C₁-C₆)-alkyl, N-C₁-C₆-Alkyl-N-C₃-C₆-Cycloalkylaminocarbonyl-(C₁-C₆)-alkyl, Heterocyclylcarbonyl-(C₁-C₆)-alkyl, C₁-C₆-Alkylsulfanyl, C₁-C₆-Halogenalkylsulfanyl, C₁-C₆-Alkylsulfinyl, C₁-C₆-Halogenalkylsulfinyl, C₁-C₆-Alkylsulfonyl, C₁-C₆-Halogenalkylsulfonyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkylcarbonyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl-C₁-C₆-alkyl, gegebenenfalls durch Halogen, Cyano (auch im Alkylteil), Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Heterocyclyl-C₁-C₆-alkyl, gegebenenfalls durch Halogen, Cyano, Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy oder C₁-C₆-Halogenalkoxy substituiertes Aryl, gegebenenfalls durch Halogen, Cyano, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy oder C₁-C₆-Halogenalkoxy substituiertes Aryl-C₁-C₆-alkyl, gegebenenfalls durch Halogen, Cyano (auch im Alkylteil), Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl, gegebenenfalls durch Halogen, Cyano (auch im Alkylteil), Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl-C₁-C₆-alkyl.
- R²: steht für Wasserstoff oder C₁-C₆-Alkyl.
- Q: steht für Stickstoff oder C-R³.
- R³: steht für einen Rest aus der Reihe Wasserstoff, C₁-C₆-Alkyl, C₁-C₆-Halogenalkyl, OH, C₁-C₆-Alkoxy, C₁-C₆-Halogenalkoxy, SH, C₁-C₆-Alkylsulfanyl, C₁-C₆-Alkylsulfinyl, C₁-C₆-Alkylsulfonyl, NH₂, C₁-C₆-Alkylamino und Di-(C₁-C₆-alkyl)-amino.
- V: steht für einen Rest aus der Reihe Sauerstoff, Schwefel und NR⁴.
- R⁴: steht für einen Rest aus der Reihe Wasserstoff, Cyano, C₁-C₆-Alkyl, C₂-C₆-Halogenalkyl und C₃-C₆-Cycloalkyl.

Besonders bevorzugte Substituenten bzw. Bereiche der in den Verbindungen der Formel (I) aufgeführten Reste werden im Folgenden erläutert.

Het steht für einen Rest aus der Reihe 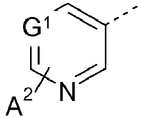 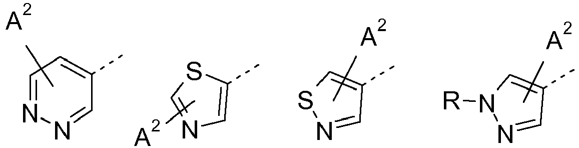 worin die gestrichelte Linie die Bindung zu einem der Stickstoffatome im Pyrazolring darstellt.
- G¹: steht für N oder C-A¹.
- A¹: steht für einen Rest aus der Reihe Wasserstoff, Halogen, C₁-C₄-Alkyl und C₁-C₄-Halogenalkyl.
- A²: steht für einen Rest aus der Reihe Wasserstoff, Halogen, C₁-C₄-Alkyl und C₁-C₄-Halogenalkyl.
- R: steht für Wasserstoff, C₁-C₄-Alkyl, C₁-C₄-Halogenalkyl oder gegebenenfalls einfach bis dreifach durch Halogen, Cyano, Nitro, C₁-C₃-Alkyl, C₁-C₃-Alkoxy und einfach durch C₃-C₆-Cycloalkyl substituiertes C₃-C₆-Cycloalkyl.
- R¹: steht für Wasserstoff, C₁-C₆-Alkyl, C₂-C₄-Halogenalkyl, Cyano-C₁-C₄-alkyl, C₂-C₄-Alkenyl, C₂-C₄-Halogenalkenyl, C₂-C₄-Alkinyl, C₂-C₄-Halogenalkinyl, C₁-C₄-Alkoxy, C₁-C₄-Halogenalkoxy, gegebenenfalls durch Halogen substituiertes C₁-C₂-Alkoxy-C₁-C₄-alkyl, gegebenenfalls durch Halogen substituiertes Bis(C₁-C₂-alkoxy)-C₁-C₄-alkyl, gegebenenfalls durch Halogen substituiertes C₁-C₄-Alkylsulfanyl-C₁-C₄-alkyl, gegebenenfalls durch Halogen substituiertes C₁-C₄-Alkylcarbonyl-C₁-C₄-alkyl, gegebenenfalls durch Halogen substituiertes C₁-C₄-Alkylsulfinyl-C₁-C₄-alkyl, gegebenenfalls durch Halogen substituiertes C₁-C₄-Alkylsulfonyl-C₁-C₄-alkyl, Di-(C₁-C₄-alkyl)-aminosulfanyl-C₁-C₄-alkyl, Di-(C₁-C₄-alkyl)-aminosulfinyl-C₁-C₄-alkyl, Di-(C₁-C₄-alkyl)-aminosulfonyl-C₁-C₄-alkyl, gegebenenfalls durch Halogen substituiertes C₁-C₄-Alkoxycarbonyl-C₁-C₄-alkyl, gegebenenfalls durch Halogen substituiertes C₂-C₄-Alkinyloxy, gegebenenfalls durch Halogen substituiertes C₂-C₄-Alkinyloxycarbonyl, Di-(C₁-C₄-alkyl)-aminocarbonyl, N-C₁-C₄-Alkyl-N-C₃-C₆-Cycloalkylaminocarbonyl, Di-(C₁-C₄-alkyl)-aminocarbonyl-C₁-C₄-alkyl, N-C₁-C₄-Alkyl-N-C₃-C₆-Cycloalkylaminocarbonyl-C₁-C₄-alkyl, Heterocyclylcarbonyl-C₁-C₄-alkyl, C₁-C₄-Alkylsulfanyl, C₁-C₄-Halogenalkylsulfanyl, C₁-C₄-Alkylsulfinyl, C₁-C₄-Halogenalkylsulfinyl, C₁-C₄-Alkylsulfonyl, C₁-C₄-Halogenalkylsulfonyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkylcarbonyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl, Pyridyl, Pyrimidyl, Pyrazanyl, Pyridazinyl, Thiazolyl, Isothiazolyl, Thiadiazolyl, Oxazolyl, Isoxazolyl, Oxadiazolyl, Pyrazolyl, Triazinyl oder Triazolyl (wobei diese Hetaryle gegebenenfalls selbst durch C₁-C₄-Alkyl oder Halogen substituiert sind) substituiertes C₃-C₆-Cycloalkyl-C₁-C₄-alkyl, gegebenenfalls durch Halogen, Cyano (auch im Alkylteil), Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₃-C₆-Cycloalkyl-C₁-C₄-alkyl, C₁-C₄-Alkylcarbonyl, C₁-C₄-Alkoxycarbonyl oder Aminocarbonyl substituiertes Heterocyclyl-C₁-C₄-alkyl, gegebenenfalls durch Halogen, Cyano, Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy oder C₁-C₄-Halogenalkoxy substituiertes Aryl, gegebenenfalls durch Halogen, Cyano, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy oder C₁-C₄-Halogenalkoxy substituiertes Aryl-C₁-C₄-alkyl, gegebenenfalls durch Halogen, Cyano (auch im Alkylteil), Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl, gegebenenfalls durch Halogen, Cyano (auch im Alkylteil), Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl-C₁-C₄-alkyl.
- R²: steht für Wasserstoff oder C₁-C₄-Alkyl.
- Q: steht für Stickstoff oder C-R³.
- R³: steht für einen Rest aus der Reihe Wasserstoff, C₁-C₄-Alkyl, C₁-C₄-Halogenalkyl, OH, C₁-C₄-Alkoxy, C₁-C₄-Halogenalkoxy, SH, C₁-C₄-Alkylsulfanyl, C₁-C₄-Alkylsulfinyl, C₁-C₄-Alkylsulfonyl, NH₂, C₁-C₄-Alkylamino und Di-(C₁-C₄-alkyl)-amino
- V: steht für Sauerstoff.

Ganz besonders bevorzugte Substituenten bzw. Bereiche der in den Verbindungen der Formel (I) aufgeführten Reste werden im Folgenden erläutert
Het steht für einen Rest aus der Reihe 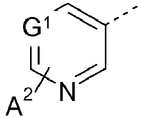 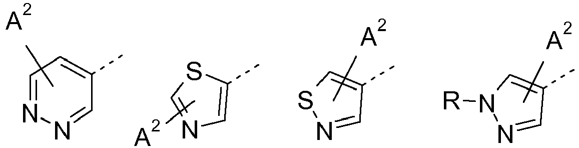 worin die gestrichelte Linie die Bindung zu einem der Stickstoffatome im Pyrazolring darstellt.
- G¹: steht für N oder C-A¹.
- A¹: steht für einen Rest aus der Reihe Wasserstoff, Fluor, Chlor, Brom, Methyl, Ethyl, Isopropyl, *tert-*Butyl, Trifluormethyl, Difluormethyl, 2,2,2-Trifluorethyl, 2,2-Difluorethyl.
- A²: steht für einen Rest aus der Reihe Wasserstoff, Fluor, Chlor, Brom, Methyl, Ethyl, Isopropyl, *tert-*Butyl, Trifluormethyl, Difluormethyl, 2,2,2-Trifluorethyl, 2,2-Difluorethyl.
- R: steht für einen Rest aus der Reihe Wasserstoff, Methyl, Ethyl, Isopropyl, *tert-*Butyl, Trifluormethyl, Difluormethyl, 2,2,2-Trifluorethyl, 2,2-Difluorethyl, Cyclopropyl oder Cyclobutyl.
- R¹: steht für einen Rest aus der Reihe Wasserstoff, Methyl, Ethyl, Isopropyl, n-Propyl, n-Butyl, sec-Butyl, *tert-*Butyl, 2,2-Dimethylpropyl, 2,2,2-Trifluorethyl, 2,2-Difluorethyl, 2,2-Difluor-n-propyl, Methylsulfanylmethyl, Methylsulfanylethyl, Methylsulfanyl-n-propyl, 1-(Methylsulfanyl)propan-2-yl, Trifluormethylsulfonylmethyl, Ethylsulfonylmethyl, 2,2,2-Trifluorethylsulfonylmethyl, 2,2-Difluorethylsulfonylmethyl, Isopropylsulfanylmethyl, Methylsulfinylmethyl, Trifluormethylsulfinylmethyl, Ethylsulfinylmethyl, 2,2,2-Trifluorethylsulfinylmethyl, 2,2-Difluorethylsulfinylmethyl, Isopropylsulfinylmethyl, Methylsulfonylmethyl, Trifluormethylsulfonylmethyl, Ethylsulfonylmethyl, 2,2,2-Trifluorethylsulfonylmethyl, 2,2-Difluorethylsulfonylmethyl, Isopropylsulfonylmethyl, Methoxycarbonylmethyl, Ethoxycarbonylmethyl, Methoxycarbonylethyl, Ethoxycarbonylethyl, Dimethylaminocarbonyl, Diethylaminocarbonyl, *N*-Ethyl-*N-*methylaminocarbonyl, *N-*Isopropyl-*N-*methylaminocarbonyl,, Dimethylaminocarbonylmethyl, Diethylaminocarbonylmethyl, *N*-Ethyl-*N-*methylaminocarbonylmethyl, *N*-Isopropyl-*N-*methylaminocarbonylmethyl, Dimethylaminocarbonylethyl, Diethylaminocarbonylethyl, *N-*Ethyl-*N-*methylaminocarbonylethyl, *N-*Isopropyl-*N-*methylaminocarbonylethyl, *N-*Cyclopropyl-*N-*methylaminocarbonylmethyl, *N*-Cyclopropyl-*N-*methylaminocarbonylethyl, Methylsulfanyl, Trifluormethylsulfanyl, Ethylsulfanyl, 2,2,2-Trifluorethylsulfanyl, 2,2-Difluorethylsulfanyl, Isopropylsulfanyl, Methylsulfinyl, Trifluormethylsulfinyl, Ethylsulfinyl, 2,2,2-Trifluorethylsulfinyl, 2,2-Difluorethylsulfinyl, Isopropylsulfinyl, Methylsulfonyl, Trifluormethylsulfonyl, Ethylsulfonyl, 2,2,2-Trifluorethylsulfonyl, 2,2-Difluorethylsulfonyl, Isopropylsulfonyl, Cyclopropyl, 1-Cyanocyclopropyl, 1-Chlorcyclopropyl, 1-Fluorcyclopropyl, 2-Cyanocyclopropyl, 2-Chlorcyclopropyl, 2-Fluorcyclopropyl, 2,2,3,3-Tetrafluorcyclopropyl, Cyclobutyl, Cyclopentyl, Cyclohexyl, 4-Trifluormethylcyclohexyl, Cyclopropylmethyl, Cyclopropylethyl, Cyclobutylmethyl, *N*-Cyclopropyl-*N*-methylaminocarbonyl, Morpholin-4-ylcarbonylmethyl, Piperazin-1-ylcarbonylmethyl, 4-Methyl-piperazin-1-ylcarbonylmethyl,
- jeweils: gegebenenfalls einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Heterocyclylmethyl und Heterocyclylethyl, jeweils durch Cyclopropyl substituiertes Heterocyclylmethyl und Heterocyclylethyl, wobei der Cyclopropylrest gegebenenfalls einfach oder zweifach durch Methyl, Fluor, Chlor, Cyano oder einfach durch Cyclopropyl substituiert ist, gegebenenfalls einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Hetaryl, durch Cyclopropyl substituiertes Hetaryl, wobei der Cyclopropylrest gegebenenfalls einfach oder zweifach durch Methyl, Fluor, Chlor, Cyano oder einfach durch Cyclopropyl substituiert ist, gegebenenfalls einfach bis dreifach, gleich oder verschieden durch Halogen, Cyano, NitroMethyl, Ethyl, Isopropyl, tert-Butyl, Trifluormethyl, Difluormethyl, Methoxy, Trifluormethoxy oder Difluormethoxy substituiertes Aryl, durch Cyclopropyl substituiertes Aryl, wobei der Cyclopropylrest gegebenenfalls einfach oder zweifach durch Methyl, Fluor, Chlor, Cyano oder einfach durch Cyclopropyl substituiert ist, jeweils gegebenenfalls einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Arylmethyl und Arylethyl, jeweils durch Cyclopropyl substituiertes Arylmethyl und Arylethyl, wobei der Cyclopropylrest gegebenenfalls einfach oder zweifach durch Methyl, Fluor, Chlor, Cyano oder einfach durch Cyclopropyl substituiert ist, jeweils gegebenenfalls jeweils einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Hetarylmethyl und Hetarylethyl, jeweils durch Cyclopropyl substituiertes Hetarylmethyl und Hetarylethyl, wobei der Cyclopropylrest gegebenenfalls einfach oder zweifach durch Methyl, Fluor, Chlor, Cyano oder einfach durch Cyclopropyl substituiert ist.
- R²: steht für Wasserstoff oder Methyl.
- Q: steht für Stickstoff oder C-R³.
- R³: steht für einen Rest aus der Reihe Wasserstoff, Methyl, Ethyl, Isopropyl, *tert-*Butyl, Cyclopropyl, Cyclobutyl, Trifluormethyl, Difluormethyl und Trifluorethyl.
- V: steht für Sauerstoff.

In den bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
Heterocyclyl für einen gesättigten 4-, 5- oder 6-Ring, der 1 oder 2 Stickstoffatome und/oder ein Sauerstoffatom und/oder ein Schwefelatom enthält, beispielsweise für Azetidinyl, Azolidinyl, Azinanyl, Oxetanyl, Oxolanyl, Oxanyl, Dioxanyl, Thiethanyl, Thiolanyl, Thianyl, Piperazinyl, Morpholinyl und Tetrahydrofuryl.
In den besonders bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl und Phenanthrenyl und steht wiederum bevorzugt für Phenyl,
Hetaryl (auch als Teil einer größeren Einheit, wie beispielsweise Hetarylalkyl) ausgewählt aus der Reihe Pyridyl, Pyrimidyl, Pyrazanyl, Thiazolyl, Isothiazolyl und Pyridazinyl und
Heterocyclyl ausgewählt aus der Reihe Azetidinyl, Azolidinyl, Azinanyl, Oxetanyl, Oxolanyl, Oxanyl, Dioxanyl, Thiethanyl, Thiolanyl, Thianyl und Tetrahydrofuryl.

In den ganz besonders bevorzugten Definitionen steht, sofern nichts anderes angegeben ist,
Aryl für Phenyl,
Hetaryl (gleichbedeutend mit Heteroaryl, auch als Teil einer größeren Einheit, wie beispielsweise Hetarylalkyl) für Pyridyl, Pyrimidyl, Pyrazanyl und Pyridazinyl und
Heterocyclyl für Tetrahydrofury und Dioxanyl.

Durch Halogen substituierte Reste, z.B. Halogenalkyl (=Haloalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt verwendet werden Verbindungen der Formel (I), in welchen eine Kombination der vorstehend als bevorzugt aufgeführten Bedeutungen vorliegt.

Erfindungsgemäß besonders bevorzugt verwendet werden Verbindungen der Formel (I), in welchen eine Kombination der vorstehend als besonders bevorzugt aufgeführten Bedeutungen vorliegt.

Erfindungsgemäß ganz besonders bevorzugt verwendet werden Verbindungen der Formel (I), in welchen eine Kombination der vorstehend als ganz besonders bevorzugt aufgeführten Bedeutungen vorliegt.

Eine bevorzugte Ausführungsform der Erfindung betrifft Verbindungen der Formel (Ia).

Eine weitere bevorzugte Ausführungsform der Erfindung betrifft Verbindungen der Formel (Ib).

Eine weitere bevorzugte Ausführungsform der Erfindung betrifft Verbindungen der Formel (Ia), in welchen Het für 3-Pyridyl steht.

Eine weitere bevorzugte Ausführungsform der Erfindung betrifft Verbindungen der Formel (Ib), in welchen Het für 3-Pyridyl steht.

Eine weitere bevorzugte Ausführungsform der Erfindung betrifft Verbindungen der Formel (Ib), in welchen Q für C-H steht.

Die Verbindungen der Formel (I) können gegebenenfalls auch in Abhängigkeit von der Art der Substituenten als Stereoisomere, d.h. als geometrische und/oder als optische Isomere oder Isomerengemische vorliegen in unterschiedlichen Zusammensetzungen vorliegen. Sowohl die reinen Stereoisomeren als auch beliebige Gemische dieser Isomeren sind Gegenstand dieser Erfindung, auch wenn hier im Allgemeinen nur von Verbindungen der Formel (I) die Rede ist.

Vorzugsweise werden jedoch die optisch aktiven, stereoisomeren Formen der Verbindungen der Formel (I) und deren Salze erfindungsgemäß verwendet.

Die Erfindung betrifft daher sowohl die reinen Enantiomeren und Diastereomeren, als auch deren Gemische zur Bekämpfung von tierischen Schädlingen, zu denen Arthropoden und insbesondere Insekten zählen.

Wenn Q für C-R³ steht und R³ für OH, SH oder NH₂ steht, können die Verbindungen der Formeln (Ia) und (Ib) in tautomeren Strukturen vorliegen, wie beispielhaft anhand der Verbindungen der Formel (Ia) mit R³ = OH gezeigt:

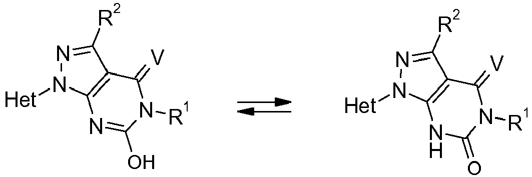

Auch die tautomeren Formen sind Gegenstand der Erfindung.

Die Anmeldung betrifft auch neue Verbindungen der Formel (Ia) 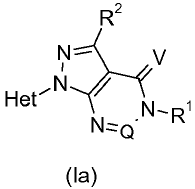 in welcher
Het für einen Rest aus der Reihe 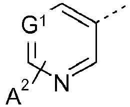 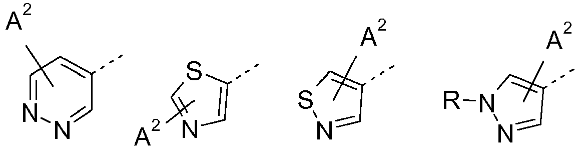 steht, worin die gestrichelte Linie die Bindung zu einem der Stickstoffatome im Pyrazolring darstellt und
- G¹: für N oder C-A¹ steht,
- A¹: für Wasserstoff, Halogen, Cyano, Nitro, Alkyl, Halogenalkyl, Alkoxy, Halogenalkoxy oder gegebenenfalls substituiertes Cycloalkyl oder Cycloalkenyl steht,
- A²: für Wasserstoff, Halogen, Cyano, Nitro, Alkyl, Halogenalkyl, Alkoxy, Halogenalkoxy oder gegebenenfalls substituiertes Cycloalkyl oder Cycloalkenyl steht,
- R: für Wasserstoff, Alkyl, Halogenalkyl oder gegebenenfalls substituiertes Cycloalkyl steht,
- R¹: für einen Rest aus der Reihe Wasserstoff, Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylthioalkyl, gegebenenfalls durch Halogen substituiertes Alkylcarbonylalkyl, gegebenenfalls durch Halogen substituiertes Alkylsulfinylalkyl, jeweils gegebenenfalls durch Halogen substituiertes Alkylsulfonylalkyl, Dialkylaminosulfanylalkyl, Dialkylaminosulfinylalkyl und Dialkylaminosulfonylalkyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylalkyl, gegebenenfalls substituiertes Heterocyclyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Heterocyclylalkyl, gegebenenfalls durch Halogen, Cyano, Nitro, Alkyl, Halogenalkyl, Cycloalkyl (welches substituiert sein kann), Alkoxy oder Halogenalkoxy substituiertes Aryl, gegebenenfalls durch Halogen, Cyano, Alkyl, Halogenalkyl, Cycloalkyl (welches substituiert sein kann), Alkoxy oder Halogenalkoxy substituiertes Arylalkyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetaryl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetarylalkyl steht,
- R²: für Wasserstoff oder Alkyl steht,
- Q: für Stickstoff oder C-R³ steht, worin
- R³: für einen Rest aus der Reihe Wasserstoff, Alkyl, Halogenalkyl, OH, Alkoxy, Halogenalkoxy, SH, Alkylsulfanyl, Alkylsulfinyl, Alkylsulfonyl, NH₂, Alkylamino und Dialkylamino steht,
- V: für einen Rest aus der Reihe Sauerstoff, Schwefel und NR⁴ steht und
- R⁴: für einen Rest aus der Reihe Wasserstoff, Cyano, Alkyl, Halogenalkyl und Cycloalkyl steht,
mit der Maßgabe, dass Verbindungen, in denen R¹ für 4-Chlorphenyl steht und gleichzeitig Q für C,
R³ für Methyl, V für O, G¹ für N oder C-A¹ steht und A¹ und A² für H stehen, ausgenommen sind und dass weiterhin Verbindungen ausgenommen sind, in denen R¹ für 3-Chlorphenyl oder gegebenenfalls substituiertes Cycloalkyl steht und gleichzeitig Q für C-R³ steht, R³ für Wasserstoff steht und V für O steht.

Bevorzugte Substituenten bzw. Bereiche der in den Verbindungen der Formel (Ia) aufgeführten Reste werden im Folgenden erläutert.

Het steht für einen Rest aus der Reihe 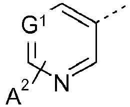 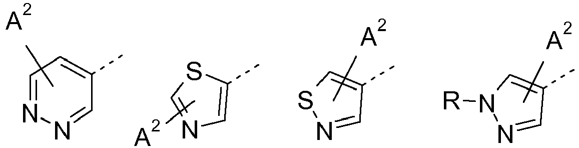 worin die gestrichelte Linie die Bindung zu einem der Stickstoffatome im Pyrazolring darstellt.
- G¹: steht für N oder C-A¹.
- A¹: steht für einen Rest aus der Reihe Wasserstoff, Halogen, C₁-C₆-Alkyl und C₁-C₄-Halogenalkyl.
- A²: steht für einen Rest aus der Reihe Wasserstoff, Halogen, C₁-C₆-Alkyl und C₁-C₄-Halogenalkyl.
- R: steht für einen Rest aus der Reihe Wasserstoff, C₁-C₆-Alkyl, C₁-C₆-Halogenalkyl und gegebenenfalls einfach bis dreifach durch Halogen, Cyano, Nitro, C₁-C₃-Alkyl, C₁-C₃-Alkoxy und C₃-C₆-Cycloalkyl substituiertes C₃-C₆-Cycloalkyl.
- R¹: steht für einen Rest aus der Reihe Wasserstoff, C₁-C₆-Alkyl, C₂-C₆-Halogenalkyl, Cyano-C₁-C₆-alkyl, C₂-C₄-Alkenyl, C₂-C₄-Halogenalkenyl, C₂-C₄-Alkinyl, C₂-C₄-Halogenalkinyl, C₁-C₆-Alkoxy, C₁-C₆-Halogenalkoxy, gegebenenfalls durch Halogen substituiertes C₁-C₆-Alkoxy-C₁-C₆-alkyl, gegebenenfalls durch Halogen substituiertes Bis(C₁-C₆-alkoxy)-C₁-C₆-alkyl, gegebenenfalls durch Halogen substituiertes C₁-C₆-Alkylsulfanyl-C₁-C₆-alkyl, gegebenenfalls durch Halogen substituiertes C₁-C₄-Alkylcarbonyl-C₁-C₄-alkyl, gegebenenfalls durch Halogen substituiertes C₁-C₆-Alkylsulfinyl-C₁-C₆-alkyl, gegebenenfalls durch Halogen substituiertes C₁-C₆-Alkylsulfonyl-C₁-C₆-alkyl, Di-(C₁-C₆-alkyl)-aminosulfanyl-C₁-C₆-alkyl, Di-(C₁-C₆-alkyl)-aminosulfinyl-C₁-C₆-alkyl, Di-(C₁-C₆-alkyl)-aminosulfonyl-C₁-C₆-alkyl, gegebenenfalls durch Halogen substituiertes C₁-C₆-Alkoxycarbonyl-C₁-C₆-alkyl, gegebenenfalls durch Halogen substituiertes C₂-C₄-Alkinyloxy, gegebenenfalls durch Halogen substituiertes C₂-C₄-Alkinyloxycarbonyl, Di-(C₁-C₆-alkyl)-aminocarbonyl, N-C₁-C₆-Alkyl-N-C₃-C₆-Cycloalkylaminocarbonyl, Di-(C₁-C₆-alkyl)-aminocarbonyl-(C₁-C₆)-alkyl, N-C₁-C₆-Alkyl-N-C₃-C₆-Cycloalkylaminocarbonyl-(C₁-C₆)-alkyl, Heterocyclylcarbonyl-(C₁-C₆)-alkyl, C₁-C₆-Alkylsulfanyl, C₁-C₆-Halogenalkylsulfanyl, C₁-C₆-Alkylsulfinyl, C₁-C₆-Halogenalkylsulfinyl, C₁-C₆-Alkylsulfonyl, C₁-C₆-Halogenalkylsulfonyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkylcarbonyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl-C₁-C₆-alkyl, gegebenenfalls durch Halogen, Cyano (auch im Alkylteil), Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Heterocyclyl-C₁-C₆-alkyl, gegebenenfalls durch Halogen, Cyano, Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy oder C₁-C₆-Halogenalkoxy substituiertes Aryl, gegebenenfalls durch Halogen, Cyano, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy oder C₁-C₆-Halogenalkoxy substituiertes Aryl-C₁-C₆-alkyl, gegebenenfalls durch Halogen, Cyano (auch im Alkylteil), Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl, gegebenenfalls durch Halogen, Cyano (auch im Alkylteil), Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl-C₁-C₆-alkyl.
- R²: steht für Wasserstoff oder C₁-C₆-Alkyl.
- Q: steht für Stickstoff oder C-R³.
- R³: steht für einen Rest aus der Reihe Wasserstoff, C₁-C₆-Alkyl, C₁-C₆-Halogenalkyl, OH, C₁-C₆-Alkoxy, C₁-C₆-Halogenalkoxy, SH, C₁-C₆-Alkylsulfanyl, C₁-C₆-Alkylsulfinyl, C₁-C₆-Alkylsulfonyl, NH₂, C₁-C₆-Alkylamino und Di-(C₁-C₆-alkyl)-amino.
- V: steht für einen Rest aus der Reihe Sauerstoff, Schwefel und NR⁴.
- R⁴: steht für einen Rest aus der Reihe Wasserstoff, Cyano, C₁-C₆-Alkyl, C₂-C₆-Halogenalkyl und C₃-C₆-Cycloalkyl.

Besonders bevorzugte Substituenten bzw. Bereiche der in den Verbindungen der Formel (Ia) aufgeführten Reste werden im Folgenden erläutert.
Het steht für einen Rest aus der Reihe 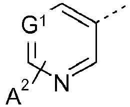 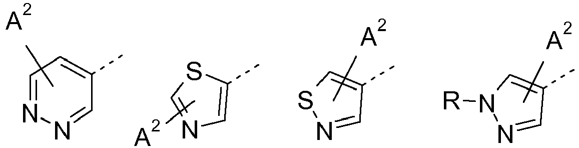 worin die gestrichelte Linie die Bindung zu einem der Stickstoffatome im Pyrazolring darstellt.
- G¹: steht für N oder C-A¹.
- A¹: steht für einen Rest aus der Reihe Wasserstoff, Halogen, C₁-C₄-Alkyl und C₁-C₄-Halogenalkyl.
- A²: steht für einen Rest aus der Reihe Wasserstoff, Halogen, C₁-C₄-Alkyl und C₁-C₄-Halogenalkyl.
- R: steht für Wasserstoff, C₁-C₄-Alkyl, C₁-C₄-Halogenalkyl oder gegebenenfalls einfach bis dreifach durch Halogen, Cyano, Nitro, C₁-C₃-Alkyl, C₁-C₃-Alkoxy und C₃-C₆-Cycloalkyl substituiertes C₃-C₆-Cycloalkyl.
- R¹: steht für Wasserstoff, C₁-C₆-Alkyl, C₂-C₄-Halogenalkyl, Cyano-C₁-C₄-alkyl, C₂-C₄-Alkenyl, C₂-C₄-Halogenalkenyl, C₂-C₄-Alkinyl, C₂-C₄-Halogenalkinyl, C₁-C₄-Alkoxy, C₁-C₄-Halogenalkoxy, gegebenenfalls durch Halogen substituiertes C₁-C₂-Alkoxy-C₁-C₄-alkyl, gegebenenfalls durch Halogen substituiertes Bis(C₁-C₂-alkoxy)-C₁-C₄-alkyl, gegebenenfalls durch Halogen substituiertes C₁-C₄-Alkylsulfanyl-C₁-C₄-alkyl, gegebenenfalls durch Halogen substituiertes C₁-C₄-Alkylcarbonyl-C₁-C₄-alkyl, gegebenenfalls durch Halogen substituiertes C₁-C₄-Alkylsulfinyl-C₁-C₄-alkyl, gegebenenfalls durch Halogen substituiertes C₁-C₄-Alkylsulfonyl-C₁-C₄-alkyl, Di-(C₁-C₄-alkyl)-aminosulfanyl-C₁-C₄-alkyl, Di-(C₁-C₄-alkyl)-aminosulfinyl-C₁-C₄-alkyl, Di-(C₁-C₄-alkyl)-aminosulfonyl-C₁-C₄-alkyl, gegebenenfalls durch Halogen substituiertes C₁-C₄-Alkoxycarbonyl-C₁-C₄-alkyl, gegebenenfalls durch Halogen substituiertes C₂-C₄-Alkinyloxy, gegebenenfalls durch Halogen substituiertes C₂-C₄-Alkinyloxycarbonyl, Di-(C₁-C₄-alkyl)-aminocarbonyl, N-C₁-C₄-Alkyl-N-C₃-C₆-Cycloalkylaminocarbonyl, Di-(C₁-C₄-alkyl)-aminocarbonyl-C₁-C₄-alkyl, N-C₁-C₄-Alkyl-N-C₃-C₆-Cycloalkylaminocarbonyl-C₁-C₄-alkyl, Heterocyclylcarbonyl-C₁-C₄-alkyl, C₁-C₄-Alkylsulfanyl, C₁-C₄-Halogenalkylsulfanyl, C₁-C₄-Alkylsulfinyl, C₁-C₄-Halogenalkylsulfinyl, C₁-C₄-Alkylsulfonyl, C₁-C₄-Halogenalkylsulfonyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkylcarbonyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl, Pyridyl, Pyrimidyl, Pyrazanyl, Pyridazinyl, Thiazolyl, Isothiazolyl, Thiadiazolyl, Oxazolyl, Isoxazolyl, Oxadiazolyl, Pyrazolyl, Triazinyl oder Triazolyl (welches gegebenenfalls selbst durch C₁-C₄-Alkyl oder Halogen substituiert ist) substituiertes C₃-C₆-Cycloalkyl-C₁-C₄-alkyl, gegebenenfalls durch Halogen, Cyano (auch im Alkylteil), Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₃-C₆-Cycloalkyl-C₁-C₄-alkyl, C₁-C₄-Alkylcarbonyl, C₁-C₄-Alkoxycarbonyl oder Aminocarbonyl substituiertes Heterocyclyl-C₁-C₄-alkyl, gegebenenfalls durch Halogen, Cyano, Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy oder C₁-C₄-Halogenalkoxy substituiertes Aryl, gegebenenfalls durch Halogen, Cyano, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy oder C₁-C₄-Halogenalkoxy substituiertes Aryl-C₁-C₄-alkyl, gegebenenfalls durch Halogen, Cyano (auch im Alkylteil), Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl, gegebenenfalls durch Halogen, Cyano (auch im Alkylteil), Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl-C₁-C₄-alkyl.
- R²: steht für Wasserstoff oder C₁-C₄-Alkyl.
- Q: steht für Stickstoff oder C-R³.
- R³: steht für einen Rest aus der Reihe Wasserstoff, C₁-C₄-Alkyl, C₁-C₄-Halogenalkyl, OH, C₁-C₄-Alkoxy, C₁-C₄-Halogenalkoxy, SH, C₁-C₄-Alkylsulfanyl, C₁-C₄-Alkylsulfinyl, C₁-C₄-Alkylsulfonyl, NH₂, C₁-C₄-Alkylamino und Di-(C₁-C₄-alkyl)-amino
- V: steht für Sauerstoff.

Ganz besonders bevorzugte Substituenten bzw. Bereiche der in den Verbindungen der Formel (Ia) aufgeführten Reste werden im Folgenden erläutert.

Het steht für einen Rest aus der Reihe 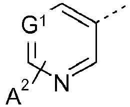 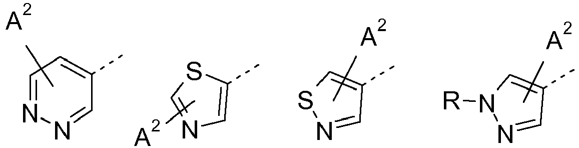 worin die gestrichelte Linie die Bindung zu einem der Stickstoffatome im Pyrazolring darstellt.
G¹ steht für N oder C-A¹.
- A¹: steht für einen Rest aus der Reihe Wasserstoff, Fluor, Chlor, Brom, Methyl, Ethyl, Isopropyl, *tert-Butyl,* Trifluormethyl, Difluormethyl, 2,2,2-Trifluorethyl, 2,2-Difluorethyl.
- A²: steht für einen Rest aus der Reihe Wasserstoff, Fluor, Chlor, Brom, Methyl, Ethyl, Isopropyl, *tert-Butyl,* Trifluormethyl, Difluormethyl, 2,2,2-Trifluorethyl, 2,2-Difluorethyl.
- R: steht für einen Rest aus der Reihe Wasserstoff, Methyl, Ethyl, Isopropyl, *tert-*Butyl, Trifluormethyl, Difluormethyl, 2,2,2-Trifluorethyl, 2,2-Difluorethyl, Cyclopropyl oder Cyclobutyl.
- R¹: für einen Rest aus der Reihe Wasserstoff, Methyl, Ethyl, Isopropyl, n-Propyl, n-Butyl, sec-Butyl, *tert-*Butyl, 2,2-Dimethylpropyl, 2,2,2-Trifluorethyl, 2,2-Difluorethyl, 2,2-Difluor-n-propyl, Methylsulfanylmethyl, Methylsulfanylethyl, Methylsulfanyl-n-propyl, 1-(Methylsulfanyl)propan-2-yl, Trifluormethylsulfonylmethyl, Ethylsulfonylmethyl, 2,2,2-Trifluorethylsulfonylmethyl, 2,2-Difluorethylsulfonylmethyl, Isopropylsulfanylmethyl, Methylsulfinylmethyl, Trifluormethylsulfinylmethyl, Ethylsulfinylmethyl, 2,2,2-Trifluorethylsulfinylmethyl, 2,2-Difluorethylsulfinylmethyl, Isopropylsulfinylmethyl, Methylsulfonylmethyl, Trifluormethylsulfonylmethyl, Ethylsulfonylmethyl, 2,2,2-Trifluorethylsulfonylmethyl, 2,2-Difluorethylsulfonylmethyl, Isopropylsulfonylmethyl, Methoxycarbonylmethyl, Ethoxycarbonylmethyl, Methoxycarbonylethyl, Ethoxycarbonylethyl, Dimethylaminocarbonyl, Diethylaminocarbonyl, *N-*Ethyl-*N-*methylaminocarbonyl, *N-*Isopropyl-*N*-methylaminocarbonyl,, Dimethylaminocarbonylmethyl, Diethylaminocarbonylmethyl, *N-*Ethyl-*N-*methylaminocarbonylmethyl, *N*-Isopropyl-*N-*methylaminocarbonylmethyl, Dimethylaminocarbonylethyl, Diethylaminocarbonylethyl, *N-*Ethyl-*N-*methylaminocarbonylethyl, *N-*Isopropyl-*N-*methylaminocarbonylethyl, *N-*Cyclopropyl-*N-*methylaminocarbonylmethyl, *N*-Cyclopropyl-*N-*methylaminocarbonylethyl, Methylsulfanyl, Trifluormethylsulfanyl, Ethylsulfanyl, 2,2,2-Trifluorethylsulfanyl, 2,2-Difluorethylsulfanyl, Isopropylsulfanyl, Methylsulfinyl, Trifluormethylsulfinyl, Ethylsulfinyl, 2,2,2-Trifluorethylsulfinyl, 2,2-Difluorethylsulfinyl, Isopropylsulfinyl, Methylsulfonyl, Trifluormethylsulfonyl, Ethylsulfonyl, 2,2,2-Trifluorethylsulfonyl, 2,2-Difluorethylsulfonyl, Isopropylsulfonyl, Cyclopropyl, 1-Cyanocyclopropyl, 1-Chlorcyclopropyl, 1-Fluorcyclopropyl, 2-Cyanocyclopropyl, 2-Chlorcyclopropyl, 2-Fluorcyclopropyl, 2,2,3,3-Tetrafluorcyclopropyl, Cyclobutyl, Cyclopentyl, Cyclohexyl, 4-Trifluormethylcyclohexyl, Cyclopropylmethyl, Cyclopropylethyl, Cyclobutylmethyl, *N*-Cyclopropyl-*N*-methylaminocarbonyl, Morpholin-4-ylcarbonylmethyl, Piperazin-1-ylcarbonylmethyl, 4-Methyl-piperazin-1-ylcarbonylmethyl,
jeweils gegebenenfalls einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Heterocyclylmethyl und Heterocyclylethyl, jeweils durch Cyclopropyl substituiertes Heterocyclylmethyl und Heterocyclylethyl, wobei der Cyclopropylrest gegebenenfalls einfach oder zweifach durch Methyl, Fluor, Chlor, Cyano oder einfach durch Cyclopropyl substituiert ist, gegebenenfalls einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Hetaryl, durch Cyclopropyl substituiertes Hetaryl, wobei der Cyclopropylrest gegebenenfalls einfach oder zweifach durch Methyl, Fluor, Chlor, Cyano oder einfach durch Cyclopropyl substituiert ist, gegebenenfalls einfach bis dreifach, gleich oder verschieden durch Halogen, Cyano, NitroMethyl, Ethyl, Isopropyl, tert-Butyl, Trifluormethyl, Difluormethyl, Methoxy, Trifluormethoxy oder Difluormethoxy substituiertes Aryl, durch Cyclopropyl substituiertes Aryl, wobei der Cyclopropylrest gegebenenfalls einfach oder zweifach durch Methyl, Fluor, Chlor, Cyano oder einfach durch Cyclopropyl substituiert ist, jeweils gegebenenfalls einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Arylmethyl und Arylethyl, jeweils durch Cyclopropyl substituiertes Arylmethyl und Arylethyl, wobei der Cyclopropylrest gegebenenfalls einfach oder zweifach durch Methyl, Fluor, Chlor, Cyano oder einfach durch Cyclopropyl substituiert ist, jeweils gegebenenfalls jeweils einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Hetarylmethyl und Hetarylethyl, jeweils durch Cyclopropyl substituiertes Hetarylmethyl und Hetarylethyl, wobei der Cyclopropylrest gegebenenfalls einfach oder zweifach durch Methyl, Fluor, Chlor, Cyano oder einfach durch Cyclopropyl substituiert ist.
- R²: steht für Wasserstoff oder Methyl.
- Q: steht für Stickstoff oder C-R³.
- R³: steht für einen Rest aus der Reihe Wasserstoff, Methyl, Ethyl, Isopropyl, *tert*-Butyl, Cyclopropyl, Cyclobutyl, Trifluormethyl, Difluormethyl und Trifluorethyl.
- V: steht für Sauerstoff.

Für die neuen Verbindungen der Formel (Ia) gilt jeweils, dass Verbindungen, in denen R¹ für 4-Chlorphenyl steht und gleichzeitig Q für C, R³ für Methyl, V für O, G¹ für N oder C-A¹ steht und A¹ und A² für H stehen, ausgenommen sind und dass weiterhin Verbindungen ausgenommen sind, in denen R¹ für 3-Chlorphenyl oder gegebenenfalls substituiertes Cycloalkyl steht und gleichzeitig Q für C-R³ steht, R³ für Wasserstoff steht und V für O steht.

In den bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
Heterocyclyl für einen gesättigten 4-, 5- oder 6-Ring, der 1 oder 2 Stickstoffatome und/oder ein Sauerstoffatom und/oder ein Schwefelatom enthält, beispielsweise für Azetidinyl, Azolidinyl, Azinanyl, Oxetanyl, Oxolanyl, Oxanyl, Dioxanyl, Thiethanyl, Thiolanyl, Thianyl, Piperazinyl, Morpholinyl und Tetrahydrofuryl.
In den besonders bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (auch als Teil einer größeren Einheit, wie beispielsweise Hetarylalkyl) ausgewählt aus der Reihe Pyridyl, Pyrimidyl, Pyrazanyl, Thiazolyl, Isothiazolyl und Pyridazinyl.

Heterocyclyl für Azetidinyl, Azolidinyl, Azinanyl, Oxetanyl, Oxolanyl, Oxanyl, Dioxanyl, Thiethanyl, Thiolanyl, Thianyl und Tetrahydrofuryl.

In den ganz besonders bevorzugten Definitionen steht, sofern nichts anderes angegeben ist,
Aryl für Phenyl,
Hetaryl (gleichbedeutend mit Heteroaryl, auch als Teil einer größeren Einheit, wie beispielsweise Hetarylalkyl) für Pyridyl, Pyrimidyl, Pyrazanyl und Pyridazinyl und
Heterocyclyl für Tetrahydrofuryl und Dioxanyl.

Durch Halogen substituierte Reste, z.B. Halogenalkyl (= Haloalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt sind neue Verbindungen der Formel (Ia), in welchen eine Kombination der vorstehend als bevorzugt aufgeführten Bedeutungen vorliegt.

Erfindungsgemäß besonders bevorzugt sind neue Verbindungen der Formel (Ia), in welchen eine Kombination der vorstehend als besonders bevorzugt aufgeführten Bedeutungen vorliegt.

Erfindungsgemäß ganz besonders bevorzugt sind neue Verbindungen der Formel (Ia), in welchen eine Kombination der vorstehend als ganz besonders bevorzugt aufgeführten Bedeutungen vorliegt.

Erfindungsgemäß ausdrücklich ganz besonders bevorzugt sind neue Verbindungen der Formel (Ia), in welchen eine Kombination der vorstehend als ausdrücklich ganz besonders bevorzugt aufgeführten Bedeutungen vorliegt.

Die Anmeldung betrifft auch neue Verbindungen der Formel (Ib) 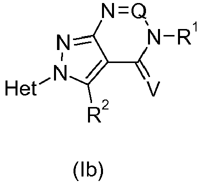 in welcher
Het für einen Rest aus der Reihe 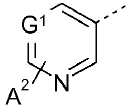 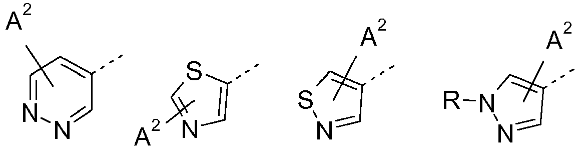 steht, worin die gestrichelte Linie die Bindung zu einem der Stickstoffatome im Pyrazolring darstellt und
- G¹: für N oder C-A¹ steht,
- A¹: für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
- A²: für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
- R: für Wasserstoff, Alkyl, Halogenalkyl oder gegebenenfalls substituiertes Cycloalkyl steht,
- R¹: für einen Rest aus der Reihe Wasserstoff, Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylthioalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, Dialkylaminosulfanylalkyl, Dialkylaminosulfinylalkyl, Dialkylaminosulfonylalkyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylalkyl, gegebenenfalls substituiertes Heterocyclyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfonyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Heterocyclylalkyl, gegebenenfalls durch Halogen, Cyano, Nitro, Alkyl, Halogenalkyl, Cycloalkyl (welches substituiert sein kann), Alkoxy oder Halogenalkoxy substituiertes Aryl, gegebenenfalls durch Halogen, Cyano, Alkyl, Halogenalkyl, Cycloalkyl (welches substituiert sein kann), Alkoxy oder Halogenalkoxy substituiertes Arylalkyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfonyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetaryl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetarylalkyl steht,
- R²: für Wasserstoff oder Alkyl steht,
- Q: für Stickstoff oder C-R³ steht, worin
- R³: für einen Rest aus der Reihe Wasserstoff, Alkyl, Halogenalkyl, OH, Alkoxy, Halogenalkoxy, SH, Alkylsulfanyl, Alkylsulfinyl, Alkylsulfonyl, NH₂, Alkylamino und Dialkylamino steht,
- V: für einen Rest aus der Reihe Sauerstoff, Schwefel und NR⁴ steht und
- R⁴: für einen Rest aus der Reihe Wasserstoff, Cyano, Alkyl, Halogenalkyl und Cycloalkyl steht,
mit der Maßgabe, dass Verbindungen, in denen R¹ für Phenyl oder 4-Chlorphenyl steht und gleichzeitig Q für C, R³ für Methyl, V für O, G¹ für N oder C-A¹ steht und A¹ und A² für H stehen, ausgenommen sind.
Bevorzugte Substituenten bzw. Bereiche der in den Verbindungen der Formel (Ib) aufgeführten Reste
werden im Folgenden erläutert.
Het steht für einen Rest aus der Reihe 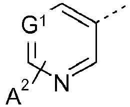 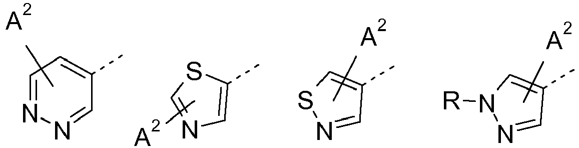 worin die gestrichelte Linie die Bindung zu einem der Stickstoffatome im Pyrazolring darstellt.
- G¹: steht für N oder C-A¹.
- A¹: steht für einen Rest aus der Reihe Wasserstoff, Halogen, C₁-C₆-Alkyl und C₁-C₄-Halogenalkyl.
- A²: steht für einen Rest aus der Reihe Wasserstoff, Halogen, C₁-C₆-Alkyl und C₁-C₄-Halogenalkyl.
- R: steht für einen Rest aus der Reihe Wasserstoff, C₁-C₆-Alkyl, C₁-C₆-Halogenalkyl und gegebenenfalls einfach bis dreifach durch Halogen, Cyano, Nitro, C₁-C₃-Alkyl, C₁-C₃-Alkoxy und C₃-C₆-Cycloalkyl substituiertes C₃-C₆-Cycloalkyl.
- R¹: steht für einen Rest aus der Reihe Wasserstoff, C₁-C₆-Alkyl, C₂-C₆-Halogenalkyl, Cyano-C₁-C₆-alkyl, C₂-C₄-Alkenyl, C₂-C₄-Halogenalkenyl, C₂-C₄-Alkinyl, C₂-C₄-Halogenalkinyl, C₁-C₆-Alkoxy, C₁-C₆-Halogenalkoxy, gegebenenfalls durch Halogen substituiertes C₁-C₆-Alkoxy-C₁-C₆-alkyl, gegebenenfalls durch Halogen substituiertes Bis(C₁-C₆-alkoxy)-C₁-C₆-alkyl, gegebenenfalls durch Halogen substituiertes C₁-C₆-Alkylsulfanyl-C₁-C₆-alkyl, gegebenenfalls durch Halogen substituiertes C₁-C₄-Alkylcarbonyl-C₁-C₄-alkyl, gegebenenfalls durch Halogen substituiertes C₁-C₆-Alkylsulfinyl-C₁-C₆-alkyl, gegebenenfalls durch Halogen substituiertes C₁-C₆-Alkylsulfonyl-C₁-C₆-alkyl, Di-(C₁-C₆-alkyl)-aminosulfanyl-C₁-C₆-alkyl, Di-(C₁-C₆-alkyl)-aminosulfinyl-C₁-C₆-alkyl, Di-(C₁-C₆-alkyl)-aminosulfonyl-C₁-C₆-alkyl, gegebenenfalls durch Halogen substituiertes C₁-C₆-Alkoxycarbonyl-C₁-C₆-alkyl, gegebenenfalls durch Halogen substituiertes C₂-C₄-Alkinyloxy, gegebenenfalls durch Halogen substituiertes C₂-C₄-Alkinyloxycarbonyl, Di-(C₁-C₆-alkyl)-aminocarbonyl, N-C₁-C₆-Alkyl-N-C₃-C₆-Cycloalkylaminocarbonyl, Di-(C₁-C₆-alkyl)-aminocarbonyl-(C₁-C₆)-alkyl, N-C₁-C₆-Alkyl-N-C₃-C₆-Cycloalkylaminocarbonyl-(C₁-C₆)-alkyl, Heterocyclylcarbonyl-(C₁-C₆)-alkyl, C₁-C₆-Alkylsulfanyl, C₁-C₆-Halogenalkylsulfanyl, C₁-C₆-Alkylsulfinyl, C₁-C₆-Halogenalkylsulfinyl, C₁-C₆-Alkylsulfonyl, C₁-C₆-Halogenalkylsulfonyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkylcarbonyl, gegebenenfalls durch Halogen, Cyano, C₁-C₆-Alkyl, C₁-C₆-Halogenalkyl, C₁-C₆-Alkoxy, C₁-C₆-Halogenalkoxy, C₁-C₆-Alkoxycarbonyl, C₁-C₆-Halogenalkoxycarbonyl oder Hetaryl (welches gegebenenfalls selbst durch C₁-C₆-Alkyl oder Halogen substituiert ist) substituiertes C₃-C₆-Cycloalkyl-C₁-C₆-alkyl, gegebenenfalls durch Halogen, Cyano (auch im Alkylteil), Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Heterocyclyl-C₁-C₆-alkyl, gegebenenfalls durch Halogen, Cyano, Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy oder C₁-C₆-Halogenalkoxy substituiertes Aryl, gegebenenfalls durch Halogen, Cyano, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy oder C₁-C₆-Halogenalkoxy substituiertes Aryl-C₁-C₆-alkyl, gegebenenfalls durch Halogen, Cyano (auch im Alkylteil), Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl, gegebenenfalls durch Halogen, Cyano (auch im Alkylteil), Nitro, C₁-C₆-Alkyl, C₁-C₆-Halogenalkyl, C₃-C₆-Cycloalkyl (welches durch Halogen, Cyano, C₁-C₆-Alkyl und C₃-C₆-Cycloalkyl substituiert sein kann), C₁-C₆-Alkoxy, C₁-C₆-Halogenalkoxy, C₁-C₆-Alkylthio, C₁-C₆-Haloalkylthio, C₁-C₆-Alkylsulfinyl, C₁-C₆-Alkylsulfonyl, C₁-C₆-Haloalkylsulfinyl, C₁-C₆-Haloalkylsulfonyl, Amino, C₁-C₆-Alkylamino, Di-(C₁-C₆-alkyl)-amino, C₁-C₆-Alkylcarbonylamino, C₁-C₆-Alkoxycarbonylamino, C₁-C₆-Alkoxy-C₁-C₆-alkyl, C₁-C₆-Halogenalkoxy-C₁-C₆-alkyl, C₂-C₆-Alkenyl, C₂-C₆-Alkinyl, C₃-C₆-Cycloalkyl-C₁-C₆-alkyl, C₁-C₆-Alkylcarbonyl, C₁-C₆-Alkoxycarbonyl oder Aminocarbonyl substituiertes Hetaryl-C₁-C₆-alkyl.
- R²: steht für Wasserstoff oder C₁-C₆-Alkyl.
- Q: steht für Stickstoff oder C-R³.
- R³: steht für einen Rest aus der Reihe Wasserstoff, C₁-C₆-Alkyl, C₁-C₆-Halogenalkyl, OH, C₁-C₆-Alkoxy, C₁-C₆-Halogenalkoxy, SH, C₁-C₆-Alkylsulfanyl, C₁-C₆-Alkylsulfinyl, C₁-C₆-Alkylsulfonyl, NH₂, C₁-C₆-Alkylamino und Di-(C₁-C₆-alkyl)-amino,
- V: steht für einen Rest aus der Reihe Sauerstoff, Schwefel und NR⁴.
- R⁴: steht für einen Rest aus der Reihe Wasserstoff, Cyano, C₁-C₆-Alkyl, C₂-C₆-Halogenalkyl und C₃-C₆-Cycloalkyl.

Besonders bevorzugte Substituenten bzw. Bereiche der in den Verbindungen der Formel (Ib) aufgeführten Reste werden im Folgenden erläutert.

Het steht für einen Rest aus der Reihe 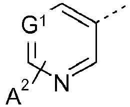 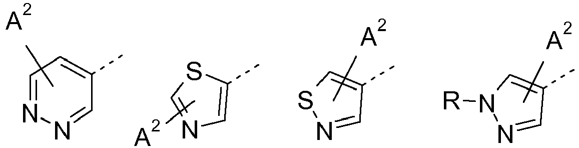 worin die gestrichelte Linie die Bindung zu einem der Stickstoffatome im Pyrazolring darstellt.
- G¹: steht für N oder C-A¹.
- A¹: steht für einen Rest aus der Reihe Wasserstoff, Halogen, C₁-C₄-Alkyl und C₁-C₄-Halogenalkyl.
- A²: steht für einen Rest aus der Reihe Wasserstoff, Halogen, C₁-C₄-Alkyl und C₁-C₄-Halogenalkyl.
- R: steht für einen Rest aus der Reihe Wasserstoff, C₁-C₄-Alkyl, C₁-C₄-Halogenalkyl und gegebenenfalls einfach bis dreifach durch Halogen, Cyano, Nitro, C₁-C₃-Alkyl, C₁-C₃-Alkoxy und C₃-C₆-Cycloalkyl substituiertes C₃-C₆-Cycloalkyl.
- R¹: steht für einen Rest aus der Reihe Wasserstoff, C₁-C₆-Alkyl, C₂-C₄-Halogenalkyl, Cyano-C₁-C₄-alkyl, C₂-C₄-Alkenyl, C₂-C₄-Halogenalkenyl, C₂-C₄-Alkinyl, C₂-C₄-Halogenalkinyl, C₁-C₄-Alkoxy, C₁-C₄-Halogenalkoxy, gegebenenfalls durch Halogen substituiertes C₁-C₂-Alkoxy-C₁-C₄-alkyl, gegebenenfalls durch Halogen substituiertes Bis(C₁-C₂-alkoxy)-C₁-C₄-alkyl, gegebenenfalls durch Halogen substituiertes C₁-C₄-Alkylsulfanyl-C₁-C₄-alkyl, gegebenenfalls durch Halogen substituiertes C₁-C₄-Alkylcarbonyl-C₁-C₄-alkyl, gegebenenfalls durch Halogen substituiertes C₁-C₄-Alkylsulfinyl-C₁-C₄-alkyl, gegebenenfalls durch Halogen substituiertes C₁-C₄-Alkylsulfonyl-C₁-C₄-alkyl, Di-(C₁-C₄-alkyl)-aminosulfanyl-C₁-C₄-alkyl, Di-(C₁-C₄-alkyl)-aminosulfinyl-C₁-C₄-alkyl, Di-(C₁-C₄-alkyl)-aminosulfonyl-C₁-C₄-alkyl, gegebenenfalls durch Halogen substituiertes C₁-C₄-Alkoxycarbonyl-C₁-C₄-alkyl, gegebenenfalls durch Halogen substituiertes C₂-C₄-Alkinyloxy, gegebenenfalls durch Halogen substituiertes C₂-C₄-Alkinyloxycarbonyl, Di-(C₁-C₄-alkyl)-aminocarbonyl, N-C₁-C₄-Alkyl-N-C₃-C₆-Cycloalkylaminocarbonyl, Di-(C₁-C₄-alkyl)-aminocarbonyl-C₁-C₄-alkyl, N-C₁-C₄-Alkyl-N-C₃-C₆-Cycloalkylaminocarbonyl-C₁-C₄-alkyl, Heterocyclylcarbonyl-C₁-C₄-alkyl, C₁-C₄-Alkylsulfanyl, C₁-C₄-Halogenalkylsulfanyl, C₁-C₄-Alkylsulfinyl, C₁-C₄-Halogenalkylsulfinyl, C₁-C₄-Alkylsulfonyl, C₁-C₄-Halogenalkylsulfonyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl oder Pyridyl (welches gegebenenfalls selbst durch C₁-C₄-Alkyl oder Halogen substituiert ist) substituiertes C₃-C₆-Cycloalkylcarbonyl, gegebenenfalls durch Halogen, Cyano, C₁-C₄-Alkyl, C₁-C₄-Halogenalkyl, C₁-C₄-Alkoxy, C₁-C₄-Halogenalkoxy, C₁-C₄-Alkoxycarbonyl, C₁-C₄-Halogenalkoxycarbonyl, Pyridyl, Pyrimidyl, Pyrazanyl, Pyridazinyl, Thiazolyl, Isothiazolyl, Thiadiazolyl, Oxazolyl, Isoxazolyl, Oxadiazolyl, Pyrazolyl, Triazinyl oder Triazolyl (welches gegebenenfalls selbst durch C₁-C₄-Alkyl oder Halogen substituiert ist) substituiertes C₃-C₆-Cycloalkyl-C₁-C₄-alkyl, gegebenenfalls durch Halogen, Cyano (auch im Alkylteil), Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₃-C₆-Cycloalkyl-C₁-C₄-alkyl, C₁-C₄-Alkylcarbonyl, C₁-C₄-Alkoxycarbonyl oder Aminocarbonyl substituiertes Heterocyclyl-C₁-C₄-alkyl, gegebenenfalls durch Halogen, Cyano, Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy oder C₁-C₄-Halogenalkoxy substituiertes Aryl, gegebenenfalls durch Halogen, Cyano, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy oder C₁-C₄-Halogenalkoxy substituiertes Aryl-C₁-C₄-alkyl, gegebenenfalls durch Halogen, Cyano (auch im Alkylteil), Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl, gegebenenfalls durch Halogen, Cyano (auch im Alkylteil), Nitro, C₁-C₄-Alkyl, C₁-C₄-Halogenalkyl, C₃-C₆-Cycloalkyl (welches durch Halogen, Cyano, C₁-C₄-Alkyl und C₃-C₆-Cycloalkyl substituiert sein kann), C₁-C₄-Alkoxy, C₁-C₄-Halogenalkoxy, C₁-C₄-Alkylthio, C₁-C₄-Halogenalkylthio, C₁-C₄-Alkylsulfinyl, C₁-C₄-Alkylsulfonyl, C₁-C₄-Halogenalkylsulfinyl, C₁-C₄-Halogenalkylsulfonyl, Amino, C₁-C₄-Alkylamino, Di-(C₁-C₄-alkyl)-amino, C₁-C₄-Alkylcarbonylamino, C₁-C₄-Alkoxycarbonylamino, C₁-C₄-Alkoxy-C₁-C₄-alkyl, C₁-C₄-Halogenalkoxy-C₁-C₄-alkyl, C₂-C₄-Alkenyl, C₂-C₄-Alkinyl, C₁-C₄-Alkyl-C₃-C₆-cycloalkyl, C₃-C₆-Cycloalkyl-C₁-C₄-alkyl, C₁-C₄-Alkylcarbonyl, C₁-C₄-Alkoxycarbonyl oder Aminocarbonyl substituiertes Hetaryl-C₁-C₄-alkyl.
- R²: steht für Wasserstoff oder C₁-C₄-Alkyl.
- Q: steht für Stickstoff oder C-R³.
- R³: steht für einen Rest aus der Reihe Wasserstoff, C₁-C₄-Alkyl, C₁-C₄-Halogenalkyl, OH, C₁-C₄-Alkoxy, C₁-C₄-Halogenalkoxy, SH, C₁-C₄-Alkylsulfanyl, C₁-C₄-Alkylsulfinyl, C₁-C₄-Alkylsulfonyl, NH₂, C₁-C₄-Alkylamino und Di-(C₁-C₄-alkyl)-amino.
- V: steht für Sauerstoff.

Ganz besonders bevorzugte Substituenten bzw. Bereiche der in den Verbindungen der Formel (Ib) aufgeführten Reste werden im Folgenden erläutert.

Het steht für einen Rest aus der Reihe 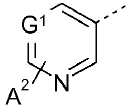 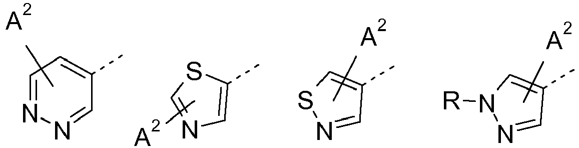 worin die gestrichelte Linie die Bindung zu einem der Stickstoffatome im Pyrazolring darstellt.
- G¹: steht für N oder C-A¹.
- A¹: steht für einen Rest aus der Reihe Wasserstoff, Fluor, Chlor, Brom, Methyl, Ethyl, Isopropyl, *tert*-Butyl, Trifluormethyl, Difluormethyl, 2,2,2-Trifluorethyl, 2,2-Difluorethyl.
- A²: steht für einen Rest aus der Reihe Wasserstoff, Fluor, Chlor, Brom, Methyl, Ethyl, Isopropyl, *tert*-Butyl, Trifluormethyl, Difluormethyl, 2,2,2-Trifluorethyl, 2,2-Difluorethyl.
- R: steht für einen Rest aus der Reihe Wasserstoff, Methyl, Ethyl, Isopropyl, *tert*-Butyl, Trifluormethyl, Difluormethyl, 2,2,2-Trifluorethyl, 2,2-Difluorethyl, Cyclopropyl oder Cyclobutyl.
- R¹: für einen Rest aus der Reihe Wasserstoff, Methyl, Ethyl, Isopropyl, n-Propyl, n-Butyl, sec-Butyl, *tert*-Butyl, 2,2-Dimethylpropyl, 2,2,2-Trifluorethyl, 2,2-Difluorethyl, 2,2-Difluor-n-propyl, Methylsulfanylmethyl, Methylsulfanylethyl, Methylsulfanyl-n-propyl, 1-(Methylsulfanyl)propan-2-yl, Trifluormethylsulfonylmethyl, Ethylsulfonylmethyl, 2,2,2-Trifluorethylsulfonylmethyl, 2,2-Difluorethylsulfonylmethyl, Isopropylsulfanylmethyl, Methylsulfinylmethyl, Trifluormethylsulfinylmethyl, Ethylsulfinylmethyl, 2,2,2-Trifluorethylsulfinylmethyl, 2,2-Difluorethylsulfinylmethyl, Isopropylsulfinylmethyl, Methylsulfonylmethyl, Trifluormethylsulfonylmethyl, Ethylsulfonylmethyl, 2,2,2-Trifluorethylsulfonylmethyl, 2,2-Difluorethylsulfonylmethyl, Isopropylsulfonylmethyl, Methoxycarbonylmethyl, Ethoxycarbonylmethyl, Methoxycarbonylethyl, Ethoxycarbonylethyl, Dimethylaminocarbonyl, Diethylaminocarbonyl, *N*-Ethyl-*N-*methylaminocarbonyl, *N*-Isopropyl-*N*-methylaminocarbonyl,, Dimethylaminocarbonylmethyl, Diethylaminocarbonylmethyl, *N*-Ethyl-*N*-methylaminocarbonylmethyl, *N*-Isopropyl-*N-*methylaminocarbonylmethyl, Dimethylaminocarbonylethyl, Diethylaminocarbonylethyl, *N-*Ethyl-*N*-methylaminocarbonylethyl, *N*-Isopropyl-*N*-methylaminocarbonylethyl, *N-*Cyclopropyl-*N*-methylaminocarbonylmethyl, *N*-Cyclopropyl-*N*-methylaminocarbonylethyl, Methylsulfanyl, Trifluormethylsulfanyl, Ethylsulfanyl, 2,2,2-Trifluorethylsulfanyl, 2,2-Difluorethylsulfanyl, Isopropylsulfanyl, Methylsulfinyl, Trifluormethylsulfinyl, Ethylsulfinyl, 2,2,2-Trifluorethylsulfinyl, 2,2-Difluorethylsulfinyl, Isopropylsulfinyl, Methylsulfonyl, Trifluormethylsulfonyl, Ethylsulfonyl, 2,2,2-Trifluorethylsulfonyl, 2,2-Difluorethylsulfonyl, Isopropylsulfonyl, Cyclopropyl, 1-Cyanocyclopropyl, 1-Chlorcyclopropyl, 1-Fluorcyclopropyl, 2-Cyanocyclopropyl, 2-Chlorcyclopropyl, 2-Fluorcyclopropyl, 2,2,3,3-Tetrafluorcyclopropyl, Cyclobutyl, Cyclopentyl, Cyclohexyl, 4-Trifluormethylcyclohexyl, Cyclopropylmethyl, Cyclopropylethyl, Cyclobutylmethyl, *N*-Cyclopropyl-*N*-methylaminocarbonyl, Morpholin-4-ylcarbonylmethyl, Piperazin-1-ylcarbonylmethyl, 4-Methyl-piperazin-1-ylcarbonylmethyl,
jeweils gegebenenfalls einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Heterocyclylmethyl und Heterocyclylethyl, jeweils durch Cyclopropyl substituiertes Heterocyclylmethyl und Heterocyclylethyl, wobei der Cyclopropylrest gegebenenfalls einfach oder zweifach durch Methyl, Fluor, Chlor, Cyano oder einfach durch Cyclopropyl substituiert ist, gegebenenfalls einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Hetaryl, durch Cyclopropyl substituiertes Hetaryl, wobei der Cyclopropylrest gegebenenfalls einfach oder zweifach durch Methyl, Fluor, Chlor, Cyano oder einfach durch Cyclopropyl substituiert ist, gegebenenfalls einfach bis dreifach, gleich oder verschieden durch Halogen, Cyano, NitroMethyl, Ethyl, Isopropyl, tert-Butyl, Trifluormethyl, Difluormethyl, Methoxy, Trifluormethoxy oder Difluormethoxy substituiertes Aryl, durch Cyclopropyl substituiertes Aryl, wobei der Cyclopropylrest gegebenenfalls einfach oder zweifach durch Methyl, Fluor, Chlor, Cyano oder einfach durch Cyclopropyl substituiert ist, jeweils gegebenenfalls einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Arylmethyl und Arylethyl, jeweils durch Cyclopropyl substituiertes Arylmethyl und Arylethyl, wobei der Cyclopropylrest gegebenenfalls einfach oder zweifach durch Methyl, Fluor, Chlor, Cyano oder einfach durch Cyclopropyl substituiert ist, jeweils gegebenenfalls jeweils einfach bis dreifach, gleich oder verschieden durch Halogen, Cyano, Nitro, Methyl, Ethyl, Isopropyl, tert-Butyl, Trifluormethyl, Difluormethyl, Methoxy, Trifluormethoxy oder Difluormethoxy substituiertes Hetarylmethyl und Hetarylethyl, jeweils durch Cyclopropyl substituiertes Hetarylmethyl und Hetarylethyl, wobei der Cyclopropylrest gegebenenfalls einfach oder zweifach durch Methyl, Fluor, Chlor, Cyano oder einfach durch Cyclopropyl substituiert ist.
- R²: steht für Wasserstoff oder Methyl.
- Q: steht für Stickstoff oder C-R³.
- R³: steht für einen Rest aus der Reihe Wasserstoff, Methyl, Ethyl, Isopropyl, *tert*-Butyl, Cyclopropyl, Cyclobutyl, Trifluormethyl, Difluormethyl und Trifluorethyl.
- V: steht für Sauerstoff.

Für die neuen Verbindungen der Formel (Ib) gilt jeweils, dass Verbindungen, in denen R¹ für Phenyl oder 4-Chlorphenyl steht und gleichzeitig Q für C, R³ für Methyl, V für O, G¹ für N oder C-A¹ steht und A¹ und A² für H stehen, ausgenommen sind.
In den bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl, Phenanthrenyl und steht wiederum bevorzugt für Phenyl,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
Heterocyclyl für einen gesättigten 4-, 5- oder 6-Ring, der 1 oder 2 Stickstoffatome und/oder ein Sauerstoffatom und/oder ein Schwefelatom enthält, beispielsweise für Azetidinyl, Azolidinyl, Azinanyl, Oxetanyl, Oxolanyl, Oxanyl, Dioxanyl, Thiethanyl, Thiolanyl, Thianyl, Piperazinyl, Morpholinyl und Tetrahydrofuryl.

In den besonders bevorzugten Definitionen ist, sofern nichts anderes angegeben ist,
Halogen ausgewählt aus der Reihe Fluor, Chlor, Brom und Iod, bevorzugt wiederum aus der Reihe Fluor, Chlor und Brom,
Aryl (auch als Teil einer größeren Einheit, wie beispielsweise Arylalkyl) ausgewählt aus der Reihe Phenyl, Naphthyl, Anthryl und Phenanthrenyl und steht wiederum bevorzugt für Phenyl,
Hetaryl (auch als Teil einer größeren Einheit, wie beispielsweise Hetarylalkyl) ausgewählt aus der Reihe Pyridyl, Pyrimidyl, Pyrazanyl, Thiazolyl, Isothiazolyl und Pyridazinyl und
Heterocyclyl ausgewählt aus der Reihe Azetidinyl, Azolidinyl, Azinanyl, Oxetanyl, Oxolanyl, Oxanyl, Dioxanyl, Thiethanyl, Thiolanyl, Thianyl und Tetrahydrofuryl.

In den ganz besonders bevorzugten Definitionen steht, sofern nichts anderes angegeben ist,
Aryl für Phenyl,
Hetaryl (gleichbedeutend mit Heteroaryl, auch als Teil einer größeren Einheit, wie beispielsweise Hetarylalkyl) für Pyridyl, Pyrimidyl, Pyrazanyl und Pyridazinyl und
Heterocyclyl für Tetrahydrofuryl und Dioxanyl.

Durch Halogen substituierte Reste, z.B. Halogenalkyl (= Haloalkyl), sind einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt sind neue Verbindungen der Formel (Ib), in welchen eine Kombination der vorstehend als bevorzugt aufgeführten Bedeutungen vorliegt.

Erfindungsgemäß besonders bevorzugt sind neue Verbindungen der Formel (Ib), in welchen eine Kombination der vorstehend als besonders bevorzugt aufgeführten Bedeutungen vorliegt.

Erfindungsgemäß ganz besonders bevorzugt sind neue Verbindungen der Formel (Ib), in welchen eine Kombination der vorstehend als ganz besonders bevorzugt aufgeführten Bedeutungen vorliegt.

Erfindungsgemäß ausdrücklich ganz besonders bevorzugt sind neue Verbindungen der Formel (Ib), in welchen eine Kombination der vorstehend als ausdrücklich ganz besonders bevorzugt aufgeführten Bedeutungen vorliegt.

In einer hervorgehobenen Gruppe von Verbindungen der Formel (I) haben die Reste folgende Bedeutungen.

Het steht für den Rest 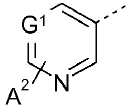 worin die gestrichelte Linie die Bindung zu einem der Stickstoffatome im Pyrazolring darstellt.
- G¹: steht für N oder C-A¹.
- A¹: steht für Wasserstoff.
- A²: steht für Wasserstoff.
- R¹: für einen Rest aus der Reihe Methyl, Ethyl, Isopropyl, n-Propyl, n-Butyl, sec-Butyl, *tert*-Butyl, 2,2-Dimethylpropyl, Trifluormethyl, 2,2,2-Trifluorethyl, 2,2-Difluorethyl, 2,2-Difluor-n-propyl, Methylsulfanylmethyl, Methylsulfanylethyl, Methylsulfanyl-n-propyl, 1-(Methylsulfanyl)propan-2-yl, Methoxycarbonylmethyl, Ethoxycarbonylmethyl, Methoxycarbonylethyl, Ethoxycarbonylethyl, Dimethylaminocarbonylmethyl, Diethylaminocarbonylmethyl, *N*-Ethyl-*N*-methylaminocarbonylmethyl, *N*-Isopropyl-*N-*methylaminocarbonylmethyl, Dimethylaminocarbonylethyl, Diethylaminocarbonylethyl, *N-*Ethyl-*N*-methylaminocarbonylethyl, *N*-Isopropyl-*N*-methylaminocarbonylethyl, *N-*Cyclopropyl-*N*-methylaminocarbonylmethyl, *N*-Cyclopropyl-*N*-methylaminocarbonylethyl, Cyclopropyl, 1-Cyanocyclopropyl, 1-Chlorcyclopropyl, 1-Fluorcyclopropyl, 2-Cyanocyclopropyl, 2-Chlorcyclopropyl, 2-Fluorcyclopropyl, 2,2,3,3-Tetrafluorcyclopropyl, Cyclobutyl, Cyclopentyl, Cyclohexyl, 4-Trifluormethylcyclohexyl, Cyclopropylmethyl, Heterocyclylmethyl und Heterocyclylethyl, jeweils einfach bis dreifach, gleich oder verschieden durch Halogen substituiertes Aryl, Arylmethyl und Arylethyl, Hetarylmethyl und Hetarylethyl, worin Heterocyclyl für Tetrahydrofuran-2-yl, Hetaryl für Pyrimidin-2-yl und Pyridin-2-yl und Aryl für Phenyl, 4-Methylphenyl und 4-Chlorphenyl steht.
- R²: steht für Wasserstoff.
- Q: steht für C-R³.
- R³: steht für einen Rest aus der Reihe Wasserstoff, Methyl, Ethyl, Isopropyl, *tert*-Butyl und Trifluormethyl.
- V: steht für Sauerstoff.

Weiter wurde gefunden, dass sich die neuen Verbindungen der Formel (I) nach den im Folgenden beschriebenen Verfahren herstellen lassen.

Die Verbindungen der Formel (Ia) und (Ib) können beispielsweise nach Verfahren A in zwei Schritten hergestellt werden, wie im folgenden Schema dargestellt. Die dafür benötigten Aminopyrazole der Formeln (IIIa) und (IIIb) können beispielweise nach den Verfahren B, C und D hergestellt werden. 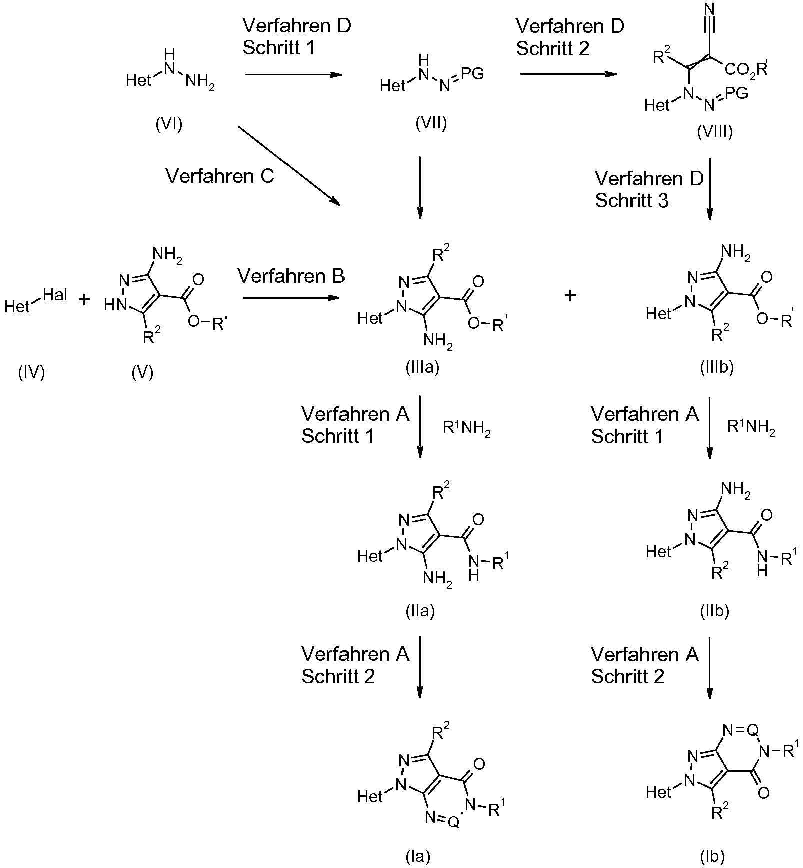 wobei Het, Q, R¹ und R² die oben angegebenen Bedeutungen haben, R'für Wasserstoff oder Alkyl (insbesondere Methyl und Ethyl) steht, Hal für Halogen (bevorzugt Chlor, Brom und Iod) und PG für eine geeignete Schutzgruppe steht.

### Verfahren A

Die Synthese der Verbindungen der Formeln (Ia) und (Ib) kann mit Hilfe von literaturbekannten Methoden in zwei Schritten erfolgen.

Im ersten Syntheseschritt können Verbindungen der Formeln (IIIa) bzw. (IIIb) nach verschiedenen Methoden zu Carbonsäureamiden der Formeln (IIa) bzw. (IIb) umgesetzt werden. Für R' = Alkyl kann diese Umsetzung ohne Aktivierung erfolgen, (vgl. B. M. Trost und I. Fleming in Comprehensive Organic Synthesis, Ed. Pergamon, 1991, Vol. 6). Alternativ sind in der Literatur Aktivierungsmethoden durch die Bildung eines Aluminiumamids bekannt (siehe T. Ooi und K. Marouka in Science of Synthesis, Ed. Georg Thieme, 2003, Vol. 7, 225-246). Diese Aluminiumamide können beispielweise aus den Aminen oder deren Salze durch Umsetzung mit Trimethylaluminium oder deren luft-stabilem Addukt mit 1,4-Diazobicyclo[2.2.2]oktan (DABCO) hergestellt werden (vgl. S. Woodward in Tet. Lett. 2006, 47, 5767-5769).

Alternativ können die Aminopyrazole der Formeln (IIIa) bzw. (IIIb), mit R' = Alkyl, in zwei Stufen zu den Amiden der Formeln (IIa) bzw. (IIb) umgesetzt werden: zuerst Verseifung zu den Carboxylaten, zum Beispiel durch Umsetzung mit einer anorganischen Base (bevorzugt Natron- und Kalilaugen), gegebenenfalls in einem inerten organischen Lösungsmittel, gegebenenfalls lassen sich durch Ansäuern mit einer verdünnten Säure (z.B. wässrige Salzsäure) die Carbonsäuren der Formeln (IIIa) bzw. (IIIb) mit R' = Wasserstoff herstellen und isolieren; anschließende Amidierungsreaktion mit den gewünschten Aminen führt zu den Verbindungen der Formeln (IIa) bzw. (IIb). Für den Amidierungsschritt sind zahlreiche Reaktionsbedingungen beschrieben worden, z.B. G. Benz in Comprehensive Organic Synthesis, 1st Ed., Pergamon Press, Oxford, 1991, Vol. 6, S. 381-417; P.D. Bailey et al. in Comprehensive Organic Functional Group Transformation, 1st Ed., Elsevier Science Ltd., Oxford, 1995, Vol. 5, S. 257-308 und R.C. Larock in Comprehensive Organic Transformations, 2nd Ed., Wiley-VCH, New York, Weinheim, 1999, S. 1929-1994. Einige dieser Reaktionen verlaufen über intermediäre Carbonsäurechloride, die isoliert oder in-situ erzeugt eingesetzt werden können.

Die Amidierungsreaktionen erfolgen gegebenenfalls in Gegenwart eines Kondensationsmittels, gegebenenfalls in Gegenwart eines Säureakzeptors und gegebenenfalls in Gegenwart eines Lösungsmittels.

Als Kondensationsmittel kommen alle üblicherweise für derartige Amidierungsreaktionen verwendbaren Kondensationsmittel infrage. Beispielhaft genannt seien Säurehalogenidbildner wie Phosgen, Phosphortrichlorid, Oxalylchlorid oder Thionylchlorid; Carbodiimide, wie *N,N*'-Dicyclohexylcarbodiimid (DCC) und 1-(3-Dimethylaminopropyl)-3-ethyl-carbodümid (EDCI), oder andere übliche Kondensationsmittel, wie Phosphorpentoxid, Polyphosphorsäure, *N,N'-*Carbonyldiimidazol, 2-Chlorpyridin 1-Methoiodid (Mukaiyamas Reagenz), 2-Ethoxy-N-ethoxycarbonyl-1,2-dihydrochinolin (EEDQ), Triphenylphosphin/Tetrachlorkohlenstoff, Bromtripyrrolidino-phosphonium-hexafluorphosphat (BROP), O-(*1H*-Benzotriazol-1-yloxy)tris(dimethylamino)phosphonium-hexafluorphosphat (BOP), *N,N,N',N'-*Bis(tetramethylen)cloruronium-tetrafluorborat, O-(*1H*-Benzotriazol-1-yl)-*N,N,N',N'-*tetramethyluronium-hexafluorphosphat (HBTU), O-(*1H*-Benzotriazol-1-yl)-*N,N,N',N'-*bis(tetramethylen)uronium-hexafluorphosphat, O-(*1H*-Benzotriazol-1-yl)-*N,N,N',N'-*tetramethyluronium-tetrafluorborat (TBTU), O-(*1H*-Benzotriazol-1-yl)-*N,N,N',N'-*bis(tetramethylen)uronium-tetrafluorborat, O-(7-Azabenzotriazol-1-yl)-*N,N,N',N'-*tetramethyluronium-hexafluorphosphat (HATU), 1-Hydroxybenzotriazol (HOBt) und 4-(4,6-Dimethoxy-1,3,5-triazin-2-yl)-4-methylmorpholiniumsalz (DMT.MM), meistens als Chlorid verfügbar. Diese Reagenzien können separat oder gegebenenfalls in Kombination eingesetzt werden.

Als Säureakzeptor kommen alle üblichen anorganischen oder organischen Basen infrage, beispielsweise Triethylamin, Diisopropylethylamin, *N*-Methylmorpholin oder *N,N-*Dimethylaminopyridin. Das erfindungsgemäße Verfahren A wird gegebenenfalls in Gegenwart eines geeigneten Reaktionshilfstoffes wie beispielsweise *N,N*-Dimethylformamid oder *N,N-*Dimethylaminopyridin durchgeführt. Als Lösungs- oder Verdünnungsmittel kommen alle interten organischen Lösungsmittel in Betracht, beispielsweise aliphatische oder aromatische Kohlenwasserstoffe (wie Petrolether, Toluol), halogenierte Kohlenwasserstoffe (wie Chlortoluol, Dichlormethan, Chloroform, 1,2-Dichlorethan), Ether (wie Diethylether, Dioxan, Tetrahydrofuran, 1,2-Dimethoxyethan), Ester (wie Essigsäureethylester- oder -methylester), Nitrokohlenwasserstoffe (wie Nitromethan, Nitroethan, Nitrobenzol), Nitrile (wie Acetonitril, Benzonitril), Amide (wie *N,N-*Dimethylformamid, *N,N*-Dimethylacetamid, *N*-Methylformanilid, *N*-Methylpyrrolidon, Hexamethylphosphorsäuretriamid) sowie Dimethylsulfoxid oder Wasser oder Gemische der genannten Lösungsmittel.

Es können auch gemischte Anhydride zur Darstellung von Verbindungen der Formel (III) verwendet werden (vgl. J. Am. Chem. Soc. 1967, 5012). Bei diesem Verfahren können verschiedene Chlorameisensäureester zum Einsatz kommen, wie z.B. Chlorameisensäureisobutylester und Chlorameisensäureisopropylester. Ebenfalls können dafür Diethylacetylchlorid, Trimethylacetylchlorid und ähnliche verwendet werden.

In einem zweiten Syntheseschritt können die Carbonsäureamide der Formeln (IIa) bzw. (IIb) zu den Verbindungen der Formeln (Ia) bzw. (Ib) cyclisiert werden.

Für Q = C-R³, worin R³ für H oder Alkyl steht, kann die Cyclisierung von Carbonsäurenamiden der Formeln (IIa) bzw. (IIb) mit einem Orthoester, wie Orthoameisensäuretriethylester oder Orthoessigsäuretriethylester, gegebenenfalls in Gegenwart eines Lösungs- und Verdünnungsmittel (beispielsweise in Gegenwart von Alkoholen wie Ethanol, aber auch in Gegenwart von *N,N-*Dimethylformamid oder *N,N*-Dimethylacetamid), gegebenenfalls in Gegenwart einer organischen Säure (zum Beispiel *para*-Toluolsulfonsäure oder Essigsäure) oder anorganischen Säure (zum Beispiel Salzsäure oder Schwefelsäure) in katalytischen oder stöchiometrischen Mengen oder im Überschuss durchgeführt werden. Die genannten Säuren können auch anstelle des Lösungs- oder Verdünnungsmittels eingesetzt werden. Für R³ = H finden sich Beispiele für solche Reaktionen mit Orthoameisensäuretriethylester in Archiv der Pharmazie 2000, 333(8), 261-266 (zur Herstellung von Chinazolinonen), J. Het. Chem. 1990, 27(7), 1953-1956 (idem.), WO2010/54398 (zur Herstellung von Pyrazinopyrimidinonen). Für R³ = Methyl siehe zum Beispiel WO2010/100189 (zur Herstellung von Chinazolinonen).

Für Q = C-R³, worin R³ für Alkyl oder Haloalkyl steht, kann die Herstellung der Pyrazolopyrimidinone der Formeln (Ia) bzw. (Ib) auch durch Umsetzung der Carbonsäurenamide der Formeln (IIa) bzw. (IIb) mit entsprechenden Carbonsäurehalogeniden oder Carbonsäurenanhydriden nach literaturbekannten Methoden erfolgen, wie zum Beispiel in WO2009/143049 für R³ = Methyl und in WO 2008/039489 für R³ = Trifluormethyl beschrieben.

Für Q = N können die Pyrazolopyrimidinone der Formeln (Ia) bzw. (Ib) durch Azodiazotierung der Carbonsäureamide der Formeln (IIa) bzw. (IIb) nach literaturbekannten Methoden hergestellt werden. Beispielsweise werden Verbindungen der Formeln (IIa) bzw. (IIb) bei 0 bis 5 °C mit einer Nitritquelle, wie Natriumnitrit oder Isobutylnitrit versetzt, typischerweise in Wasser, Alkohol oder einem polaren, inerten Lösungsmittel und in Gegenwart einer organischen oder anorganischen Säure. Beispiele für Reaktionsbedingungen finden sich in WO 2004/242572 oder in J. Chem. Soc. Perkin Trans. 1, 1980, 633-638.

Die im Verfahren A benötigten Aminopyrazole der Formeln (IIIa) und (IIIb) können beispielweise nach den Verfahren B, C und D hergestellt werden.

### Verfahren B

Aminopyrazole der Formeln (IIIa) und (IIIb) können in einem Schritt, zum Beispiel mittels einer Ullmann Reaktion, nach im Prinzip bekannten Methoden (vgl. Chem. Rev. 2008, 108, 3054-3131) aus den entsprechenden Bromiden der Formel (IV) und den Aminopyrazolen der Formel (V) hergestellt werden.

Beispiele für die Arylierung von Aminopyrazolen sind in WO2007/039146 beschrieben. Für derartige Reaktionen werden beispielsweise Katalysatoren auf Kupfer(I)-Basis (z.B. Kupfer(I)iodid) benutzt, in Anwesenheit einer Base (z.B. Kaliumcarbonat), und eines Ligandes (z.B. *trans-*1,2-Diaminocyclohexan oder *trans*-*N,N'*-Dimethyl-1,2-cyclohexandiamin) oder einer Kombination davon, in einem geeignetem Lösungsmittel (z.B. Dioxan, *N,N*-Dimethylformamid, *N,N-*Dimethylacetamid oder Pyridin) oder einer Kombination von Lösungsmitteln. Für die Reaktion werden meistens Temperaturen zwischen 80 und 180°C benötigt.

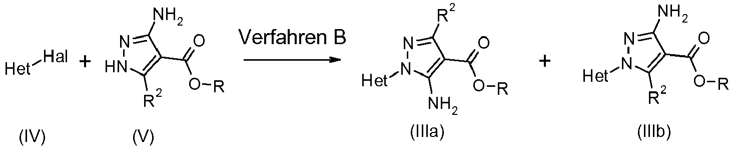

Bei der Durchführung des erfindungsgemäßen Verfahrens B kann jede für diese Reaktionen geeignete, handelsübliche Mikrowellenapparatur verwendet werden (z.B. Anton Paar Monowave 300, CEM Discover S, Biotage Initiator 60).

Die so hergestellten Aminopyrazole der Formeln (IIIa) und (IIIb) können getrennt werden, zum Beispiel mittels chromatographischer Trennung über Kieselgel oder RP(C-18) oder durch Verrühren oder Umkristallisieren unter Verwendung geeigneter Lösungsmittel.

### Verfahren C

Alternativ können Aminopyrazole der Formel (IIIa) nach im Prinzip bekannten Methoden aus Hydrazinen der Formel (VI) oder deren Salzen (bevorzugt sind Hydrochloride) hergestellt werden. Zum Beispiel nach Robins' Synthesen in J. Amer. Chem. Soc. 1956, 78, 784-790 oder nach J. Med. Chem. 2005, 48, 5162-5174, indem sie bei einer Temperatur zwischen 50 °C und 100°C mit einer geeigneten Cyanoverbindung der Formel (IX), in welcher R und R" für Alkyl (bevorzugt Methyl und Ethyl) stehen, umgesetzt werden, gegebenenfalls in einem inerten organischen Lösungsmittel (z.B. Alkohole).

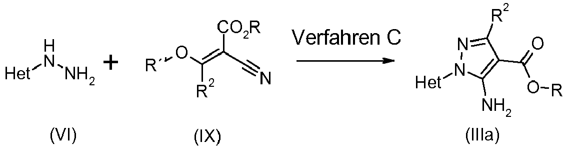

### Verfahren D

Alternativ zu Verfahren B können Aminopyrazole der Formel (IIIb) nach literaturbekannten Methoden aus Hydrazinen der Formel (VI) oder deren Salzen (bevorzugt Hydrochloride) hergestellt werden (vgl. E. J. Med. Chem. 2011, 46, 3867-3876).

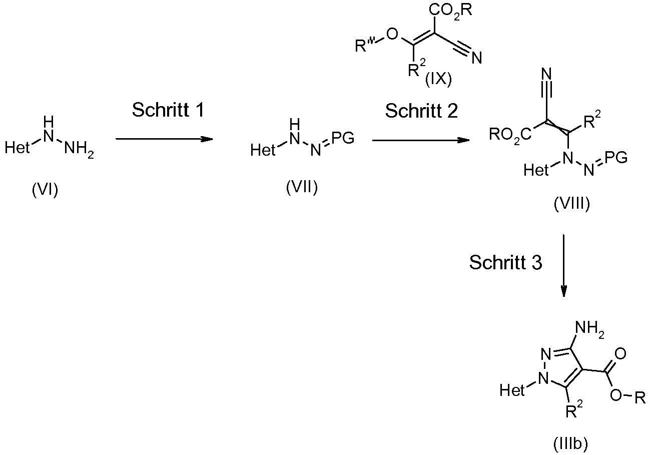

Im Schritt 1 wird das Hydrazin der Formel (VI) geschützt, z.B. durch Reaktion mit einem Aldehyd (typischerweise Benzaldehyd, vgl. Biorg. Med. Chem. Let 2004, 14, 4585-4589) unter Bildung eines Hydrazons. Die geschützte Verbindung der Formel (VII) kann mit einem Cyanoderivat der Formel (IX) zu Verbindungen der Formel (VIII) umgesetzt werden (vgl. J. Het. Chem. 1990, 27, 1805-1807), typischerweise in einem inerten organischen Lösungsmittel (z.B. einem Alkohol) bei einer Temperatur zwischen 50°C und 100°C (Schritt 2). Anschließende Umsetzung von Verbindungen der Formel (VIII) mit einer Säure (z.B. Salzsäure) führt zum Aminopyrazol der Formel (IIIb) (Schritt 3).

Halogenide der Formel (IV) (bevorzugt Chloride, Bromide und Iodide) sind kommerziell erhältlich oder können nach literaturbekannten Methoden synthetisiert werden, vgl. beispielsweise Y. Yamamoto, Heterocycles 1981, 16 (7), 1161-1164, für 3-Iodpyridin (Hal = Iod); S. M. E. Englert, J. Amer. Chem. Soc. 1929, 51(3), 863-866, für 3-Brompyridin (Hal = Brom); D. E. Pearson, J. Org. Chem. 1961, 26, 789-792, und für 3-Chlorpyridin (Hal = Chlor); WO 2006/074884 für 3-Brom-5-fluorpyridin und M. Schlosser, Eur. J. Org. Chem. 2002, 24, 4174-4180, für 3-Fluor-5-iodpyridin; D. Kikelj, U. Urleb, Science of Synthesis 2002, 11, 627-833, für 5-Iodthiazol (Hal = Iod) und WO 2008/057336 für 5-Bromthiazol (Hal = Brom); D. W. Brown, M. Sainsbury, Science of Synthesis 2002, 11, 507-572, für 4-Iodisothiazol (Hal = Iod), I. F. Huebenett et al., Angew. Chem. 1963, 75 (24), 1189-1193, für 4-Bromisothiazol (Hal = Brom) oder 4-Chlorisothiazol (Hal = Chlor); WO 2010/083283 für 4-Iod-1H-pyrazol (Hal = Iod), WO2010/018481 für 4-Brom-1H-pyrazol (Hal = Brom); WO 2001/034137 für 4-Iod-1-methyl-1H-pyrazol (Hal = Iod), WO 93/21186 für 4-Brom-1-methyl-1H-pyrazol (Hal = Brom); WO 2010/090290 für 1-Cyclopropyl-4-iod-1H-pyrazol (Hal = Iod), WO 2008/088692 für 1-(Difluormethyl)-4-iod-1H-pyrazol und 4-Iod-(1,1,2,2-tetrafluorethal)-1H-pyrazol (Hal = Iod); A. Seggio et al., J. Org. Chem. 2007, 72 (17), 6602-6605, für 4-Iodpyridazin (Hal = Iod) und JP 63250385 für 4-Brompyridazin (Hal = Brom).

Heterocyclische Hydrazine der Formel (VI) sind kommerziell erhältlich oder können nach literaturbekannten Methoden aus den entsprechenden Halogeniden der Formel (IV) hergestellt werden, wie z.B. in WO 2010/015849 für diverse Heterocyclen beschrieben: durch Reaktion mit Hydrazinhydrat, gegebenenfalls in einem inerten organischen Lösungsmittel (z.B. Ethanol), bei Temperaturen zwischen 60 und 120 °C; oder durch Reaktion mit Di-*tert*-butyl-hydrazodicarboxylat und anschließender Spaltung der *tert*-Butylcarboxylatgruppen durch Versetzung mit einer Säure (typischerweise Salzsäure in einem organischen Lösungsmittel wie z.B. Dioxan; auch mit Trifluoressigsäure), was zur Bildung der entsprechenden Salze führt.

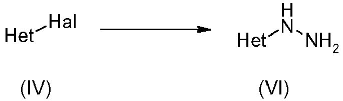

Kommerziell erhältlich sind z.B. folgende Hydrazine der Formel (VI): 3-Hydrazinylpyridin, 5-Hydrazinylpyrimidin, 4-Hydrazinylpyridazin.

Cyanoverbindungen der Formel (IX) können aus Cyanessigsäure-alkylester (bevorzugt -methylester und -ethylester) nach literaturbekannten Methoden hergestellt werden.

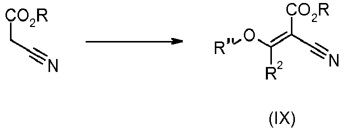

Kommerziell erhältlich sind z.B. Ethyl-2-cyan-3-ethoxyacrylat (R = R" = Ethyl; R² = Wasserstoff), Ethyl-2-cyan-3-ethoxybut-2-enoat - auch Ethyl-2-cyano-3-ethoxycrotonat genannt - (R = R" = Ethyl; R² = Methyl), Ethyl-(2E)-2-cyan-3-ethoxypent-2-enoat (R = R" = R² = Ethyl). Für andere Alkylgruppen kann die Synthese der Cyanoverbindungen der Formel (IX) nach der in J. Amer. Chem. Soc. 1956, 75, 5294-5299 beschriebenen Methode durchgeführt werden. Diese sieht die Umsetzung der entsprechenden Orthoester (z.B. Orthopropionsäuretriethylester für R² = Propyl) mit einem Cyanessigsäure-alkylester bei höheren Temperaturen vor.

### Verfahren E

*N*-Substituierte Pyrazolopyrimidinone der Formel (I) mit Q = C-R³, worin R³ für H oder Alkyl steht, können auch in zwei Schritten aus den Aminopyrazolen der Formel (III) hergestellt werden, wie in folgenden Schemata dargestellt.

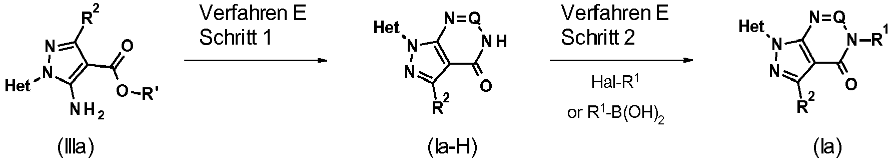

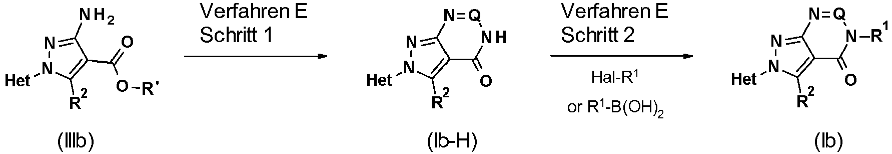

Die Umsetzung der Aminopyrazole der Formeln (IIIa) bzw. (IIIb) zu Pyrazolopyrimidinonen der Formeln (Ia-H) bzw. (Ib-H) durch Reaktion mit Formamidinacetat in Methoxyethanol über Nacht unter Rückfluss ist für Q = C-H in der Literatur bekannt, vgl. US 2007/0281949.

Die *N*-Substitution der Pyrazolopyrimidinone der Formeln (Ia-H) bzw. (Ib-H) kann auf unterschiedliche Weise erfolgen. Literaturbekannt sind *N*-Arylierungen (für R¹ = Aryl, Hetaryl) von Pyrimidinonen durch S_{N}Ar-Reaktion mit einem geeignetem Arylsubstrat, wie z.B. durch Nitro-, Nitril- oder Trifluoromethylgruppen aktivierten Arylfluoriden in Anwesenheit einer Base und eines inerten organischen Lösungsmittels, vgl. Beispiele in DE 4431218. Für unterschiedliche Aryl- und Hetarylverbindungen findet die Umsetzung bevorzugt unter Übergangsmetall-Katalyse oder - Vermittlung statt. Zahlreiche beispielhafte Reaktionsbedingungen sind in der Literatur beschrieben, zum Beispiel in WO2007/146824. Bevorzugt werden Kupfer oder Kupfersalze, zum Beispiel Kupfer(I)-iodid, Kupfer(I)-oxid, Kupfer(I)-triflat oder Kupfer(II)-triflat, als Katalysator verwendet, häufig in Gegenwart von Liganden, zum Beispiel Diamin-Liganden wie *N*,*N*'-Dimethylethylendiamin, *N,N-*Dimethylethylendiamin oder *trans-N,N'*-Dimethyl-1,2-cyclohexandiamin. Eine Übersicht findet sich in Chem. Sci. 2010, Vol. 1, 13-31. Alternativ können 1,3-Diketone, wie z. B. 2,4-Pentandion, 2,2,6,6-Tetramethyl-3,5-heptandion oder Dibenzoylmethan, Aminosäuren, wie z.B. L-Prolin oder Glycin oder andere Verbindungen wie 8-Hydroxychinolin (Tetrahedron Lett. 2009, Vol. 50, 7293-7296), Dibenzylidenaceton, Bipyridin oder Phenanthrolin verwendet werden. In der Regel wird die Umsetzung in Anwesenheit einer Base, häufig Carbonat-oder Phosphat-Basen, wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N,N-*Dimethylformamid durchgeführt. Es können auch Additive wie z. B. Kaliumiodid, Cäsiumfluorid oder andere Salze eingesetzt werden.

Alternativ lassen sich derartige Umsetzungen unter Palladiumkatalyse durchführen, etwa durch Einsatz von Katalysatoren wie Palladiumacetat, Tetrakis(triphenylphosphin)palladium, Bis-(triphenylphosphin)-palladium(II)-chlorid, Tris-(dibenzylidenaceton)-dipalladium(0) in Gegenwart von Liganden, zum Beispiel 2,2'-Bis-(diphenylphosphino)-1,1'-binaphthyl, 9,9-Dimethyl-4,5-bis-(diphenylphosphino)-xanthen, 1,1'-Bis-(diphenylphosphino)-ferrocen, und Basen wie zum Beispiel Kaliumcarbonat, Natriumcarbonat, Cäsiumcarbonat oder Kaliumphosphat, in geeigneten Lösungsmitteln wie zum Beispiel Dioxan, Toluol, Dimethylsulfoxid oder *N,N*-Dimethylformamid.

Im Allgemeinen können Verbindungen der Formeln (Ia) bzw. (Ib) alternativ durch Umsetzung von geeigneten Boronsäuren mit den Pyrazolopyrimidinonen der Formeln (Ia-H) bzw. (Ib-H) hergestellt werden. In der Regel finden die Reaktionen unter Katalyse oder Vermittlung durch Kupfer(II)-salze wie zum Beispiel Kupfer(II)-acetat, Kupfer(II)triflat oder auch durch Kupfer(I)-salze wie zum Beispiel Kupfer(I)-chlorid, Kupfer(I)-acetat unter Luft- oder Sauerstoffatmosphäre, häufig unter wasserentziehenden Bedingungen (zum Beispiel in Gegenwart von Molekularsieb) statt. Als Basen werden zum Beispiel Triethylamin, *N*-Ethyldiisopropylamin, Pyridin, 2,6-Lutidin, *N*-Methylmorpholin oder 1,8-Diazabicycloundec-7-en in geeigneten Lösungmitteln wie zum Beispiel Dichlormethan, Dichlorethan, Methanol, *N,N*-Dimethylformamid, Tetrahydrofuran, Dioxan, Acetonitril, Essigester oder Toluol verwendet. In der Literatur werden zahlreiche Beispiele beschrieben, unter anderem in WO 2008/062905 oder in WO 2009/133970 für Pyrimidinone. Zusammenfassende Übersichten finden sich in Synthesis 2011, No. 6, 829-856 oder in Tetrahedron 2012, Vol. 68, 7735-7754. Anstelle der Boronsäure können auch andere Borverbindungen wie etwa Kaliumtrifluorborate, Boronsäureester etc. sowie andere Organometallverbindungen wie etwa Stannane, Silane oder Bismuthane verwendet werden.

Schließlich wurde gefunden, dass die neuen Verbindungen der Formel (I) stark ausgeprägte biologische Eigenschaften besitzen und vor allem zur Bekämpfung von tierischen Schädlingen, insbesondere von Insekten, Spinnentieren und Nematoden, die in der Landwirtschaft, in den Forsten, im Vorrats- und Materialschutz sowie auf dem Hygienesektor vorkommen, geeignet sind.

Die erfindungsgemäßen Wirkstoffe eignen sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Forsten, in Gärten und Freizeiteinrichtungen, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Anoplura (Phthiraptera) z.B. Damalinia spp., Haematopinus spp., Linognathus spp., Pediculus spp., Trichodectes spp..

Aus der Klasse der Arachnida z.B. Acarus siro, Aceria sheldoni, Aculops spp., Aculus spp., Amblyomma spp., Argas spp., Boophilus spp., Brevipalpus spp., Bryobia praetiosa, Chorioptes spp., Dermanyssus gallinae, Eotetranychus spp., Epitrimerus pyri, Eutetranychus spp., Eriophyes spp., Hemitarsonemus spp., Hyalomma spp., Ixodes spp., Latrodectus mactans, Metatetranychus spp., Oligonychus spp., Ornithodoros spp., Panonychus spp., Phyllocoptruta oleivora, Polyphagotarsonemus latus, Psoroptes spp., Rhipicephalus spp., Rhizoglyphus spp., Sarcoptes spp., Scorpio maurus, Stenotarsonemus spp., Tarsonemus spp., Tetranychus spp., Vasates lycopersici.

Aus der Klasse der Bivalva z.B. Dreissena spp..

Aus der Ordnung der Chilopoda z.B. Geophilus spp., Scutigera spp..

Aus der Ordnung der Coleoptera z.B. Acanthoscelides obtectus, Adoretus spp., Agelastica alni, Agriotes spp., Amphimallon solstitialis, Anobium punctatum, Anoplophora spp., Anthonomus spp., Anthrenus spp., Apogonia spp., Atomaria spp., Attagenus spp., Bruchidius obtectus, Bruchus spp., Ceuthorhynchus spp., Cleonus mendicus, Conoderus spp., Cosmopolites spp., Costelytra zealandica, Curculio spp., Cryptorhynchus lapathi, Dermestes spp., Diabrotica spp., Epilachna spp., Faustinus cubae, Gibbium psylloides, Heteronychus arator, Hylamorpha elegans, Hylotrupes bajulus, Hypera postica, Hypothenemus spp., Lachnosterna consanguinea, Leptinotarsa decemlineata, Lissorhoptrus oryzophilus, Lixus spp., Lyctus spp., Meligethes aeneus, Melolontha melolontha, Migdolus spp., Monochamus spp., Naupactus xanthographus, Niptus hololeucus, Oryctes rhinoceros, Oryzaephilus surinamensis, Otiorrhynchus sulcatus, Oxycetonia jucunda, Phaedon cochleariae, Phyllophaga spp., Popillia japonica, Premnotrypes spp., Psylliodes chrysocephala, Ptinus spp., Rhizobius ventralis, Rhizopertha dominica, Sitophilus spp., Sphenophorus spp., Sternechus spp., Symphyletes spp., Tenebrio molitor, Tribolium spp., Trogoderma spp., Tychius spp., Xylotrechus spp., Zabrus spp..

Aus der Ordnung der Collembola z.B. Onychiurus armatus.

Aus der Ordnung der Dermaptera z.B. Forficula auricularia.

Aus der Ordnung der Diplopoda z.B. Blaniulus guttulatus.

Aus der Ordnung der Diptera z.B. Aedes spp., Anopheles spp., Bibio hortulanus, Calliphora erythrocephala, Ceratitis capitata, Chrysomyia spp., Cochliomyia spp., Cordylobia anthropophaga, Culex spp., Cuterebra spp., Dacus oleae, Dermatobia hominis, Drosophila spp., Fannia spp., Gastrophilus spp., Hylemyia spp., Hyppobosca spp., Hypoderma spp., Liriomyza spp.. Lucilia spp., Musca spp., Nezara spp., Oestrus spp., Oscinella frit, Pegomyia hyoscyami, Phorbia spp., Stomoxys spp., Tabanus spp., Tannia spp., Tipula paludosa, Wohlfahrtia spp.

Aus der Klasse der Gastropoda z.B. Arion spp., Biomphalaria spp., Bulinus spp., Deroceras spp., Galba spp., Lymnaea spp., Oncomelania spp., Succinea spp..

Aus der Klasse der Helminthen z.B. Ancylostoma duodenale, Ancylostoma ceylanicum, Acylostoma braziliensis, Ancylostoma spp., Ascaris lubricoides, Ascaris spp., Brugia malayi, Brugia timori, Bunostomum spp., Chabertia spp., Clonorchis spp., Cooperia spp., Dicrocoelium spp., Dictyocaulus filaria, Diphyllobothrium latum, Dracunculus medinensis, Echinococcus granulosus, Echinococcus multilocularis, Enterobius vermicularis, Faciola spp., Haemonchus spp., Heterakis spp., Hymenolepis nana, Hyostrongulus spp., Loa Loa, Nematodirus spp., Oesophagostomum spp., Opisthorchis spp., Onchocerca volvulus, Ostertagia spp., Paragonimus spp., Schistosomen spp, Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Wuchereria bancrofti.

Weiterhin lassen sich Protozoen, wie Eimeria, bekämpfen.

Aus der Ordnung der Heteroptera z.B. Anasa tristis, Antestiopsis spp., Blissus spp., Calocoris spp., Campylomma livida, Cavelerius spp., Cimex spp., Creontiades dilutus, Dasynus piperis, Dichelops furcatus, Diconocoris hewetti, Dysdercus spp., Euschistus spp., Eurygaster spp., Heliopeltis spp., Horcias nobilellus, Leptocorisa spp., Leptoglossus phyllopus, Lygus spp., Macropes excavatus, Miridae, Nezara spp., Oebalus spp., Pentomidae, Piesma quadrata, Piezodorus spp., Psallus seriatus, Pseudacysta persea, Rhodnius spp., Sahlbergella singularis, Scotinophora spp., Stephanitis nashi, Tibraca spp., Triatoma spp.

Aus der Ordnung der Homoptera z.B. Acyrthosipon spp., Aeneolamia spp., Agonoscena spp., Aleurodes spp., Aleurolobus barodensis, Aleurothrixus spp., Amrasca spp., Anuraphis cardui, Aonidiella spp., Aphanostigma piri, Aphis spp., Arboridia apicalis, Aspidiella spp., Aspidiotus spp., Atanus spp., Aulacorthum solani, Bemisia spp., Brachycaudus helichrysii, Brachycolus spp., Brevicoryne brassicae, Calligypona marginata, Carneocephala fulgida, Ceratovacuna lanigera, Cercopidae, Ceroplastes spp., Chaetosiphon fragaefolii, Chionaspis tegalensis, Chlorita onukii, Chromaphis juglandicola, Chrysomphalus ficus, Cicadulina mbila, Coccomytilus halli, Coccus spp., Cryptomyzus ribis, Dalbulus spp., Dialeurodes spp., Diaphorina spp., Diaspis spp., Doralis spp., Drosicha spp., Dysaphis spp., Dysmicoccus spp., Empoasca spp., Eriosoma spp., Erythroneura spp., Euscelis bilobatus, Geococcus coffeae, Homalodisca coagulata, Hyalopterus arundinis, Icerya spp., Idiocerus spp., Idioscopus spp., Laodelphax striatellus, Lecanium spp., Lepidosaphes spp., Lipaphis erysimi, Macrosiphum spp., Mahanarva fimbriolata, Melanaphis sacchari, Metcalfiella spp., Metopolophium dirhodum, Monellia costalis, Monelliopsis pecanis, Myzus spp., Nasonovia ribisnigri, Nephotettix spp., Nilaparvata lugens, Oncometopia spp., Orthezia praelonga, Parabemisia myricae, Paratrioza spp., Parlatoria spp., Pemphigus spp., Peregrinus maidis, Phenacoccus spp., Phloeomyzus passerinii, Phorodon humuli, Phylloxera spp., Pinnaspis aspidistrae, Planococcus spp., Protopulvinaria pyriformis, Pseudaulacaspis pentagona, Pseudococcus spp., Psylla spp., Pteromalus spp., Pyrilla spp., Quadraspidiotus spp., Quesada gigas, Rastrococcus spp., Rhopalosiphum spp., Saissetia spp., Scaphoides titanus, Schizaphis graminum, Selenaspidus articulatus, Sogata spp., Sogatella furcifera, Sogatodes spp., Stictocephala festina, Tenalaphara malayensis, Tinocallis caryaefoliae, Tomaspis spp., Toxoptera spp., Trialeurodes vaporariorum, Trioza spp., Typhlocyba spp., Unaspis spp., Viteus vitifolii.

Aus der Ordnung der Hymenoptera z.B. Diprion spp., Hoplocampa spp., Lasius spp., Monomorium pharaonis, Vespa spp..

Aus der Ordnung der Isopoda z.B. Armadillidium vulgare, Oniscus asellus, Porcellio scaber.

Aus der Ordnung der Isoptera z.B. Reticulitermes spp., Odontotermes spp..

Aus der Ordnung der Lepidoptera z.B. Acronicta major, Aedia leucomelas, Agrotis spp., Alabama argillacea, Anticarsia spp., Barathra brassicae, Bucculatrix thurberiella, Bupalus piniarius, Cacoecia podana, Capua reticulana, Carpocapsa pomonella, Cheimatobia brumata, Chilo spp., Choristoneura fumiferana, Clysia ambiguella, Cnaphalocerus spp., Earias insulana, Ephestia kuehniella, Euproctis chrysorrhoea, Euxoa spp., Feltia spp., Galleria mellonella, Helicoverpa spp., Heliothis spp., Hofmannophila pseudospretella, Homona magnanima, Hyponomeuta padella, Laphygma spp., Lithocolletis blancardella, Lithophane antennata, Loxagrotis albicosta, Lymantria spp., Malacosoma neustria, Mamestra brassicae, Mocis repanda, Mythimna separata, Oria spp., Oulema oryzae, Panolis flammea, Pectinophora gossypiella, Phyllocnistis citrella, Pieris spp., Plutella xylostella, Prodenia spp., Pseudaletia spp., Pseudoplusia includens, Pyrausta nubilalis, Spodoptera spp., Thermesia gemmatalis, Tinea pellionella, Tineola bisselliella, Tortrix viridana, Trichoplusia spp..

Aus der Ordnung der Orthoptera z.B. Acheta domesticus, Blatta orientalis, Blattella germanica, Gryllotalpa spp., Leucophaea maderae, Locusta spp., Melanoplus spp., Periplaneta americana, Schistocerca gregaria.

Aus der Ordnung der Siphonaptera z.B. Ceratophyllus spp., Xenopsylla cheopis.

Aus der Ordnung der Symphyla z.B. Scutigerella immaculata.

Aus der Ordnung der Thysanoptera z.B. Baliothrips biformis, Enneothrips flavens, Frankliniella spp., Heliothrips spp., Hercinothrips femoralis, Kakothrips spp., Rhipiphorothrips cruentatus, Scirtothrips spp., Taeniothrips cardamoni, Thrips spp..

Aus der Ordnung der Thysanura z.B. Lepisma saccharina.

Zu den pflanzenparasitären Nematoden gehören z.B. Anguina spp., Aphelenchoides spp., Belonoaimus spp., Bursaphelenchus spp., Ditylenchus dipsaci, Globodera spp., Heliocotylenchus spp., Heterodera spp., Longidorus spp., Meloidogyne spp., Pratylenchus spp., Radopholus similis, Rotylenchus spp., Trichodorus spp., Tylenchorhynchus spp., Tylenchulus spp., Tylenchulus semipenetrans, Xiphinema spp..

Die erfindungsgemäßen Verbindungen können in bestimmten Konzentrationen bzw. Aufwandmengen auch als Herbizide, Safener, Wachstumsregulatoren oder Mittel zur Verbesserung der Pflanzeneigenschaften, oder als Mikrobizide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich auch als Zwischen- oder Vorprodukte für die Synthese weiterer Wirkstoffe einsetzen.

Die Wirkstoffe können in die üblichen Formulierungen überführt werden, wie Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoffimprägnierte Naturstoffe, Wirkstoff-imprägnierte synthetische Stoffe, Düngemittel sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsysulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Papier, Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Alkohol-POE- und/oder POP-Ether, Säure- und/oder POP- POE-Ester, Alkyl-Aryl- und/oder POP-POE-Ether, Fett- und/oder POP- POE-Addukte, POE- und/oder POP-Polyol Derivate, POE- und/oder POP-Sorbitan- oder-Zucker-Addukte, Alky- oder Aryl-Sulfate, Sulfonate und Phosphate oder die entsprechenden PO-Ether-Addukte. Ferner geeignete Oligo- oder Polymere, z.B. ausgehend von vinylischen Monomeren, von Acrylsäure, aus EO und/oder PO allein oder in Verbindung mit z.B. (poly-) Alkoholen oder (poly-) Aminen. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel.

Die Formulierungen enthalten im allgemeinen zwischen 0,01 und 98 Gew.-% Wirkstoff, vorzugsweise zwischen 0,5 und 90 %.

Die erfindungsgemäßen Wirkstoffe können als solche oder in ihren Formulierungen auch in Mischung mit einem oder mehreren geeigneten Fungiziden, Bakteriziden, Akariziden, Nematiziden, Insektiziden, Mikrobiziden, Düngemittel, Lockstoffen, Sterilantien, Synergisten, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des weiteren können solche Kombinationen das Pflanzenwachstum verbessern, die Toleranz gegenüber hohen oder niedrigen Temperaturen, gegen Trockenheit oder gegen Wasser- bzw. Bodensalzgehalt erhöhen, die Blühleistung steigern, die Ernte erleichtern und Ernteerträge steigern, die Reife beschleunigen, die Qualität und/oder den Ernährungswert der Ernteprodukte steigern, die Lagerfähigkeit verlängern und/oder die Bearbeitbarkeit der Ernteprodukte verbessern. In der Regel erhält man durch Kombination der erfindungsgemäßen Wirkstoffe und Mischpartnern synergistische Effekte, d.h. die Wirksamkeit der jeweiligen Mischung ist größer als die Wirksamkeit der Einzelkomponenten. Generell können die Kombinationen sowohl als Saatgutanwendungen als auch in Vor-, Tank- oder Fertigmischungen verwendet werden.

Besonders günstige Mischungspartner sind z.B. die folgenden

### Insektizide / Akarizide / Nematizide:

Die hier mit ihrem "common name" genannten Wirkstoffe sind bekannt und beispielsweise im Pestizidhandbuch ("The Pesticide Manual" 14th Ed., British Crop Protection Council 2006) beschrieben oder im Internet recherchierbar (z.B. http://www.alanwood.net/pesticides).
(1) Acetylcholinesterase (AChE) Inhibitoren, wie beispielsweise
   Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb; oder
   Organophosphate, z.B. Acephate, Azamethiphos, Azinphos (-methyl, -ethyl), Cadusafos, Chlorethoxyfos, Chlorfenvinphos, Chlormephos, Chlorpyrifos (-methyl), Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion (-methyl), Phenthoate, Phorate, Phosalone, Phosmet, Phosphamidon, Phoxim, Pirimiphos (-methyl), Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise
   Organochlorine, z.B. Chlordane und Endosulfan (alpha-); oder
   Fiprole (Phenylpyrazole), z.B. Ethiprole, Fipronil, Pyrafluprole und Pyriprole.
(3) Natrium-Kanal-Modulatoren / Spannungsabhängige Natrium-Kanal-Blocker, wie beispielsweise
   Pyrethroide, z.B. Acrinathrin, Allethrin (d-cis-trans, d-trans), Bifenthrin, Bioallethrin, Bioallethrin-S-cyclopentenyl, Bioresmethrin, Cycloprothrin, Cyfluthrin (beta-), Cyhalothrin (gamma-, lambda), Cypermethrin (alpha-, beta-, theta-, zeta-), Cyphenothrin [(1*R*)-*trans*-Isomere], Deltamethrin, Dimefluthrin, Empenthrin [(*EZ*)-(1*R*)-Isomere], Esfenvalerate, Etofenprox, Fenpropathrin, Fenvalerate, Flucythrinate, Flumethrin, Fluvalinate (tau-), Halfenprox, Imiprothrin, Metofluthrin, Permethrin, Phenothrin [(1*R*)-trans-Isomer], Prallethrin, Profluthrin, Pyrethrine (pyrethrum), Resmethrin, RU 15525, Silafluofen, Tefluthrin, Tetramethrin [(1R)- Isomere], Tralomethrin, Transfluthrin und ZXI 8901; oder
   DDT; oder Methoxychlor.
(4) Nikotinerge Acetylcholin-Rezeptor-Agonisten, wie beispielsweise
   Neonikotinoide, z.B. Acetamiprid, Clothianidin, Dinotefuran, Imidacloprid, Nitenpyram, Thiacloprid, Thiamethoxam; oder
   Nikotin.
(5) Allosterische Acetylcholin-Rezeptor-Modulatoren (Agonisten), wie beispielsweise
   Spinosyne, z.B. Spinetoram und Spinosad.
(6) Chlorid-Kanal-Aktivatoren, wie beispielsweise
   Avermectine/Milbemycine, z.B. Abamectin, Emamectin-benzoate, Lepimectin und Milbemectin.
(7) Juvenilhormon-Analoge, z.B. Hydroprene, Kinoprene, Methoprene; oder Fenoxycarb; Pyriproxyfen.
(8) Wirkstoffe mit unbekannten oder nicht spezifischen Wirkmechanismen, wie beispielsweise Begasungsmittel, z.B. Methylbromid und andere Alkylhalogenide; oder
   Chloropicrin; Sulfurylfluorid; Borax; Brechweinstein.
(9) Selektive Fraßhemmer, z.B. Pymetrozine; oder Flonicamid.
(10) Milbenwachstumsinhibitoren, z.B. Clofentezine, Diflovidazin, Hexythiazox, Etoxazole.
(11) Mikrobielle Disruptoren der Insektendarmmembran, wie beispielsweise *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis,* und BT-Pflanzen-Proteine, z.B. Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron; oder
   Organozinnverbindungen, z.B. Azocyclotin, Cyhexatin, Fenbutatin oxide; oder
   Propargite; Tetradifon.
(13) Entkoppler der oxidativen Phoshorylierung durch Unterbrechung des H-Protongradienten, wie beispielsweise Chlorfenapyr und DNOC.
(14) Nikotinerge Acetylcholin-Rezeptor-Antagonisten, wie beispielsweise Bensultap, Cartap (-Hydrochlorid), Thiocylam, und Thiosultap (-sodium).
(15) Inhibitoren der Chitinbiosynthese, Typ 0, wie beispielsweise Benzoylharnstoffe, z.B. Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsstörende Wirkstoffe, wie beispielsweise Cyromazine.
(18) Ecdysonagonisten/-disruptoren, wie beispielsweise
   Diacylhydrazine, z.B. Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon; Acequinocyl; Fluacrypyrim.
(21) Komplex-I-Elektronentransportinhibitoren, beispielsweise aus der Gruppe der METI-Akarizide, z.B. Fenazaquin, Fenpyroximate, Pyrimidifen, Pyridaben, Tebufenpyrad, Tolfenpyrad; oder
   Rotenone (Derris).
(22) Spannungsabhängige Natriumkanal-Blocker, z.B. Indoxacarb; Metaflumizone.
(23) Inhibitoren der Acetyl-CoA-Carboxylase, wie beispielsweise Tetronsäure-Derivate, z.B. Spirodiclofen und Spiromesifen; oder Tetramsäure-Derivate, z.B. Spirotetramat.
(24) Komplex-IV-Elektronentransportinhibitoren, wie beispielsweise Phosphine, z.B. Aluminiumphosphid, Kalziumphosphid, Phosphin, Zinkphosphid; oder Cyanid.
(25) Komplex-II-Elektronentransportinhibitoren, wie beispielsweise Cyenopyrafen.
(28) Ryanodinrezeptor-Effektoren, wie beispielsweise Diamide, z.B. Flubendiamide, Chlorantraniliprole (Rynaxypyr), Cyantraniliprole (Cyazypyr) sowie 3-Brom-N-{2-brom-4-chlor-6-[(1-cyclopropylethyl)carbamoyl]phenyl}-1-(3-chlorpyridin-2-yl)-1H-pyrazol-5-carboxamid (bekannt aus WO2005/077934) oder Methyl-2-[3,5-dibrom-2-({[3-brom-1-(3-chlorpyridin-2-yl)-1H-pyrazol-5-yl]carbonyl}amino)benzoyl]-1,2-dimethylhydrazincarboxylat (bekannt aus WO2007/043677).

Weitere Wirkstoffe mit unbekanntem Wirkmechanismus, wie beispielsweise Azadirachtin, Amidoflumet, Benzoximate, Bifenazate, Chinomethionat, Cryolite, Cyflumetofen, Dicofol, Fluensulfone (5-chlor-2-[(3,4,4-trifluorbut-3-en-1-yl)sulfonyl]-1,3-thiazole), Flufenerim, Pyridalyl und Pyrifluquinazon; desweiteren Präparate auf Basis von Bacillus firmus (I-1582, BioNeem, Votivo) sowie folgende bekannte wirksame Verbindungen

4-{[(6-Brompyrid-3-yl)methyl](2-fluorethyl)amino}furan-2(5H)-on (bekannt aus WO 2007/115644), 4-{[(6-Fluorpyrid-3-yl)methyl](2,2-difluorethyl)amino}furan-2(5H)-on (bekannt aus WO 2007/115644), 4-{[(2-Chlor-1,3-thiazol-5-yl)methyl](2-fluorethyl)amino}furan-2(5H)-on (bekannt aus WO 2007/115644), 4-{[(6-Chlorpyrid-3-yl)methyl](2-fluorethyl)amino}furan-2(5H)-on (bekannt aus WO 2007/ 115644), 4-{[(6-Chlorpyrid-3-yl)methyl](2,2-difluorethyl)amino}furan-2(5H)-on (bekannt aus WO 2007/115644), 4-{[(6-Chlor-5-fluorpyrid-3-yl)methyl](methyl)amino}furan-2(5H)-on (bekannt aus WO 2007/115643), 4-{[(5,6-Dichlorpyrid-3-yl)methyl](2-fluorethyl)amino}furan-2(5H)-on (bekannt aus WO 2007/115646), 4-{[(6-Chlor-5-fluorpyrid-3-yl)methyl](cyclopropyl)amino}furan-2(5H)-on (bekannt aus WO 2007/115643), 4-{[(6-Chlorpyrid-3-yl)methyl](cyclopropyl)amino}furan-2(5H)-on (bekannt aus EP-A-0 539 588), 4-{[(6-Chlorpyrid-3-yl)methyl](methyl)amino}furan-2(5H)-on (bekannt aus EP-A-0 539 588), [1-(6-Chlorpyridin-3-yl)ethyl](methyl)oxido-λ⁴-sulfanylidencyanamid (bekannt aus WO 2007/149134) und seine Diastereomere {[(1*R*)-1-(6-Chlorpyridin-3-yl)ethyl](methyl)oxido-lambda⁶-sulfanyliden}cyanamid (A) und {[(1*S*)-1-(6-Chlorpyridin-3-yl)ethyl](methyl)oxido-lambda⁶-sulfanyliden}cyanamid (B) (ebenfalls bekannt aus WO 2007/149134) sowie Sulfoxaflor (ebenfalls bekannt aus WO 2007/149134) und seine Diastereomere {*(R)*-1-[6-(trifluormethyl)pyridin-3-yl]ethyl}-*(R)-*(methyl)oxido-lambda⁴-sulfanylidenecyanamid (A¹) und {*(S)*-1-[6-(trifluormethyl)pyridin-3-yl]ethyl}-*(S)*-(methyl)oxido-lambda⁴-sulfanylidenecyanamid (A²), bezeichnet als Diastereomerengruppe A (bekannt aus WO 2010/074747, WO 2010/074751), {*(R)*-1-[6-(trifluormethyl)pyridin-3-yl]ethyl}-*(S)*-(methyl)oxido-lambda⁴-sulfanylidene-cyanamid (B¹) und {*(S)*-1-[6-(trifluormethyl)pyridin-3-yl]ethyl}-*(R)*-(methyl)oxido-lambda⁴-sulfanylidenecyanamid (B²), bezeichnet als Diastereomerengruppe B (ebenfalls bekannt aus WO 2010/074747, WO 2010/074751), 11-(4-Chlor-2,6-dimethylphenyl)-12-hydroxy-1,4-dioxa-9-azadispiro[4.2.4.2]tetra-dec-11-en-10-on (bekannt aus WO 2006/089633), 3-(4'-Fluor-2,4-dimethylbiphenyl-3-yl)-4-hydroxy-8-oxa-1-azaspiro[4.5]dec-3-en-2-on (bekannt aus WO 2008/067911),
1-[2-fluor-4-methyl-5-[(2,2,2-trifluorethyl)sulfinyl]phenyl]-3-(trifluormethyl)-1H-1,2,4-Triazol-5-amine (bekannt aus WO 2006/043635),
[(3S,4aR,12R,12aS,12bS)-3-[(Cyclopropylcarbonyl)oxy]-6,12-dihydroxy-4,12b-dimethyl-11-oxo-9-(pyridin-3-yl)-1,3,4,4a,5,6,6a,12,12a,12b-decahydro-2H,11H-benzo[f]pyrano[4,3-b]chromen-4-yl]methylcyclopropancarboxylat (bekannt aus WO 2006/129714),
2-Cyan-3-(difluormethoxy)-N,N-dimethylbenzolsulfonamid (bekannt aus WO2006/056433),
2-Cyan-3-(difluormethoxy)-N-methylbenzolsulfonamid (bekannt aus WO2006/100288), 2-Cyan-3-(difluormethoxy)-N-ethylbenzolsulfonamid (bekannt aus WO2005/035486), 4-(Difluormethoxy)-N-ethyl-N-methyl-1,2-benzothiazol-3-amin-1,1-dioxid (bekannt aus WO2007/057407) und
N-[1-(2,3-Dimethylphenyl)-2-(3,5-dimethylphenyl)ethyl]-4,5-dihydro-1,3-thiazol-2-amin (bekannt aus WO2008/104503).

In einer bevorzugten Ausführungsform der Erfindung wird den Pflanzenschutzmitteln zur Wirkungssteigerung zusätzlich ein Penetrationsförderer zugegeben. Als Penetrationsförderer kommen beispielsweise auch Substanzen in Betracht, die die Verfügbarkeit der Verbindungen der Formel (I) im Spritzbelag fördern. Dazu gehören beispielsweise mineralische oder vegetabile Öle. Als Öle kommen alle üblicherweise in agrochemischen Mitteln einsetzbaren mineralischen oder vegetabilen - gegebenenfalls modifizierte - Öle in Frage. Beispielhaft genannt seien Sonnenblumenöl, Rapsöl, Olivenöl, Rizinusöl, Rüböl, Maiskernöl, Baumwollsaatöl und Sojabohnenöl oder die Ester der genannten Öle. Bevorzugt sind Rapsöl, Sonnenblumenöl und deren Methyl- oder Ethylester, insbesondere Rapsölmethylester.

Die Konzentration an Penetrationsförderer kann in den erfindungsgemäßen Mitteln in einem weiten Bereich variiert werden. Bei einem formulierten Pflanzenschutzmittel liegt sie im allgemeinen bei 1 bis 95 Gew.-%, bevorzugt bei 1 bis 55 Gew.-%, besonders bevorzugt bei 15 - 40 Gew.-%. In den anwendungsfertigen Mitteln (Spritzbrühen) liegen die Konzentration im allgemeinen zwischen 0,1 und 10 g/l, bevorzugt zwischen 0,5 und 5 g/l.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Die erfindungsgemäßen Wirkstoffe können ferner beim Einsatz als Insektizide in ihren handelsüblichen Formulierungen sowie in den aus diesen Formulierungen bereiteten Anwendungsformen in Mischungen mit Hemmstoffen vorliegen, die einen Abbau des Wirkstoffes nach Anwendung in der Umgebung der Pflanze, auf der Oberfläche von Pflanzenteilen oder in pflanzlichen Geweben vermindern.

Der Wirkstoffgehalt der aus den handelsüblichen Formulierungen bereiteten Anwendungsformen kann in weiten Bereichen variieren. Die Wirkstoffkonzentration der Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Beispielhaft seien die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) genannt. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein-oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Die Begriffe "Teile" bzw. "Teile von Pflanzen" oder "Pflanzenteile" wurden oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Zuckerrüben, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Kartoffel, Baumwolle, Tabak und Raps besonders hervorgehoben werden. Als Eigenschaften ("Traits") werden besonders hervorgehoben die erhöhte Abwehr der Pflanzen gegen Insekten, Spinnentiere, Nematoden und Schnecken durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD^{®} (z.B. Mais, Baumwolle, Soja), KnockOut^{®} (z.B. Mais), StarLink^{®} (z.B. Mais), Bollgard^{®} (Baumwolle), Nucotn^{®} (Baumwolle) und NewLeaf^{®} (Kartoffel) vertrieben werden. Als Beispiele für Herbizid-tolerante Pflanzen seien Maissorten, Baumwollsorten und Sojasorten genannt, die unter den Handelsbezeichnungen Roundup Ready^{®} (Toleranz gegen Glyphosate z.B. Mais, Baumwolle, Soja), Liberty Link^{®} (Toleranz gegen Phosphinotricin, z.B. Raps), IMI^{®} (Toleranz gegen Imidazolinone) und STS^{®} (Toleranz gegen Sulfonylharnstoffe z.B. Mais) vertrieben werden. Als Herbizid- resistente (konventionell auf Herbizid-Toleranz gezüchtete) Pflanzen seien auch die unter der Bezeichnung Clearfield^{®} vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufrieden stellend gelöst werden können. So ist es erstrebenswert, Verfahren zum Schutz des Saatguts und der keimenden Pflanze zu entwickeln, die das zusätzliche Ausbringen von Pflanzenschutzmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch Tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. toleranter transgener Pflanzen einbeziehen, um einen optimalen Schutz des Saatguts und der keimenden Pflanze bei einem minimalen Aufwand an Pflanzenschutzmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer Verbindung der Formel (I) behandelt wird. Das erfindungsgemäße Verfahren zum Schutz von Saatgut und keimenden Pflanzen vor dem Befall von Schädlingen umfasst ein Verfahren, in dem das Saatgut gleichzeitig in einem Vorgang mit einem Wirkstoff der Formel (I) und einem oder mehreren Mischungspartnern behandelt wird. Es umfasst auch ein Verfahren, in dem das Saatgut zu unterschiedlichen Zeiten mit einem Wirkstoff der Formel (I) und einem oder mehreren Mischungspartnern behandelt wird.

Die Erfindung bezieht sich ebenfalls auf die Verwendung von Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (I) behandelt wurde. Die Erfindung bezieht sich auch auf Saatgut, welches zur gleichen Zeit mit einem Wirkstoff der Formel (I) und einem oder mehreren Mischungspartner behandelt wurde. Die Erfindung bezieht sich weiterhin auf Saatgut, welches zu unterschiedlichen Zeiten mit einem Wirkstoff der Formel (I) und einem oder mehreren Mischungspartnern behandelt wurde. Bei Saatgut, welches zu unterschiedlichen Zeiten mit einem Wirkstoff der Formel (I) und einem oder mehreren Mischungspartnern behandelt wurde, können die einzelnen Wirkstoffe des erfindungsgemäßen Mittels in unterschiedlichen Schichten auf dem Saatgut enthalten sein. Dabei können die Schichten, die einen Wirkstoff der Formel (I) und den oder die Mischungspartner enthalten, durch eine Zwischenschicht getrennt sein. Die Erfindung bezieht sich auch auf Saatgut, bei dem ein Wirkstoff der Formel (I) und der oder die Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating - Verfahren unterzogen wird, um Staubabrieb am Saatgut zu vermeiden.

Einer der Vorteile der vorliegenden Erfindung ist es, dass aufgrund der besonderen systemischen Eigenschaften der erfindungsgemäßen Mittel die Behandlung des Saatguts mit diesen Mitteln nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Zu nennen ist auch, dass Verbindungen der Formel (I) in Kombination mit Mitteln der Signaltechnologie eingesetzt werden können, wodurch beispielhaft eine bessere Besiedlung mit Symbionten, wie zum Beispiel Rhizobien, Mycorrhiza und/oder endophytischen Bakterien, stattfindet und/oder es zu einer optimierten Stickstofffixierung kommt.

Die erfindungsgemäßen Mittel eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird eine Verbindung der Formel (I) allein oder in einer geeigneten Formulierung auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem Feuchtigkeitsgehalt von unter 15 Gew.-% getrocknet wurde. Alternativ kann auch Saatgut verwendet werden, das nach dem Trocknen z.B. mit Wasser behandelt und dann erneut getrocknet wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agro-chemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorphen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen und Rüben oder auch von Gemüsesaatgut der verschiedensten Natur. Die erfindungsgemäß verwendbaren Beizmittel-Formulierungen oder deren verdünnte Zubereitungen können auch zum Beizen von Saatgut transgener Pflanzen eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt des/der Wirkstoffs/Wirkstoffe in den Formulierungen und nach dem Saatgut. Die Aufwandmengen an Wirkstoffen bzw. Wirkstoffkombinationen liegen im Allgemeinen zwischen 0,001 und 50 g pro Kilogramm Saatgut, vorzugsweise zwischen 0,01 und 15 g pro Kilogramm Saatgut.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ekto- und Endoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp. (Ctenocephalides canis, Ctenocephalides felis), Xenopsylla spp., Ceratophyllus spp..

Aus der Ordnung der Heteropterida z.B. Cimex spp., Triatoma spp., Rhodnius spp., Panstrongylus spp..

Aus der Ordnung der Blattarida z.B. Blatta orientalis, Periplaneta americana, Blattela germanica, Supella spp..

Aus der Unterklasse der Acari (Acarina) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor und bei der Tierhaltung in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Außerdem wurde gefunden, daß die erfindungsgemäßen Verbindungen eine hohe insektizide Wirkung gegen Insekten zeigen, die technische Materialien zerstören.

Beispielhaft und vorzugsweise - ohne jedoch zu limitieren - seien die folgenden Insekten genannt:
Käfer wie Hylotrupes bajulus, Chlorophorus pilosis, Anobium punctatum, Xestobium rufovillosum, Ptilinus pecticornis, Dendrobium pertinex, Ernobius mollis, Priobium carpini, Lyctus brunneus, Lyctus africanus, Lyctus planicollis, Lyctus linearis, Lyctus pubescens, Trogoxylon aequale, Minthes rugicollis, Xyleborus spec. Tryptodendron spec. Apate monachus, Bostrychus capucins, Heterobostrychus brunneus, Sinoxylon spec. Dinoderus minutus;
Hautflügler wie Sirex juvencus, Urocerus gigas, Urocerus gigas taignus, Urocerus augur;
Termiten wie Kalotermes flavicollis, Cryptotermes brevis, Heterotermes indicola, Reticulitermes flavipes, Reticulitermes santonensis, Reticulitermes lucifugus, Mastotermes darwiniensis, Zootermopsis nevadensis, Coptotermes formosanus;
Borstenschwänze wie Lepisma saccharina.

Unter technischen Materialien sind im vorliegenden Zusammenhang nicht-lebende Materialien zu verstehen, wie vorzugsweise Kunststoffe, Klebstoffe, Leime, Papiere und Kartone, Leder, Holz, Holzverarbeitungsprodukte und Anstrichmittel.

Die anwendungsfertigen Mittel können gegebenenfalls noch weitere Insektizide und gegebenenfalls noch ein oder mehrere Fungizide enthalten.

Hinsichtlich möglicher zusätzlicher Zumischpartner sei auf die oben genannten Insektizide und Fungizide verwiesen.

Zugleich können die erfindungsgemäßen Verbindungen zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, eingesetzt werden.

Weiter können die erfindungsgemäßen Verbindungen allein oder in Kombinationen mit anderen Wirkstoffen als Antifouling-Mittel eingesetzt werden.

Die Wirkstoffe eignen sich auch zur Bekämpfung von tierischen Schädlingen im Haushalts-, Hygiene- und Vorratsschutz, insbesondere von Insekten, Spinnentieren und Milben, die in geschlossenen Räumen, wie beispielsweise Wohnungen, Fabrikhallen, Büros, Fahrzeugkabinen u.ä. vorkommen. Sie können zur Bekämpfung dieser Schädlinge allein oder in Kombination mit anderen Wirk- und Hilfsstoffen in Haushaltsinsektizid-Produkten verwendet werden. Sie sind gegen sensible und resistente Arten sowie gegen alle Entwicklungsstadien wirksam. Zu diesen Schädlingen gehören:
Aus der Ordnung der Scorpionidea z.B. Buthus occitanus.

Aus der Ordnung der Acarina z.B. Argas persicus, Argas reflexus, Bryobia ssp., Dermanyssus gallinae, Glyciphagus domesticus, Ornithodorus moubat, Rhipicephalus sanguineus, Trombicula alfreddugesi, Neutrombicula autumnalis, Dermatophagoides pteronissimus, Dermatophagoides forinae.

Aus der Ordnung der Araneae z.B. Aviculariidae, Araneidae.

Aus der Ordnung der Opiliones z.B. Pseudoscorpiones chelifer, Pseudoscorpiones cheiridium, Opiliones phalangium.

Aus der Ordnung der Isopoda z.B. Oniscus asellus, Porcellio scaber.

Aus der Ordnung der Diplopoda z.B. Blaniulus guttulatus, Polydesmus spp..

Aus der Ordnung der Chilopoda z.B. Geophilus spp..

Aus der Ordnung der Zygentoma z.B. Ctenolepisma spp., Lepisma saccharina, Lepismodes inquilinus.

Aus der Ordnung der Blattaria z.B. Blatta orientalies, Blattella germanica, Blattella asahinai, Leucophaea maderae, Panchlora spp., Parcoblatta spp., Periplaneta australasiae, Periplaneta americana, Periplaneta brunnea, Periplaneta fuliginosa, Supella longipalpa.

Aus der Ordnung der Saltatoria z.B. Acheta domesticus.

Aus der Ordnung der Dermaptera z.B. Forficula auricularia.

Aus der Ordnung der Isoptera z.B. Kalotermes spp., Reticulitermes spp.

Aus der Ordnung der Psocoptera z.B. Lepinatus spp., Liposcelis spp.

Aus der Ordnung der Coleoptera z.B. Anthrenus spp., Attagenus spp., Dermestes spp., Latheticus oryzae, Necrobia spp., Ptinus spp., Rhizopertha dominica, Sitophilus granarius, Sitophilus oryzae, Sitophilus zeamais, Stegobium paniceum.

Aus der Ordnung der Diptera z.B. Aedes aegypti, Aedes albopictus, Aedes taeniorhynchus, Anopheles spp., Calliphora erythrocephala, Chrysozona pluvialis, Culex quinquefasciatus, Culex pipiens, Culex tarsalis, Drosophila spp., Fannia canicularis, Musca domestica, Phlebotomus spp., Sarcophaga carnaria, Simulium spp., Stomoxys calcitrans, Tipula paludosa.

Aus der Ordnung der Lepidoptera z.B. Achroia grisella, Galleria mellonella, Plodia interpunctella, Tinea cloacella, Tinea pellionella, Tineola bisselliella.

Aus der Ordnung der Siphonaptera z.B. Ctenocephalides canis, Ctenocephalides felis, Pulex irritans, Tunga penetrans, Xenopsylla cheopis.

Aus der Ordnung der Hymenoptera z.B. Camponotus herculeanus, Lasius fuliginosus, Lasius niger, Lasius umbratus, Monomorium pharaonis, Paravespula spp., Tetramorium caespitum.

Aus der Ordnung der Anoplura z.B. Pediculus humanus capitis, Pediculus humanus corporis, Pemphigus spp., Phylloera vastatrix, Phthirus pubis.

Aus der Ordnung der Heteroptera z.B. Cimex hemipterus, Cimex lectularius, Rhodinus prolixus, Triatoma infestans.

Die Anwendung im Bereich der Haushaltsinsektizide erfolgt allein oder in Kombination mit anderen geeigneten Wirkstoffen wie Phosphorsäureestern, Carbamaten, Pyrethroiden, Neo-nicotinoiden, Wachstumsregulatoren oder Wirkstoffen aus anderen bekannten Insektizidklassen.

Die Anwendung erfolgt in Aerosolen, drucklosen Sprühmitteln, z.B. Pump- und Zerstäubersprays, Nebelautomaten, Foggern, Schäumen, Gelen, Verdampferprodukten mit Verdampferplättchen aus

Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Die folgenden Herstellungs- und Verwendungsbeispiele illustrieren die Erfindung, ohne sie zu beschränken. Die Produkte wurden mittels 1H-NMR Spektroskopie und/oder LC/MS (Liquid Chromatography Mass Spectrometry) charakterisiert.

Die Bestimmung der logP Werte erfolgte gemäß OECD Guideline 117 (EC Directive 92/69/EEC) durch HPLC (High Performance Liquid Chromatography) an reversed-phase (RP) Säulen (C18), mit nachfolgenden Methoden:
[a] Die Bestimmung mit der LC-MS im sauren Bereich erfolgt bei pH 2,7 mit 0,1 % wässriger Ameisensäure und Acetonitril (enthält 0,1% Ameisensäure) als Eluenten; linearer Gradient von 10% Acetonitril bis 95% Acetonitril.
[b] Die Bestimmung mit der LC-MS im neutralen Bereich erfolgt bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit einem Bruker Avance 400, ausgestattet mit einem Durchflussprobenkopf (60 µl Volumen), bestimmt. In Einzelfällen wurden die NMR Spektren mit einem Bruker Avance II 600 gemessen.

Die NMR-Daten ausgewählter Beispiele werden in klassischer Form (δ-Werte, Multiplettaufspaltung, Anzahl der H-Atome) aufgeführt. Die Aufspaltung der Signale wurde wie folgt beschrieben: s (Singulett), d (Duplett), t (Triplett), q (Quartett), quint (Quintuplett), m (Multiplett), b (für breite Signale). Als Lösungsmittel wurden CD₃CN, CDCl₃ oder D6-DMSO verwendet, wobei als Referenz Tetramethylsilan (0.00 ppm) eingesetzt wurde.

### Synthese von Verbindungen der Formel (Ia) nach Verfahren A

### Beispiel 1

### 5-Ethyl-1-(pyridin-3-yl)-1,5-dihydro-4H-pyrazolo[3,4-d]pyrimidin-4-on (6)

### Schritt 1: 5-Amino-N-ethyl-1-(pyridin-3-yl)-1H-pyrazol-4-carboxamid (IIa-1-1)

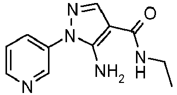

1,50 g (6,46 mmol) Ethyl-5-amino-1-(pyridin-3-yl)-*1H*-pyrazol-4-carboxylat wurden in 10 ml Ethanol und 15 ml Wasser vorgelegt und mit 1,03 g (25,84 mmol) Natriumhydroxid versetzt. Das Reaktionsgemisch wurde über Nacht bei Raumtemperatur und anschließend 2 h bei 40 °C gerührt. Die Reaktionsmischung wurde dann fast zur Trockene eingeengt. Der Rückstand wurde in wenig Wasser angeschlemmt, der unlösliche Anteil wurde abgesaugt und gut unter Vakuum getrocknet. Das so erhaltene Natrium-5-amino-1-(pyridin-3-yl)-*1H*-pyrazol-4-carboxylat wurde direkt weiter umgesetzt.
1H-NMR (D6-DMSO, 400MHz) δ ppm: 8,87(d,1H), 8,50(dd,1H), 8,04-8,01(m,1H), 7,53-7,50(m,2H), 6,41(bs,2H)

150 mg (0,66 mmol, angenommen 100% Reinheit) Natrium-5-amino-1-(pyridin-3-yl)-*1H*-pyrazol-4-carboxylat wurden in 10 ml *N,N*-Dimethylformamid vorgelegt. Dazu wurden 826 mg (2,98 mmol) 4-(4,6-Dimethoxy-1,3,5-triazin-2-yl)-4-methylmorpholiniumchlorid (DMT.MM) und 1,33 ml (2,65 mmol) einer 2M Ethylamin-Lösung in Tetrahydrofuran gegeben. Das Reaktionsgemisch wurde über Nacht bei 50 °C Badtemperatur gerührt und anschließend eingeengt. Der Rückstand wurde in Acetonitril aufgenommen und der unlösliche Teil abfiltriert. Das Filtrat wurde eingeengt und der Rückstand mittels MPLC über einer RP(C-18) Säule mit Acetonitril / Wasser / 0.1% Ameisensäure gereinigt. Man erhielt 89 mg (94% Reinheit, 55% d. Th.) der Titelverbindung.
logP[a]: 0,50; logP[b]: 0,56; 1H-NMR (D6-DMSO, 400MHz) δ ppm: 8,80(d,1H), 8,58(dd,1H), 8,02-7,97(m,2H), 7,91(bt,1H), 7,56(dd,1H), 6,51(bs,2H), 3,29-3,19(m,2H), 1,10(t,3H)

### Schritt 2: 5-Ethyl-1-(pyridin-3-yl)-1,5-dihydro-4H-pyrazolo[3,4-d]pyrimidin-4-on (6)

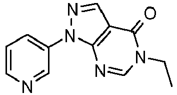

60 mg (0,26 mmol) 5-Amino-*N*-ethyl-1-(pyridin-3-yl)-*1H*-pyrazol-4-carboxamid wurden in 0,40 ml *N,N*-Dimethylacetamid vorgelegt. 22 mg (0,13 mmol) *p*-Toluolsulfonsäure und 58 mg (0,39 mmol) Orthoameisensäuretriethylester wurden dazu gegeben und das Reaktionsgemisch für 1 h in einer CEM Discover Mikrowelle bei 200 Watt auf 120 °C erhitzt. Nach einer Reaktionskontrolle mittels LC/MS wurden 0,05 ml Orthoameisensäuretriethylester zugegeben und 15 Minuten in der Mikrowelle bei 120 °C gerührt. Nach dem Abkühlen wurde das Reaktionsgemisch eingeengt und der Rückstand mittels MPLC über einer RP(C-18) Säule mit Acetonitril / Wasser gereinigt. Man erhielt 25 mg (100% Reinheit, 39% d. Th.) der Titelverbindung.
logP[a]: 0,98; logP[b]: 1,15; 1H-NMR (D6-DMSO, 400MHz) δ ppm: 9,27(d,1H), 8,63-8,60(m,2H), 8,45-8,42(m,2H), 7,64(dd,1H), 4,06(q,2H), 1,29(t,3H)

### Beispiel 2

### 5-Methyl-1-(pyrimidin-5-yl)-1,5-dihydro-4H-pyrazolo[3,4-d]pyrimidin-4-on (8)

### Schritt 1: 5-Amino-N-methyl-1-(pyrimidin-5-yl)-1H-pyrazol-4-carboxamid (IIb-2-1)

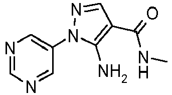

Zu 1,00 g (4,29 mmol) Ethyl-5-amino-1-(pyrimidin-5-yl)-*1H*-pyrazol-4-carboxylat in 30 ml Ethanol gab man 17 ml (17,2 mmol) einer IN Natronlauge. Das Reaktionsgemisch wurde über Nacht bei Raumtemperatur gerührt und anschließend eingeengt. Der Rückstand wurde in wenig Wasser verrührt. Der unlösliche Anteil wurde abgesaugt und gut unter Vakuum getrocknet. Das so erhaltene Natrium-5-amino-1-(pyrimidin-3-yl)-*1H*-pyrazol-4-carboxylat wurde direkt weiter umgesetzt.
1H-NMR (D6-DMSO, 400MHz) δ ppm: 9,10(s,2H), 8,33(s,1H), 7,56(s,1H), 6,57(bs,2H)

Zu 129 mg (0,53 mmol, angenommen 100% Reinheit) Natrium-5-amino-1-(pyrimidin-5-yl)-*1H-*pyrazol-4-carboxylat in 3 ml *N,N*-Dimethylformamid gab man 187 mg (0,58 mmol) (Benzotriazol-1-yloxy)bisdimethylaminomethyliumfluorborat (TBTU). Die resultierende Suspension wurde 10 Min bei Raumtemperatur gerührt. Zu der Lösung wurden 0,29 ml (0,58 mmol) einer 2M Methanamin-Lösung in Tetrahydrofuran und 0,14 ml (0,79 mmol) *N,N*-Diisopropylethylamin gegeben. Das Reaktionsgemisch wurde über Nacht bei Raumtemperatur gerührt und anschließend eingeengt. Der Rückstand wurde mittels MPLC über einer RP(C-18) Säule mit Acetonitril / Wasser / 0.05% Ammoniumformiat aufgereinigt. Man erhielt 22 mg (97% Reinheit, 18% d. Th.) der Titelverbindung.
logP[a]: 0,18; logP[b]: -0,01; 1H-NMR (D6-DMSO, 400MHz) δ ppm: 9,19(s,1H), 9,06(s,2H), 7,99(s,1H), 7,95-7,91(m,1H), 6,66(bs,2H), 2,72(d,3H)

### Schritt 2: 5-Methyl-1-(pyrimidin-5-yl)-1,5-dihydro-4H-pyrazolo[3,4-d]pyrimidin-4-on (8)

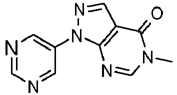

55 mg (0,25 mmol) 5-Amino-*N*-methyl-1-(pyrimidin-5-yl)-*1H*-pyrazol-4-carboxamid wurden in 1 ml *N,N*-Dimethylacetamid vorgelegt. 22 mg (0,13 mmol) *p*-Toluolsulfonsäure und 84 µl (0,50 mmol) Orthoameisensäuretriethylester wurden dazu gegeben und das Reaktionsgemisch für 1 h bei 200 Watt in einer CEM Discover Mikrowelle auf 140 °C erhitzt. Nach einer Reaktionskontrolle mittels LC/MS wurden 85 µl Orthoameisensäuretriethylester zugegeben und das Reaktionsgemisch weitere 60 Min bei 140 °C gerührt. Nach dem Abkühlen wurde das Reaktionsgemisch eingeengt und der Rückstand mittels MPLC über einer RP(C-18) Säule mit Acetonitril / Wasser gereinigt. Man erhielt 16 mg (95% Reinheit, 26% d. Th.) der Titelverbindung.
logP[a]: 0,55; logP[b]: 0,55; 1H-NMR (D6-DMSO, 400MHz) δ ppm: 9,23(s,1H), 8,61(s,1H), 8,52(s,1H), 7,47(d,1H), 7,11(d,1H), 3,54(s,3H)

### Beispiel 3

### 5-Isopropyl-2-(pyridin-3-yl)-2,5-dihydro-4H-pyrazolo[3,4-d]pyrimidin-4-on (15)

### Schritt 1: 3-Amino-N-isopropyl-1-(pyridin-3-yl)-1H-pyrazol-4-carboxamid (IIb-1-2)

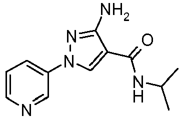

Zu 5,50 ml (64,6 mmol) Isopropylamin in 250 ml 1,2-Dichlorethan wurden 32 ml einer 2M Trimethylaluminium-Lösung in Toluol langsam getropft und 1 h bei Raumtemperatur gerührt. 5,00 g (21,5 mmol) Ethyl-3-amino-1-(pyridin-3-yl)-*1H*-pyrazol-4-carboxylat wurden portionsweise dazu gegeben und das Reaktionsgemisch im Anschluss für 4 h unter Rückfluss erhitzt. Nach dem Abkühlen wurde das Reaktionsgemisch mit Argon vorsichtig auf eine 10%ige Kalium-Natrium-Tartrat-Lösung (ca. 600 ml) gedrückt. Dabei entwickelte sich etwas Wärme. Das Gemisch wurde drei Mal mit Methylenchlorid ausgeschüttelt, die organische Phase abgetrennt, über Natriumsulfat getrocknet, filtriert und eingeengt. Der Rückstand wurde mit etwas Acetonitril zu einer Suspension verrührt. Der unlösliche Teil wurde abgesaugt und unter Vakuum getrocknet. Man erhielt 2,41 g (99% Reinheit, 45% d. Th.) der Titelverbindung.
logP[a]: 0,82; logP[b]: 1,05; 1H-NMR (D6-DMSO, 400MHz) δ ppm: 8,88(d,1H), 8,82(s,1H), 8,44(dd,1H), 7,99-7,96(m,1H), 7,69(bd,1H), 7,51(dd,1H), 5,81(s,2H), 4,06-4,01(m,1H), 1,15(d,6H)

Alternativ konnte die Titelverbindung wie folgt hergestellt werden:
Zu 3,20 g (13,8 mmol) Ethyl-3-amino-1-(pyridin-3-yl)-*1H*-pyrazol-4-carboxylat in 38 ml Ethanol wurden 55 ml (55,1 mmol) einer IN Natronlauge gegeben. Das Reaktionsgemisch wurde über Nacht bei Raumtemperatur gerührt und anschließend eingeengt. Der Rückstand wurde in wenig Wasser verrührt. Der unlösliche Anteil wurde abgesaugt und gut unter Vakuum getrocknet. Das so erhaltene Natrium-3-amino-1-(pyridin-3-yl)-*1H*-pyrazol-4-carboxylat wurde direkt weiter umgesetzt.
   1H-NMR (D6-DMSO, 400MHz) δ ppm: 8,95(d,1H), 8,32(s,2H), 8,06-8,03(m,1H), 7,40(dd,1H)
Zu 1,44 g (6,37 mmol, angenommen 100% Reinheit) Natrium-3-amino-1-(pyridin-3-yl)-*1H*-pyrazol-4-carboxylat in 15 ml *N,N*-Dimethylformamid wurden 2,25 mg (7,00 mmol) (Benzotriazol-1-yloxy)bisdimethylaminomethyliumfluorborat (TBTU) gegeben und die resultierende Suspension bei 10 Min bei Raumtemperatur gerührt. Zu der Lösung wurden 2,17 ml (25,5 mmol) Isopropylamin und 1,66 ml (9,55 mmol) *N,N*-Diisopropylethylamin gegeben. Das Reaktionsgemisch wurde über Nacht bei Raumtemperatur gerührt und anschließend eingeengt. Der Rückstand wurde mittels MPLC über einer RP(C-18) Säule mit Acetonitril / Wasser / 0.1% Ammoniumformiat gereinigt. Man erhielt 732 mg (92% Reinheit, 43% d. Th.) der Titelverbindung.

### Schritt 2: 5-Isopropyl-2-(pyridin-3-yl)-2,5-dihydro-4H-pyrazolo[3,4-d]pyrimidin-4-on (15)

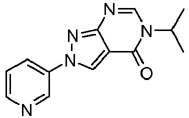

Zu 800 mg (3,26 mmol) 3-Amino-*N*-isopropyl-1-(pyridin-3-yl)-*1H*-pyrazol-4-carboxamid in 15 ml *N,N*-Dimethylacetamid wurden 281 mg (1,63 mmol) *p*-Toluolsulfonsäure und 967 mg (6,52 mmol) Orthoameisensäuretriethylester gegeben. Das Reaktionsgemisch wurde für 1 h bei 200 Watt in einer CEM Discover Mikrowelle auf 130 °C erhitzt. Nach Abkühlen wurde der ausgefallene Feststoff abgesaugt, mit wenig Acetonitril nachgewaschen und unter Vakuum getrocknet. Man erhielt 400 mg (99% Reinheit, 48% d. Th.) der Titelverbindung.
logP[a]: 1,06; logP[b]: 1,12; 1H-NMR (D6-DMSO, 400MHz) δ ppm: 9,47(s,1H), 9,27(d,1H), 8,66(d,1H), 8,46-8,42(m,2H), 7,64(dd,1H), 5,00(quint,1H), 1,42(d,6H)

### Beispiel 4

### 5-Ethyl-2-(pyridin-3-yl)-2,5-dihydro-4H-pyrazolo[3,4-d]pyrimidin-4-on (16)

### Schritt 1: 3-Amino-N-ethyl-1-(pyridin-3-yl)-1H-pyrazol-4-carboxamid (IIb-1-1)

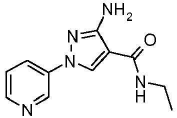

Zu 400 mg (1,77 mmol, angenommen 100% Reinheit) Natrium-3-amino-1-(pyridin-3-yl)-*1H-*pyrazol-4-carboxylat in 10 ml *N,N*-Dimethylformamid wurden 625 mg (1,95 mmol) (Benzotriazol-1-yloxy)bisdimethylaminomethyliumfluorborat (TBTU) gegeben und die resultierende Suspension bei 10 Min bei Raumtemperatur gerührt. Zu der gelblichen Lösung wurden 0,97 ml (1,95 mmol) einer 2M Ethylamin-Lösung in Tetrahydrofuran und 0,46 ml (2,65 mmol) *N,N*-Diisopropylethylamin gegeben. Das Reaktionsgemisch wurde über Nacht bei Raumtemperatur gerührt und anschließend eingeengt. Der Rückstand wurde mittels MPLC über einer RP(C-18) Säule mit Acetonitril / Wasser / 0.05% Ammoniumformiat aufgereinigt. Man erhielt 234 mg (99% Reinheit, 57% d. Th.) der Titelverbindung.
logP[a]: 0,45; logP[b]: 0,77; 1H-NMR (D6-DMSO, 400MHz) δ ppm: 8,88(d,1H), 8,76(s,1H), 8,44(dd,1H), 7,98-7,92(m,2H), 7,52(dd,1H), 5,80(bs,2H), 3,32-3,21(m,2H), 1,12(t,3H)

### Schritt 2: 5-Ethyl-2-(pyridin-3-yl)-2,5-dihydro-4H-pyrazolo[3,4-d]pyrimidin-4-on (16)

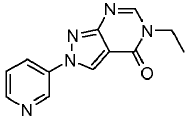

Zu 234 mg (1,01 mmol) 3-Amino-*N*-ethyl-1-(pyridin-3-yl)-*1H*-pyrazol-4-carboxamid in 5 ml *N,N-*Dimethylacetamid wurden 87 mg (0,51 mmol) *p*-Toluolsulfonsäure und 300 mg (2,02 mmol) Orthoameisensäuretriethylester gegeben. Das Reaktionsgemisch wurde für 1 h bei 200 Watt in einer CEM Discover Mikrowelle auf 140 °C erhitzt. Nach Abkühlen wurde das Reaktionsgemisch eingeengt, in wenig Wasser aufgenommen und der Feststoff abgesaugt und unter Vakuum getrocknet. Man erhielt 198 mg (99% Reinheit, 80% d. Th.) der Titelverbindung.
logP[a]: 0,70; logP[b]: 0,84; 1H-NMR (D6-DMSO, 400MHz) δ ppm: 9,47(s, 1H), 9,26(d,1H), 8,65(dd,1H), 8,43(dd,1H), 8,39(s,1H), 7,64(dd,1H), 3,98(q,2H), 1,27(t,3H)

### Beispiel 5

### 5-Ethyl-6-methyl-2-(pyridin-3-yl)-2,5-dihydro-4H-pyrazolo[3,4-d]pyrimidin-4-on (29)

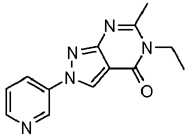

Zu 40 mg (0,17 mmol) 3-Amino-*N*-ethyl-1-(pyridin-3-yl)-*1H*-pyrazol-4-carboxamid in 2 ml *N,N-*Dimethylacetamid wurden 15 mg (0,09 mmol) *p*-Toluolsulfonsäure und 56 mg (0,35 mmol) Orthoessigsäuretriethylester gegeben. Das Reaktionsgemisch wurde für 2 h bei 200 Watt in einer CEM Discover Mikrowelle auf 140 °C erhitzt. Anschließend wurde noch 1 h bei 130 °C gerührt. Nach dem Abkühlen wurde das Reaktionsgemisch eingeengt und der Rückstand mittels MPLC über einer RP(C-18) Säule mit Acetonitril / Wasser gereinigt. Die daraus isolierte Fraktion wurde mittels präparativer HPLC mit Acetonitril / Wasser nochmal gereinigt. Es konnten 6 mg (100% Reinheit, 14% d. Th.) der Titelverbindung isoliert werden.
logP[a]: 0,93; logP[b]: 1,03; 1H-NMR (D6-DMSO, 400MHz) δ ppm: 9,40(s,1H), 9,25(bs,1H), 8,64(bd,1H), 8,40(m,1H), 7,63(dd,1H), 4,06(q,2H), 2,62(s,3H), 1,24(t,3H)

### Beispiel 6

### 5-Ethyl-2-(pyridin-3-yl)-6-(trifluormethyl)-2,5-dihydro-4H-pyrazolo[3,4-d]pyrimidin-4-on (32)

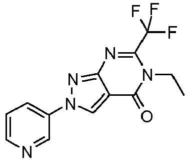

Zu 50 mg (0,22 mmol) 3-Amino-*N*-ethyl-1-(pyridin-3-yl)-*1H*-pyrazol-4-carboxamid und 90 µl (0,65 mmol) Triethylamin in 10 ml Acetonitril wurden bei 0 °C 52 µl (0,37 mmol) Trifluoressigsäureanhydrid langsam zugegeben. Anschließend wurde 2 h bei 0 °C und dann 12 h bei 50 °C gerührt. Nach Zugabe von 52 µl Trifluoressigsäureanhydrid und 90 µl Triethylamin wurde das Gemisch über Nacht unter Rückfluss erhitzt. Nach dem Abkühlen wurde das Reaktionsgemisch eingeengt und der Rückstand mittels MPLC über einer RP(C-18) Säule mit Acetonitril / Wasser gereinigt. Man erhielt 44 mg (95% Reinheit, 62% d. Th.) der Titelverbindung.
logP[a]: 2,07; logP[b]: 2,06; 1H-NMR (D6-DMSO, 400MHz) δ ppm: 9,63(s,1H), 9,29(d,1H), 8,69(bd,1H), 8,45(m,1H), 7,67(dd,1H), 4,10(q,2H), 1,29(t,3H)

### Beispiel 7

### 3-Isopropyl-6-(3-pyridyl)pyrazolo[3,4-d]triazin-4-on (36)

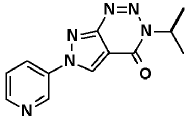

Zu 100 mg (0,41 mmol) 3-Amino-*N*-isopropyl-1-(pyridin-3-yl)-*1H*-pyrazol-4-carboxamid in 2,5 ml Wasser und 2,5 ml konzentrierter Salzsäure wurden 239 mg (3,47 mmol) Natriumnitrit in 2,5 ml Wasser gegeben. Das Reaktionsgemisch wurde über Nacht bei 70 °C gerührt. Nach dem Abkühlen wurde das Reaktionsgemisch auf Wasser ausgetragen und der pH Wert der Lösung wurde mit gesättigter Natriumbicarbonat-Lösung auf 7 eingestellt. Es wurde drei Mal mit Methylenchlorid ausgeschüttelt, die organische Phasen wurden verenigt, über Magnesiumsulfat getrocknet, filtriert und eingeengt. Der Rückstand wurde auf RP(C-18) Material aufgezogen und mittels MPLC über einer RP(C-18) Kartusche mit Laufmittel Wasser / Acetonitril / 0.1% Ameisensäure gereinigt. Man erhielt 6 mg (100% Reinheit, 6% d. Th.) der Titelverbindung
logP[a]: 1,72; logP[b]: 1,70; 1H-NMR (D6-DMSO, 400MHz) δ ppm: 9,71(s,1H), 9,33(d,1H), 8,74(dd,1H), 8,53-8,50(m,1H), 7,72-7,69(m,1H), 5,31(quint,1H), 1,49(d,6H)

### Beispiel 8

### 5-Isopropyl-2-pyridazin-4-yl-pyrazolo[3,4-d]pyrimidin-4-on (38)

### Schritt 1: 3-Amino-N-isopropyl-1-pyridazin-4-yl-pyrazol-4-carboxamid (IIb-3-1)

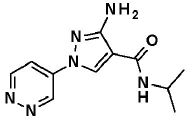

Zu 0,55 ml (6,43 mmol) Isopropylamin in 2,5 ml 1,2-Dichlorethan wurden 3,22 ml einer 2M Trimethylaluminium-Lösung in Toluol langsam getropft und 30 Minuten bei Raumtemperatur gerührt. 500 mg (2,14 mmol) Ethyl-3-amino-1-pyridazin-4-yl-pyrazole-4-carboxylat wurden portionsweise dazu gegeben und das Reaktionsgemisch im Anschluss über Nacht unter Rückfluss erhitzt. Nach dem Abkühlen wurde das Reaktionsgemisch mit Argon vorsichtig auf eine 10%ige Kalium-Natrium-Tartrat-Lösung (ca. 100 ml) gedrückt. Das Gemisch wurde drei Mal mit Methylenchlorid ausgeschüttelt, die organische Phase abgetrennt, über Natriumsulfat getrocknet, filtriert und eingeengt. Der Rückstand wurde auf RP(C-18) Material aufgezogen und mittels MPLC über einer RP(C-18) Kartusche mit Laufmittel Wasser / Acetonitril / 0,1% Ameisensäure gereinigt. Man erhielt 100 mg (88% Reinheit, 17% d. Th.) der Titelverbindung.
logP[a]: 0,74; logP[b]: 0,74; 1H-NMR (D6-DMSO, 400MHz) δ ppm: 9,50(d,1H), 9,19(d,1H), 9,00(s,1H), 7,84(bd,1H), 7,71-7,69(m,1H), 6,04(s,2H), 4,06-4,01(m,1H), 1,17-1,15(d,6H)

### Schritt 2: 5-Isopropyl-2-pyridazin-4-yl-pyrazolo[3,4-d]pyrimidin-4-on (38)

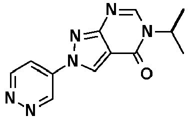

Zu 100 mg (0,41 mmol) 3-Amino-*N*-isopropyl-1-pyridazin-4-yl-pyrazole-4-carboxamid in 3 ml *N*,*N-*Dimethylacetamid wurden 35 mg (0,20 mmol) *p*-Toluolsulfonsäure und 120 mg (0,81 mmol) Orthoameisensäuretriethylester gegeben. Das Reaktionsgemisch wurde für 1 h bei 200 Watt in einer CEM Discover Mikrowelle auf 140 °C erhitzt. Nach dem Abkühlen wurde das Reaktionsgemisch eingeengt, in wenig gesättigter Natriumbicarbonat-Lösung aufgenommen und drei Mal mit Methylenchlorid extrahiert. Die organischen Phasen wurden getrennt, vereinigt, über Natriumsulfat getrocknet, filtriert und eingeengt. Der Rückstand wurde in Acetonitril verrührt, auf RP(C-18) Material aufgezogen und mittels MPLC über einer RP(C-18) Kartusche mit Laufmittel Wasser / Acetonitril / 0,1% Ameisensäure gereinigt. Man erhielt 8 mg (77% Reinheit, 6% d. Th.) der Titelverbindung.
logP[a]: 0,85; logP[b]: 0,85

### Synthese von Aminopyrazolen der Formeln (IIIa) und (IIIb)

### Ethyl-5-amino-1-(pyridin-3-yl)-1H-pyrazol-4-carboxylat (IIIa-1) und Ethyl-3-amino-1-(pyridin-3-yl)-1H-pyrazol-4-carboxylat (IIIb-1) nach Verfahren B

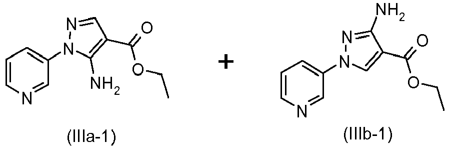

82,3 g (0,59 mol) Kaliumcarbonat wurden in einem Dreihalskolben vorgelegt. Dieser wurde unter Argon ausgeheizt und nacheinander wurden 2,70 g (0,014 mol) Kupfer(I)iodid, 44,0 g (0,28 mol) Ethyl-3-amino-4-pyrazolcarboxylat und 440 ml *N*,*N*-Dimethylacetamid zugegeben. Die Suspension wurde 10 Minuten gerührt und anschließend 7,18 g (0,056 mol) *trans*-1,2-Diaminocyclohexan und 53,77 g (0,34 mol) 3-Brompyridin zugegeben. Das Reaktionsgemisch wurde auf Rückflusstemperatur gebracht und über Nacht bei 145 °C gerührt. Nach dem Abkühlen wurde das Reaktionsgemisch abgesaugt, die Mutterlauge eingeengt und der Rückstand mittels MPLC über einer RP(C-18) Säule mit Acetonitril / Wasser / 0,1% Ameisensäure gereinigt. Eine erste Fraktion enthielt 39 g eines Gemisches von 70% Ethyl-5-amino-1-(pyridin-3-yl)-*1H*-pyrazol-4-carboxylat (IIIa-1) und 30% Ethyl-3-amino-1-(pyridin-3-yl)-*1H*-pyrazol-4-carboxylat (IIIb-1), laut LC/MS. Eine zweite Fraktion enthielt 10 g bestehend aus 83% Ethyl-3-amino-1-(pyridin-3-yl)-*1H*-pyrazol-4-carboxylat (IIIb-1) nach LC/MS. Eine Trennung von (IIIa-1) und (IIIb-1) aus der ersten Fraktion mittels präparativer HPLC lieferte weitere 24,5 g (96% Reinheit) Ethyl-5-amino-1-(pyridin-3-yl)-*1H-*pyrazol-4-carboxylat als Formiat (IIIa-1) und 11,5 g (99% Reinheit) Ethyl-3-amino-1-(pyridin-3-yl)-*1H*-pyrazol-4-carboxylat (IIIb-1).
(IIIa-1).HCOOH logP[a]: 1,18; logP[b]: 1,27; 1H-NMR (D6-DMSO, 400MHz) δ ppm: 12,8(bs,1H), 8,78(d,1H), 8,62(dd,1H), 8,14(s,1H), 7,99-7,96(m,1H), 7,76(s,1H), 7,58(dd,1H), 6,51(bs,2H), 4,23(q,2H), 1,28(t,3H)
(IIIb-1) logP[a]: 1,12; logP[b]: 1,32; 1H-NMR (D6-DMSO, 400MHz) δ ppm: 9,06(d,1H), 8,86(s,1H), 8,47(d,1H), 8,19(bd,1H), 7,49(dd,1H), 5,78(bs,2H), 4,26(q,2H), 1,30(t,3H)

### Ethyl-5-amino-1-(pyridin-3-yl)-1H-pyrazol-4-carboxylat (IIIa-1) nach Verfahren C

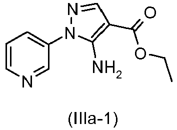

Zu 500 mg (3,43 mmol) 3-Hydrazinopyridinhydrochlorid (1:1) in 5 ml Ethanol wurden 581 mg (3,43 mmol) Ethyl-2-cyan-3-ethoxyacrylat gegeben. Das Reaktionsgemisch wurde für 2 h unter Rückfluss erhitzt, abgekühlt und mit 0,48 ml (3,43 mmol) Triethylamin versetzt. Anschließend wurde eingeengt, der Rückstand in wenig Wasser aufgenommen, leicht angesäuert und mehrmals mit Dichlormethan extrahiert. Die organischen Phasen wurden vereinigt, über Natriumsulfat getrocknet und eingeengt. Der Rückstand wurde mittels MPLC über einer Kieselgel-Säule mit Cyclohexan / Essigester gereinigt. Man erhielt 179 mg (98% Reinheit, 22% d. Th.) der Titelverbindung.
(IIIa-1) logP[a]: 1,10; 1H-NMR (D6-DMSO, 400MHz) δ ppm: 8,78(d,1H), 8,62(dd,1H), 7,99-7,96(m,1H), 7,76(s,1H), 7,58(dd,1H), 6,49(bs,2H), 4,23(q,2H), 1,28(t,3H); 13C-NMR (D6-DMSO, 150MHz) δ ppm: 163,6, 150,6, 148,6, 145,0, 141,2, 134,9, 131,7, 124,5, 95,1, 59,3, 14,7

### Ethyl-3-amino-1-(pyridin-3-yl)-1H-pyrazol-4-carboxylat (IIIb-1) nach Verfahren D

### Schritt 1: Benzaldehydpyridin-3-ylhydrazon (VII-1)

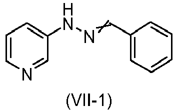

Zu 15,00 g (103,0 mol) 3-Hydrazinopyridinhydrochlorid (1:1) und 9,49 g (68,7 mmol) Kaliumcarbonat in 150 ml Toluol gab man langsam 7 ml (68,7 mmol) Benzaldehyd gelöst in 100 ml Toluol. Das Reaktionsgemisch wurde über Nacht unter Rückfluss (mit Wasserabscheider) gerührt. Nach dem Abkühlen wurden die unlöslichen Anteile abgesaugt. Der feste Rückstand wurde mehrmals in Essigester verrührt. Der Feststoff wurde abgesaugt und mehrmals in Isopropanol heiß verrührt. Die unlöslichen Anteile wurden abgesaugt und verworfen, das Filtrat wurde eingeengt. Die so erhaltenen 5,50 g (98% Reinheit, 40% d. Th.) der Titelverbindung wurden direkt weiter umgesetzt.
logP[a]: 0,86; logP[b]: 2,22

### Schritt 2: Ethyl-3-[2-benzyliden-1-(pyridin-3-yl)hydrazino]-2-cyanacrylat (VIII-1)

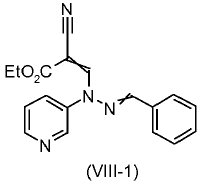

Zu 5,50 g (27,9 mmol) Benzaldehydpyridin-3-ylhydrazon in 14 ml Toluol wurden 4,72 g (27,9 mmol) Ethyl-2-cyan-3-ethoxyacrylat gegeben. Das Reaktionsgemisch wurde zunächst für 2 h und nach Zugabe einer Spatelspitze *para*-Toluolsulfonsäure für weitere 2 h unter Rückfluß erhitzt. Nach dem Abkühlen wurde der ausgefallene Feststoff abgesaugt und die organische Phase verworfen. Der Feststoff wurde nochmal in 15 ml Toluol vorgelegt und mit 1,69 g (10,0 mmol) Ethyl-2-cyan-3-ethoxyacrylat versetzt. Das Reaktionsgemisch wurde über Nacht unter Rückfluß erhitzt, bis zur Abkühlung stehen gelassen und anschließend mit Toluol verdünnt. Nach Zugabe von wenig (ca. 0.5 ml) Acetonitril wurde der unlösliche Rückstand abgesaugt und unter Vakuum getrocknet. Isoliert wurden 3,11 g (90% Reinheit, 31% d. Th.) der Titelverbindung.
logP[a]: 2,54; logP[b]: 2,48

### Schritt 3: Ethyl-3-amino-1-(pyridin-3-yl)-1H-pyrazol-4-carboxylat (IIIb-1)

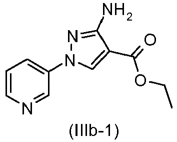

3,00 g (9,37 mmol) Ethyl-3-[2-benzyliden-1-(pyridin-3-yl)hydrazino]-2-cyanacrylat wurden in 11 ml Ethanol vorgelegt und mit 1,1 ml (13,11 mmol) einer 37%igen Salzsäurelösung versetzt. Das Reaktionsgemisch wurde 1 h unter Rückfluß erhitzt und anschließend nach dem Abkühlen eingeengt. Der Rückstand wurde zweimal in lauwarmem Toluol verrührt. Der Feststoff wurde abgesaugt und unter Vakuum getrocknet. Isoliert wurden 2,64 g (92% Reinheit, 97% d. Th.) der Titelverbindung.

### Ethyl-5-amino-1-(pyrimidin-5-yl)-1H-pyrazol-4-carboxylat (IIIa-2) und Ethyl-3-amino-1-(pyrimidin-5-yl)-1H-pyrazol-4-carboxylat (IIIb-2) nach Verfahren B

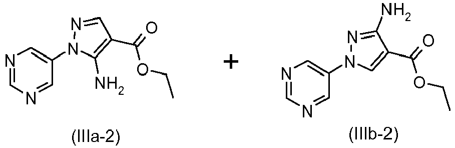

9,35 g (67,6 mmol) Kaliumcarbonat wurden in einem Dreihalskolben vorgelegt. Dieser wurde unter Argon ausgeheizt und nacheinander wurden 0,31 g (1,61 mmol) Kupfer(I)iodid, 5,00 g (32,2 mmol) Ethyl-3-amino-4-pyrazolcarboxylat und 50 ml *N*,*N*-Dimethylacetamid zugegeben. Die Suspension wurde 10 Minuten gerührt, dann wurden 0,77 ml (6,45 mmol) *trans*-1,2-Diaminocyclohexan und 6,15 g (39,7 mmol) 5-Brompyrimidin zugegeben. Das Reaktionsgemisch wurde auf Rückflusstemperatur gebracht und insgesamt 7,5 h bei ca. 150 °C gerührt. Nach dem Abkühlen wurde das Reaktionsgemisch eingeengt und der Rückstand mittels MPLC über einer RP(C-18) Säule mit Acetonitril / Wasser gereinigt. Die isolierte Fraktion enthielt 2,97 g eines Gemisches von 90% Ethyl-5-amino-1-(pyrimidin-5-yl)-*1H*-pyrazol-4-carboxylat (IIIa-2) und 9% Ethyl-3-amino-1-(pyrimidin-5-yl)-*1H*-pyrazol-4-carboxylat (IIIb-2) nach LC/MS. Die Trennung der Produkte erfolgte anschließend mittels präparativer HPLC. Zwei Fraktionen wurden isoliert: 0,99 g (100% Reinheit) Ethyl-5-amino-1-(pyrimidin-5-yl)-*1H*-pyrazol-4-carboxylat (IIIa-2) und 0,42 g (95% Reinheit) Ethyl-3-amino-1-(pyrimidin-5-yl)-*1H*-pyrazol-4-carboxylat (IIIb-2)
(IIIa-2) logP[a]: 1,16; logP[b]: 1,12; 1H-NMR (D6-DMSO, 400MHz) δ ppm: 9,23(s,1H), 9,04(s,2H), 7,81(s,1H), 6,97(bs,2H), 4,23(q,2H), 1,28(t,3H); 13C-NMR (D6-DMSO, 150MHz) δ ppm: 163,4, 156,9, 152,2, 151,1, 142,0, 133,7, 95,2, 59,3, 14,7
(IIIb-2) logP[a]: 1,12; logP[b]: 1,08; 1H-NMR (D6-DMSO, 400MHz) δ ppm: 9,24(s,1H), 9,07(s,1H), 8,96(s,1H), 5,89(bs,2H), 4,27(q,2H), 1,30(t,3H)

### Ethyl- 5-amino-1-pyridazin-4-yl-pyrazol-4-carboxylat (IIIa-3) und Ethyl-3-amino-1-pyridazin-4-yl-pyrazole-4-carboxylat (IIIb-3) nach Verfahren B

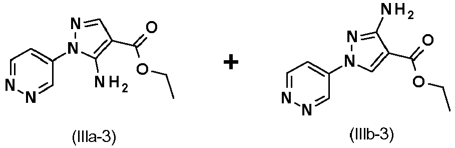

9,35 g (67,7 mmol) Kaliumcarbonat wurden in einem Dreihalskolben vorgelegt. Dieser wurde unter Argon ausgeheizt und nacheinander wurden 0,31 g (1,61 mmol) Kupfer(I)iodid, 5,00 g (32,2 mmol) Ethyl-3-amino-4-pyrazolcarboxylat und 100 ml *N*,*N*-Dimethylacetamid zugegeben. Die Suspension wurde 10 Minuten gerührt, dann wurden 0,86 ml (6,45 mmol) *trans*-1,2-Diaminocyclohexan und 6,15 g (38,7 mmol) 4-Brompyridazin zugegeben. Das Reaktionsgemisch wurde auf Rückflusstemperatur gebracht und insgesamt 7,5 h bei ca. 150 °C gerührt. Nach dem Abkühlen wurde das Reaktionsgemisch filtriert, das Filtrat wurde eingeengt und der Rückstand mittels MPLC über einer RP(C-18) Säule mit Wasser / Acetonitril / 0.1% Ameisensäure gereinigt. Die Fraktionen mit höherem Gehalt wurden in Acetonitril zwecks einer erneuten Trennung aufgenommen, dabei blieb ein unlöslicher Teil zurück. Dieser wurde abgesaugt und getrocknet. Man erhielt 1,18 g (80% Reinheit, 13% d. Th.) des Ethyl-3-amino-1-pyridazin-4-yl-pyrazol-4-carboxylats (IIIb-3).

Das Filtrat wurde auf RP(C-18) Material aufgezogen und mittels MPLC über einer RP(C-18) Säule mit Wasser / Acetonitril / 0.1% Ameisensäure gereinigt. Die isolierte Fraktion enthielt 0,41 g eines Gemisches von 73% Ethyl- 5-amino-1-pyridazin-4-yl-pyrazol-4-carboxylat (IIIa-3) und 13% Ethyl-3-amino-1-pyridazin-4-yl-pyrazol-4-carboxylat (IIIb-3) nach LC/MS.
(IIIa-3) logP[a]: 1,12; logP[b]: 1,12
(IIIb-3) logP[a]: 0,90; logP[b]: 0,94; 1H-NMR (D6-DMSO, 400MHz) δ ppm: 9,75(bs,1H), 9,22(bs,1H), 9,13(s,1H), 7,99-7,96(m,1H), 6,00(s,2H), 4,28(q,2H), 1,31(t,3H)

Weitere Verbindungen der Formeln (Ia) und (Ib) sind in den folgenden Tabellen aufgeführt.

**Tabelle 1**

| | | | | | |
|---|---|---|---|---|---|
| Verbindungen der Formel (Ia) | | | | | |
| 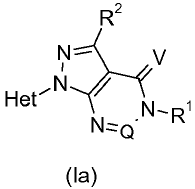 | | | | | |

| **Bsp.-Nr.** | **Het** | **Q** | **V** | **R¹** | **R²** |
|---|---|---|---|---|---|
| 1 | 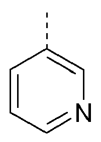 | CH | O | CH(CH₃)₂ | H |
| 2 | 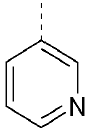 | CH | O | 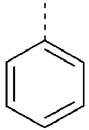 | H |
| 3 | 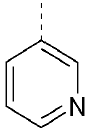 | CH | O | CH₂CF₃ | H |
| 4 | 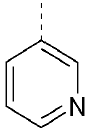 | CH | O | 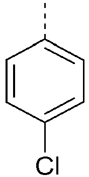 | H |
| 5 | 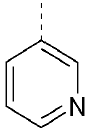 | CH | O | 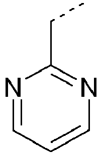 | H |
| 6 | 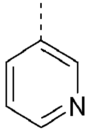 | CH | O | C₂H₅ | H |
| 7 | 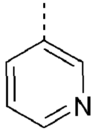 | CH | O | CH₃ | H |
| 8 | 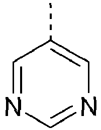 | CH | O | CH₃ | H |

**Tabelle 2**

| | | | | | |
|---|---|---|---|---|---|
| Verbindungen der Formel (Ib) | | | | | |
| 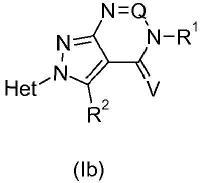 | | | | | |

| **Bsp.-Nr.** | **Het** | **Q** | **V** | **R¹** | **R²** |
|---|---|---|---|---|---|
| 9 | 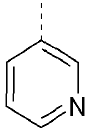 | CH | O | C₂H₅ | H |
| 10 | 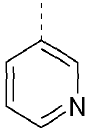 | CH | O | 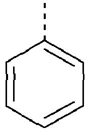 | H |
| 11 | 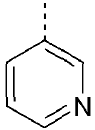 | CH | O | 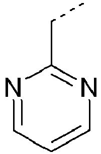 | H |
| 12 | 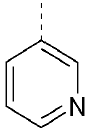 | CH | O | CH₂CF₃ | H |
| 13 | 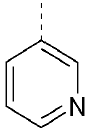 | CH | O | 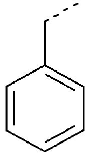 | H |
| 14 | 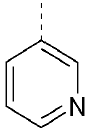 | CH | O | 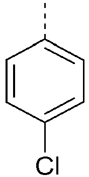 | H |
| 15 | 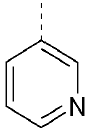 | CH | O | CH(CH₃)₂ | H |
| 16 | 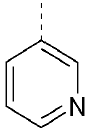 | CH | O | C₂H₅ | H |
| 17 | 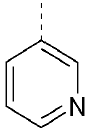 | CH | O | 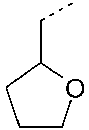 | H |
| 18 | 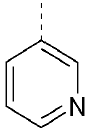 | CH | O | CH₃ | H |
| 19 | 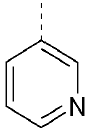 | CH | O | 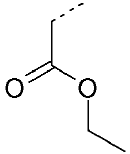 | H |
| 20 | 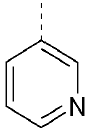 | CH | O | 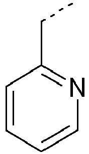 | H |
| 21 | 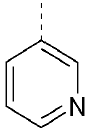 | CH | O | 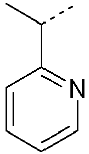 | H |
| 22 | 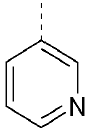 | CH | O | 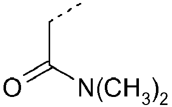 | H |
| 23 | 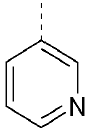 | CH | O | 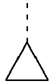 | H |
| 24 | 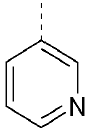 | CH | O | CH₂CF₂CH₃ | H |
| 25 | 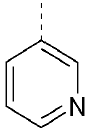 | CH | O | CH₂CHF₂ | H |
| 26 | 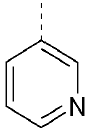 | CH | O | 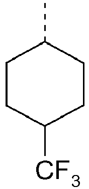 | H |
| 27 | 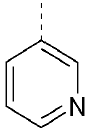 | CH | O | CH₂CH₂SCH₃ | H |
| 28 | 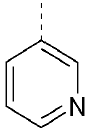 | CCH₃ | O | CH(CH₃)₂ | H |
| 29 | 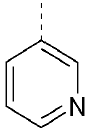 | CCH₃ | O | C₂H₅ | H |
| 30 | 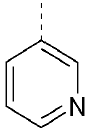 | CH | O | 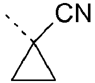 | H |
| 31 | 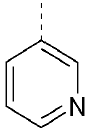 | CH | O | CHCH₃CH₂SCH₃ | H |
| 32 | 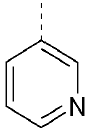 | CCF₃ | O | C₂H₅ | H |
| 33 | 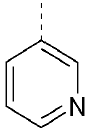 | CH | O | CH₂CH(CH₂)₂ | H |
| 34 | 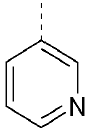 | CH | O | CH₂C(CH₃)₃ | H |
| 35 | 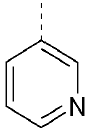 | CH | O | CH₂CH(CH₃)₂ | H |
| 36 | 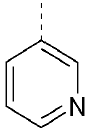 | CH | N | CH(CH₃)₂ | H |
| 37 | 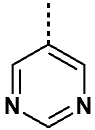 | CH | O | CH(CH₃)₂ | H |
| 38 | 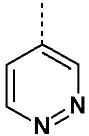 | CH | O | CH(CH₃)₂ | H |
| 39 | 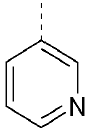 | CH | O | 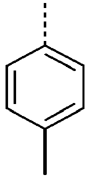 | H |

**Tabelle 3**

| Analytische Daten zu den angegebenen Verbindungen | | | |
|---|---|---|---|
| **Bsp.-Nr.** | **logP[a]** | **logP[b]** | **1H**-**NMR (D6-DMSO, 400 MHz) σ (ppm)** |
| 1 | 1,31 | | |
| 2 | 1,57 | | |
| 3 | 1,48 | | |
| 4 | 2,04 | | |
| 5 | 0,80 | 0,91 | |
| 6 | 0,98 | 1,15 | |
| 7 | 0,58 | 0,82 | 9,27(d,1H), 8,62(d,1H), 8,60(s,1H), 8,46-8,43(m,2H), 7,64(dd,1H), 3,53(s,3H) |
| 8 | 0,55 | 0,55 | |
| 9 | 0,80 | | |
| 10 | 1,40 | | |
| 11 | 0,63 | | |
| 12 | 1,27 | | |
| 13 | 1,59 | | |
| 14 | 1,74 | | |
| 15 | 1,06 | 1,12 | |
| 16 | 0,70 | 0,84 | |
| 17 | 1,07 | 1,12 | 9,47(s,1H), 9,26(d,1H), 8,65(dd,1H), 8,44-8,41(m,1H), 8,27(s,1H), 7,63(dd,1H), 4,15-4,07(m,2H), 3,92-3,86(m, 1H), 3,83-3,77(m,1H), 3,68-3,63(m,1H), 2,02-1,78(m,3H), 1,63-1,54(m,1H) |
| 18 | 0,45 | 0,52 | 9,48(s, 1H), 9,27(d,1H), 8,65(dd,1H), 8,43(dd,1H), 8,35(s,1H), 7,63(dd,1H), 3,46(s,3H) |
| 19 | 0,98 | 1,09 | 9,51(s, 1H), 9,27(d,1H), 8,66(dd,1H), 8,43(dd,1H), 8,35(s,1H), 7,64(dd,1H), 4,80(s,2H), 4,18(q,2H), 1,22(t,3H) |
| 20 | 0,76 | 1,06 | 9,45(s,1H), 9,26(d,1H), 8,66(dd,1H), 8,48-8,47(m,2H), 8,44-8,41(dd,1H), 7,80(dt,1H), 7,64(dd,1H), 7,40(bd,1H), 7,30(dd,1H), 5,29(s,2H) |
| 21 | 1,13 | 1,32 | 9,45(s,1H), 9,26(d,1H), 8,65(dd,1H), 8,53(bd,1H), 8,46(s,1H), 8,42(bd,1H), 7,82(dt,1H), 7,63(dd,1H), 7,49(d,1H), 7,32(dd,1H), 6,12(q,1H), 1,85(d,3H) |
| 22 | 0,47 | 0,51 | 9,49(s,1H), 9,27(d,1H), 8,65(dd,1H), 8,43(dt,1H), 8,19(s,1H), 7,64(dd,1H), 4,89(s,2H), 3,09(s,3H), 2,88(s,3H) |
| 23 | 0,79 | 0,83 | 9,47(s,1H), 9,26(d,1H), 8,64(dd,1H), 8,42(dt,1H), 8,27(s,1H), 7,63(dd,1H), 3,16(quint,1H), 1,06-0,90(m,4H) |
| 24 | 1,19 | 1,21 | 9,52(s,1H), 9,26(d,1H), 8,66(dd,1H), 8,44(dt,1H), 8,28(s,1H), 7,64(dd,1H), 4,53(t,2H), 1,71(t,3H) |
| 25 | 0,91 | 0,96 | 9,53(s,1H), 9,27(d,1H), 8,66(d,1H), 8,43(bd,1H), 8,35(s,1H), 7,64(dd,1H), 6,37(tt,1H), 4,47(dt,2H) |
| 26 | 2,08 | 2,06 | 9,50(s,1H), 9,27(d,1H), 8,65(bd,1H), 8,44-8,42(m,2H), 7,63(dd,1H), 4,70-4,63(m,1H), 3,81-3,75(m,1H), 2,04-1,90(m,6H), 1,56-1,46(m,2H) |
| 27 | 1,13 | 1,19 | 9,47(s,1H), 9,26(d,1H), 8,65(dd,1H), 8,43(dt,1H), 8,38(s,1H), 7,64(dd,1H), 4,14(t,2H), 2,83(t,2H), 2,12(s,3H) |
| 28 | 1,28 | 1,35 | 9,33(s,1H), 9,24(d,1H), 8,63(d,1H), 8,38(bd,1H), 7,60(dd,1H), 4,78-4,55(m,1H), 2,65(s,3H), 1,55(d,6H) |
| 29 | 0,93 | 1,03 | |
| 30 | 0,82 | 0,85 | |
| 31 | 1,36 | 1,40 | 9,45(s,1H), 9,26(d,1H), 8,65(dd,1H), 8,44-8,41(m,2H), 7,64(dd,1H), 5,05-4,94(m,1H), 3,07-2,94(m,2H), 2,04(s,3H), 1,50(d,3H) |
| 32 | 2,07 | 2,06 | |
| 33 | 1,23 | 1,27 | 9,48(s,1H), 9,27(d,1H), 8,65(dd,1H), 8,42-8,41(m,2H), 7,65-7,62(m,1H), 3,82(d,2H), 1,28-1,20(m,1H), 0,53-0,48(m,2H), 0,44-0,41(m,2H) |
| 34 | 1,70 | 1,71 | 9,46(s,1H), 9,26(d,1H), 8,65(dd,1H), 8,44-8,40(m,1H), 8,29(s,1H), 7,65-7,62(m,1H), 3,85(s,2H), 0,94(s,9H) |
| 35 | 1,42 | 1,44 | 9,47(s,1H), 9,26(d,1H), 8,65(dd,1H), 8,44-8,41(m,1H), |
| | | | 8,34(s,1H), 7,65-7,62(m,1H), 3,78(d,2H), 2,10-2,03(m,1H), 0,89(d,6H) |
| 36 | 1,72 | 1,70 | 9,71(s,1H), 9,33(d,1H), 8,74(dd,1H), 8,53-8,50(m,1H), 7,72-7,69(m,1H), 5,31(quint,1H), 1,49(d,6H) |
| 37 | 0,93 | 0,92 | 9,53(s,1H), 9,47(s,2H), 9,27(d,1H), 8,48(s,1H), 4,99(q,1H), 1,42(bd,6H) |
| 38 | 0,85 | 0,85 | |
| 39 | 1,73 | 1,73 | 9,56(s,1H), 9,29(d,1H), 8,67(dd,1H), 8,47-8,44(m,1H), 8,30(s,1H), 7,67-7,64(m,1H), 7,40-7,35(m,4H), 2,40(s,3H) |

### Biologische Beispiele

**Myzus persicae - Sprühtest**

| | |
|---|---|
| Lösungsmittel: | 78 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigten z. B. die folgenden Verbindungen der Herstellungsbeispiele Wirkung von 100% bei einer Aufwandmenge von 500 g/ha: 3, 9, 11, 12, 15, 16, 17, 18, 19, 23, 24, 25, 30, 33, 34, 37,39

Bei diesem Test zeigten z. B. die folgenden Verbindungen der Herstellungsbeispiele Wirkung von 90% bei einer Aufwandmenge von 500 g/ha: 2, 4, 5, 10, 14, 20, 21, 22, 26, 28, 29, 36, 38

Bei diesem Test zeigten z. B. die folgenden Verbindungen der Herstellungsbeispiele Wirkung von 100% bei einer Aufwandmenge von 100 g/ha: 35

**Myzus persicae - Sprühtest**

| | |
|---|---|
| Lösungsmittel: | 7 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Paprikapflanzen (*Capsicum annuum*), die stark von der Grünen Pfirsichblattlaus (*Myzus persicae*) befallen sind, werden durch Sprühen mit der Wirkstoffzubereitung in der gewünschten Konzentration behandelt.

Nach 6 Tagen wird die Abtötung in % bestimmt. Dabei bedeutet 100 %, dass alle Läuse abgetötet wurden; 0 % bedeutet, dass keine Läuse abgetötet wurden.

Bei diesem Test zeigten z. B. die folgenden Verbindungen der Herstellungsbeispiele Wirkung von 95% bei einer Aufwandmenge von 20 ppm: 31

Bei diesem Test zeigten z. B. die folgenden Verbindungen der Herstellungsbeispiele Wirkung von 90% bei einer Aufwandmenge von 20 ppm: 27

**Meloidogyne incognita- Test (MELGIN)**

| | |
|---|---|
| Lösungsmittel: | 125,0 Gewichtsteile Aceton |

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (*Meloidogyne incognita)* und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigte z. B. die folgende Verbindung der Herstellungsbeispiele Wirkung von 100% bei einer Aufwandmenge von 20 ppm : 14

## Patentansprüche

1. Nicht therapeutische Verwendung von Verbindungen der Formel (I) 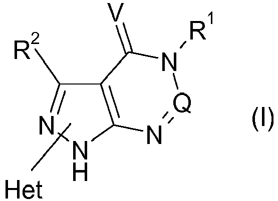 in welcher
Het für einen Rest aus der Reihe 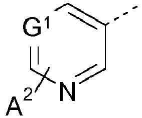 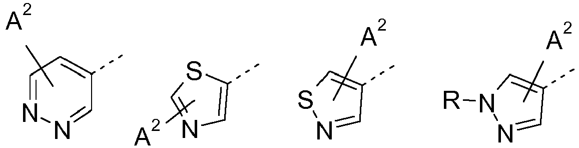 steht, worin die gestrichelte Linie die Bindung zu einem der Stickstoffatome im Pyrazolring darstellt und
G¹ für N oder C-A¹ steht,
A¹ für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
A² für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
R für Wasserstoff, Alkyl, Halogenalkyl oder gegebenenfalls substituiertes Cycloalkyl steht,
R¹ für einen Rest aus der Reihe Wasserstoff, Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylsulfanylalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, Dialkylaminosulfanylalkyl, Dialkylaminosulfinylalkyl, Dialkylaminosulfonylalkyl, gegebenenfalls durch Halogen substituiertes Alkoxycarbonylalkyl, gegebenenfalls durch Halogen substituiertes Alkinyloxy, gegebenenfalls durch Halogen substituiertes Alkinyloxycarbonyl, Dialkylaminocarbonyl, N-Alkyl-N-Cycloalkylaminocarbonyl, Dialkylaminocarbonylalkyl, N-Alkyl-N-Cycloalkylaminocarbonylalkyl, Heterocyclylcarbonylalkyl, Alkylsulfanyl, Halogenalkylsulfanyl, Alkylsulfinyl, Halogenalkylsulfinyl, Alkylsulfonyl, Halogenalkylsulfonyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls durch Halogen, Cyano, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylcarbonyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylalkyl, gegebenenfalls substituiertes Heterocyclyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Heterocyclylalkyl, gegebenenfalls durch Halogen, Cyano, Nitro, Alkyl, Halogenalkyl, Cycloalkyl (welches substituiert sein kann), Alkoxy oder Halogenalkoxy substituiertes Aryl, gegebenenfalls durch Halogen, Cyano, Alkyl, Halogenalkyl, Cycloalkyl (welches substituiert sein kann), Alkoxy oder Halogenalkoxy substituiertes Arylalkyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetaryl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetarylalkyl steht,
R² für Wasserstoff oder Alkyl steht,
Q für Stickstoff oder C-R³ steht, worin
R³ für einen Rest aus der Reihe Wasserstoff, Alkyl, Halogenalkyl, OH, Alkoxy, Halogenalkoxy, SH, Alkylsulfanyl, Alkylsulfinyl, Alkylsulfonyl, NH₂, Alkylamino und Dialkylamino steht,
V für einen Rest aus der Reihe Sauerstoff, Schwefel und NR⁴ steht und
R⁴ für einen Rest aus der Reihe Wasserstoff, Cyano, Alkyl, Halogenalkyl und Cycloalkyl steht,
zur Bekämpfung von tierischen Schädlingen.

2. Verbindungen der Formel (Ia) 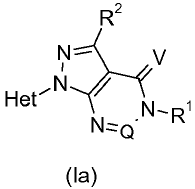 in welcher
Het für einen Rest aus der Reihe 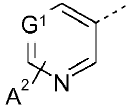 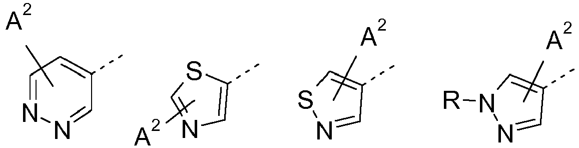 steht, worin die gestrichelte Linie die Bindung zum Stickstoffatom im Pyrazolring darstellt und
G¹ für N oder C-A¹ steht,
A¹ für Wasserstoff, Halogen, Cyano, Nitro, Alkyl, Halogenalkyl, Alkoxy, Halogenalkoxy oder gegebenenfalls substituiertes Cycloalkyl oder Cycloalkenyl steht,
A² für Wasserstoff, Halogen, Cyano, Nitro, Alkyl, Halogenalkyl, Alkoxy, Halogenalkoxy oder gegebenenfalls substituiertes Cycloalkyl oder Cycloalkenyl steht,
R für Wasserstoff, Alkyl, Halogenalkyl oder gegebenenfalls substituiertes Cycloalkyl steht,
R¹ für einen Rest aus der Reihe Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylthioalkyl, gegebenenfalls durch Halogen substituiertes Alkylcarbonylalkyl, gegebenenfalls durch Halogen substituiertes Alkylsulfinylalkyl, jeweils gegebenenfalls durch Halogen substituiertes Alkylsulfonylalkyl, Dialkylaminosulfanylalkyl, Dialkylaminosulfinylalkyl und Dialkylaminosulfonylalkyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylalkyl, gegebenenfalls substituiertes Heterocyclyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Heterocyclylalkyl, gegebenenfalls durch Halogen, Cyano, Nitro, Alkyl, Halogenalkyl, Cycloalkyl (welches substituiert sein kann), Alkoxy oder Halogenalkoxy substituiertes Aryl, gegebenenfalls durch Halogen, Cyano, Alkyl, Halogenalkyl, Cycloalkyl (welches substituiert sein kann), Alkoxy oder Halogenalkoxy substituiertes Arylalkyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetaryl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetarylalkyl steht,
R² für Wasserstoff oder Alkyl steht,
Q für Stickstoff oder C-R³ steht, worin
R³ für einen Rest aus der Reihe Wasserstoff, Alkyl, Halogenalkyl, OH, Alkoxy, Halogenalkoxy, SH, Alkylsulfanyl, Alkylsulfinyl, Alkylsulfonyl, NH₂, Alkylamino und Dialkylamino steht,
V für einen Rest aus der Reihe Sauerstoff, Schwefel und NR⁴ steht und
R⁴ für einen Rest aus der Reihe Wasserstoff, Cyano, Alkyl, Halogenalkyl und Cycloalkyl steht,
mit der Maßgabe, dass Verbindungen, in denen R¹ für 4-Chlorphenyl steht und gleichzeitig Q für C, R³ für Methyl, V für O, G¹ für N oder C-A¹ steht und A¹ und A² für H stehen, ausgenommen sind und dass weiterhin Verbindungen ausgenommen sind, in denen R¹ für 3-Chlorphenyl oder gegebenenfalls substituiertes Cycloalkyl steht und gleichzeitig Q für C-R³ steht, R³ für Wasserstoff steht und V für O steht.

3. Verbindungen der Formel (Ib) 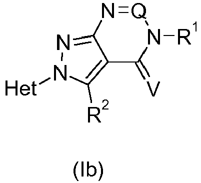 in welcher
Het für einen Rest aus der Reihe 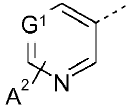 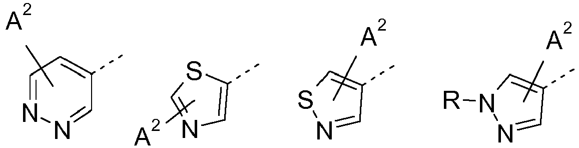 steht, worin die gestrichelte Linie die Bindung zum Stickstoffatom im Pyrazolring darstellt und
G¹ für N oder C-A¹ steht,
A¹ für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
A² für Wasserstoff, Halogen, Cyano, Nitro, Alkyl, Halogenalkyl, Alkoxy, Halogenalkoxy oder jeweils gegebenenfalls substituiertes Cycloalkyl oder Cycloalkenyl steht,
R für Wasserstoff, Alkyl, Halogenalkyl oder gegebenenfalls substituiertes Cycloalkyl steht,
R¹ für einen Rest aus der Reihe Alkyl, Halogenalkyl, Cyanoalkyl, Alkenyl, Halogenalkenyl, Alkinyl, Halogenalkinyl, Alkoxy, Halogenalkoxy, gegebenenfalls durch Halogen substituiertes Alkoxyalkyl, gegebenenfalls durch Halogen substituiertes Bis(alkoxy)alkyl, gegebenenfalls durch Halogen substituiertes Alkylthioalkyl, gegebenenfalls durch Halogen substituiertes Alkylcarbonylalkyl, gegebenenfalls durch Halogen substituiertes Alkylsulfinylalkyl, gegebenenfalls durch Halogen substituiertes Alkylsulfonylalkyl, Dialkylaminosulfanylalkyl, Dialkylaminosulfinylalkyl, Dialkylaminosulfonylalkyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkyl, gegebenenfalls durch Halogen, Cyano, Nitro, Alkyl, Halogenalkyl, Alkoxy, Halogenalkoxy, Alkoxycarbonyl, Halogenalkoxycarbonyl oder Hetaryl (welches gegebenenfalls selbst durch Alkyl oder Halogen substituiert ist) substituiertes Cycloalkylalkyl, gegebenenfalls substituiertes Heterocyclyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Heterocyclylalkyl, gegebenenfalls durch Halogen, Cyano, Nitro, Alkyl, Halogenalkyl, Cycloalkyl (welches substituiert sein kann), Alkoxy oder Halogenalkoxy substituiertes Aryl, gegebenenfalls durch Halogen, Cyano, Alkyl, Halogenalkyl, Cycloalkyl (welches substituiert sein kann), Alkoxy oder Halogenalkoxy substituiertes Arylalkyl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetaryl, gegebenenfalls durch Halogen, Cyano (auch im Alkylteil), Nitro, Alkyl, Halogenalkyl, Cycloalkyl (welches substituiert sein kann), Alkoxy, Halogenalkoxy, Alkylthio, Haloalkylthio, Alkylsulfinyl, Alkylsulfonyl, Haloalkylsulfinyl, Haloalkylsulfonyl, Amino, Alkylamino, Dialkylamino, Alkylcarbonylamino, Alkoxycarbonylamino, Alkoxyalkyl, Halogenalkoxyalkyl, Alkenyl, Alkinyl, Cycloalkylalkyl, Alkylcarbonyl, Alkoxycarbonyl oder Aminocarbonyl substituiertes Hetarylalkyl steht,
R² für Wasserstoff oder Alkyl steht,
Q für Stickstoff oder C-R³ steht, worin
R³ für einen Rest aus der Reihe Wasserstoff, Alkyl, Halogenalkyl, OH, Alkoxy, Halogenalkoxy, SH, Alkylsulfanyl, Alkylsulfinyl, Alkylsulfonyl, NH₂, Alkylamino und Dialkylamino steht,
V für einen Rest aus der Reihe Sauerstoff, Schwefel und NR⁴ steht und
R⁴ für einen Rest aus der Reihe Wasserstoff, Cyano, Alkyl, Halogenalkyl und Cycloalkyl steht,
mit der Maßgabe, dass Verbindungen, in denen R¹ für Phenyl oder 4-Chlorphenyl steht und gleichzeitig Q für C, R³ für Methyl, V für O, G¹ für N oder C-A¹ steht und A¹ und A² für H stehen, ausgenommen sind.

4. Mittel, **gekennzeichnet durch** einen Gehalt von mindestens einer Verbindung der Formel (I) gemäß Anspruch 1 und üblichen Streckmitteln und/oder oberflächenaktiven Substanzen.

5. Mittel, **gekennzeichnet durch** einen Gehalt von mindestens einer Verbindung der Formel (Ia) gemäß Anspruch 2 und üblichen Streckmitteln und/oder oberflächenaktiven Substanzen.

6. Mittel, **gekennzeichnet durch** einen Gehalt von mindestens einer Verbindung der Formel (Ib) gemäß Anspruch 3 und üblichen Streckmitteln und/oder oberflächenaktiven Substanzen.

7. Nicht therapeutische Verwendung von Verbindungen der Formel (Ia) gemäß Anspruch 2 oder von Mitteln gemäß Anspruch 5 zur Bekämpfung von Schädlingen.

8. Nicht therapeutische Verwendung von Verbindungen der Formel (Ib) gemäß Anspruch 3 oder von Mitteln gemäß Anspruch 6 zur Bekämpfung von Schädlingen.

## Claims

1. Non-therapeutic use of compounds of the formula (I) 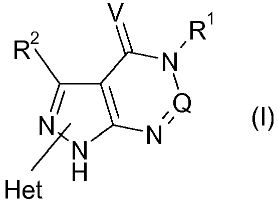 in which
Het is a radical from the group of 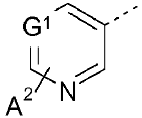 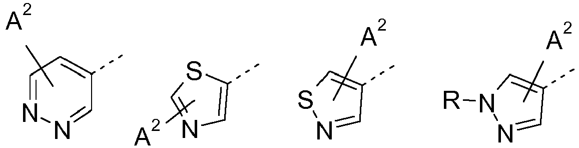 in which the dotted line represents the bond to one of the nitrogen atoms in the pyrazole ring and
G¹ is N or C-A¹,
A¹ is hydrogen, halogen, cyano, nitro, alkyl, haloalkyl, alkoxy, haloalkoxy or in each case optionally substituted cycloalkyl or cycloalkenyl,
A² is hydrogen, halogen, cyano, nitro, alkyl, haloalkyl, alkoxy, haloalkoxy or in each case optionally substituted cycloalkyl or cycloalkenyl,
R is hydrogen, alkyl, haloalkyl or optionally substituted cycloalkyl,
R¹ is a radical from the group of hydrogen, alkyl, haloalkyl, cyanoalkyl, alkenyl, haloalkenyl, alkynyl, haloalkynyl, alkoxy, haloalkoxy, optionally halogen-substituted alkoxyalkyl, optionally halogen-substituted bis(alkoxy)alkyl, optionally halogen-substituted alkylsulphanylalkyl, optionally halogen-substituted alkylcarbonylalkyl, optionally halogen-substituted alkylsulphinylalkyl, optionally halogen-substituted alkylsulphonylalkyl, dialkylaminosulphanylalkyl, dialkylaminosulphinylalkyl, dialkylaminosulphonylalkyl, optionally halogen-substituted alkoxycarbonylalkyl, optionally halogen-substituted alkynyloxy, optionally halogen-substituted alkynyloxycarbonyl, dialkylaminocarbonyl, N-alkyl-N-cycloalkylaminocarbonyl, dialkylaminocarbonylalkyl, N-alkyl-N-cycloalkylaminocarbonylalkyl, heterocyclylcarbonylalkyl, alkylsulphanyl, haloalkylsulphanyl, alkylsulphinyl, haloalkylsulphinyl, alkylsulphonyl, haloalkylsulphonyl, optionally halogen-, cyano-, nitro-, alkyl-, haloalkyl-, alkoxy-, haloalkoxy-, alkoxycarbonyl-, haloalkoxycarbonyl- or hetaryl-substituted (hetaryl itself optionally being substituted by alkyl or halogen) cycloalkyl, optionally halogen-, cyano-, alkyl-, haloalkyl-, alkoxy-, haloalkoxy-, alkoxycarbonyl-, haloalkoxycarbonyl- or hetaryl-substituted (hetaryl itself optionally being substituted by alkyl or halogen) cycloalkylcarbonyl, optionally halogen-, cyano-, nitro-, alkyl-, haloalkyl-, alkoxy-, haloalkoxy-, alkoxycarbonyl-, haloalkoxycarbonyl-or hetaryl-substituted (hetaryl itself optionally being substituted by alkyl or halogen) cycloalkylalkyl, optionally substituted heterocyclyl, optionally halogen-, cyano-(including in the alkyl moiety), nitro-, alkyl-, haloalkyl-, cycloalkyl- (which may be substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl-or aminocarbonyl-substituted heterocyclylalkyl, optionally halogen-, cyano-, nitro-, alkyl-, haloalkyl-, cycloalkyl- (which may be substituted), alkoxy- or haloalkoxy-substituted aryl, optionally halogen-, cyano-, alkyl-, haloalkyl-, cycloalkyl-(which may be substituted), alkoxy- or haloalkoxy-substituted arylalkyl, optionally halogen-, cyano-(including in the alkyl moiety), nitro-, alkyl-, haloalkyl-, cycloalkyl- (which may be substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl-or aminocarbonyl-substituted hetaryl, optionally halogen-, cyano- (including in the alkyl moiety), nitro-, alkyl-, haloalkyl-, cycloalkyl- (which may be substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl-or aminocarbonyl-substituted hetarylalkyl,
R² is hydrogen or alkyl,
Q is nitrogen or C-R³ in which
R³ is a radical from the group of hydrogen, alkyl, haloalkyl, OH, alkoxy, haloalkoxy, SH, alkylsulphanyl, alkylsulphinyl, alkylsulphonyl, NH₂, alkylamino and dialkylamino,
V is a radical from the group of oxygen, sulphur and NR⁴ and
R⁴ is a radical from the group of hydrogen, cyano, alkyl, haloalkyl and cycloalkyl, for controlling animal pests.

2. Compounds of the formula (Ia) 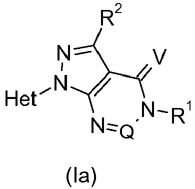 in which
Het is a radical from the group of 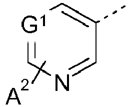 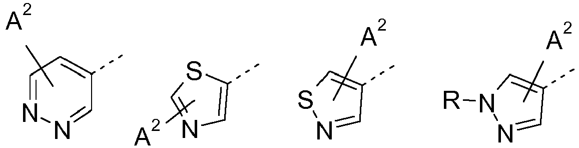 in which the dotted line represents the bond to the nitrogen atom in the pyrazole ring and
G¹ is N or C-A¹,
A¹ is hydrogen, halogen, cyano, nitro, alkyl, haloalkyl, alkoxy, haloalkoxy or optionally substituted cycloalkyl or cycloalkenyl,
A² is hydrogen, halogen, cyano, nitro, alkyl, haloalkyl, alkoxy, haloalkoxy or optionally substituted cycloalkyl or cycloalkenyl,
R is hydrogen, alkyl, haloalkyl or optionally substituted cycloalkyl,
R¹ is a radical from the group of alkyl, haloalkyl, cyanoalkyl, alkenyl, haloalkenyl, alkynyl, haloalkynyl, alkoxy, haloalkoxy, optionally halogen-substituted alkoxyalkyl, optionally halogen-substituted bis(alkoxy)alkyl, optionally halogen-substituted alkylthioalkyl, optionally halogen-substituted alkylcarbonylalkyl, optionally halogen-substituted alkylsulphinylalkyl, in each case optionally halogen-substituted alkylsulphonylalkyl, dialkylaminosulphanylalkyl, dialkylaminosulphinylalkyl and dialkylaminosulphonylalkyl, optionally halogen-, cyano-, nitro-, alkyl-, haloalkyl-, alkoxy-, haloalkoxy-, alkoxycarbonyl-, haloalkoxycarbonyl-or hetaryl-substituted (hetaryl itself optionally being substituted by alkyl or halogen) cycloalkyl, optionally halogen-, cyano-, nitro-, alkyl-, haloalkyl-, alkoxy-, haloalkoxy-, alkoxycarbonyl-, haloalkoxycarbonyl- or hetaryl-substituted (hetaryl itself optionally being substituted by alkyl or halogen) cycloalkylalkyl, optionally substituted heterocyclyl, optionally halogen-, cyano-(including in the alkyl moiety), nitro-, alkyl-, haloalkyl-, cycloalkyl- (which may be substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl-or aminocarbonyl-substituted heterocyclylalkyl, optionally halogen-, cyano-, nitro-, alkyl-, haloalkyl-, cycloalkyl- (which may be substituted), alkoxy- or haloalkoxy-substituted aryl, optionally halogen-, cyano-, alkyl-, haloalkyl-, cycloalkyl-(which may be substituted), alkoxy- or haloalkoxy-substituted arylalkyl, optionally halogen-, cyano-(including in the alkyl moiety), nitro-, alkyl-, haloalkyl-, cycloalkyl- (which may be substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl-or aminocarbonyl-substituted hetaryl, optionally halogen-, cyano- (including in the alkyl moiety), nitro-, alkyl-, haloalkyl-, cycloalkyl- (which may be substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl-or aminocarbonyl-substituted hetarylalkyl,
R² is hydrogen or alkyl,
Q is nitrogen or C-R³ in which
R³ is a radical from the group of hydrogen, alkyl, haloalkyl, OH, alkoxy, haloalkoxy, SH, alkylsulphanyl, alkylsulphinyl, alkylsulphonyl, NH₂, alkylamino and dialkylamino,
V is a radical from the group of oxygen, sulphur and NR⁴ and
R⁴ is a radical from the group of hydrogen, cyano, alkyl, haloalkyl and cycloalkyl,
with the proviso that compounds in which R¹ is 4-chlorophenyl and, at the same time, Q is C, R³ is methyl, V is O, G¹ is N or C-A¹ and A¹ and A² are each H are excluded, and that additionally excluded are compounds in which R¹ is 3-chlorophenyl or optionally substituted cycloalkyl and, at the same time, Q is C-R³, R³ is hydrogen and V is O.

3. Compounds of the formula (Ib) 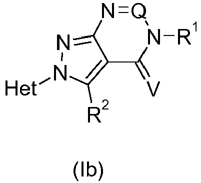 in which
Het is a radical from the group of 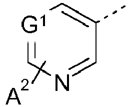 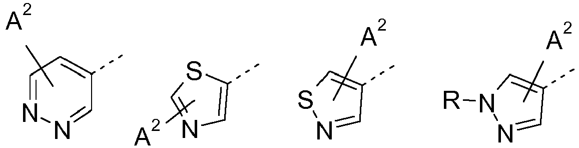 in which the dotted line represents the bond to the nitrogen atom in the pyrazole ring and
G¹ is N or C-A¹,
A¹ is hydrogen, halogen, cyano, nitro, alkyl, haloalkyl, alkoxy, haloalkoxy or in each case optionally substituted cycloalkyl or cycloalkenyl,
A² is hydrogen, halogen, cyano, nitro, alkyl, haloalkyl, alkoxy, haloalkoxy or in each case optionally substituted cycloalkyl or cycloalkenyl,
R is hydrogen, alkyl, haloalkyl or optionally substituted cycloalkyl,
R¹ is a radical from the group of alkyl, haloalkyl, cyanoalkyl, alkenyl, haloalkenyl, alkynyl, haloalkynyl, alkoxy, haloalkoxy, optionally halogen-substituted alkoxyalkyl, optionally halogen-substituted bis(alkoxy)alkyl, optionally halogen-substituted alkylthioalkyl, optionally halogen-substituted alkylcarbonylalkyl, optionally halogen-substituted alkylsulphinylalkyl, optionally halogen-substituted alkylsulphonylalkyl, dialkylaminosulphanylalkyl, dialkylaminosulphinylalkyl, dialkylaminosulphonylalkyl, optionally halogen-, cyano-, nitro-, alkyl-, haloalkyl-, alkoxy-, haloalkoxy-, alkoxycarbonyl-, haloalkoxycarbonyl-or hetaryl-substituted (hetaryl itself optionally being substituted by alkyl or halogen) cycloalkyl, optionally halogen-, cyano-, nitro-, alkyl-, haloalkyl-, alkoxy-, haloalkoxy-, alkoxycarbonyl-, haloalkoxycarbonyl- or hetaryl-substituted (hetaryl itself optionally being substituted by alkyl or halogen) cycloalkylalkyl, optionally substituted heterocyclyl, optionally halogen-, cyano-(including in the alkyl moiety), nitro-, alkyl-, haloalkyl-, cycloalkyl- (which may be substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl-or aminocarbonyl-substituted heterocyclylalkyl, optionally halogen-, cyano-, nitro-, alkyl-, haloalkyl-, cycloalkyl- (which may be substituted), alkoxy- or haloalkoxy-substituted aryl, optionally halogen-, cyano-, alkyl-, haloalkyl-, cycloalkyl-(which may be substituted), alkoxy- or haloalkoxy-substituted arylalkyl, optionally halogen-, cyano-(including in the alkyl moiety), nitro-, alkyl-, haloalkyl-, cycloalkyl- (which may be substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl-or aminocarbonyl-substituted hetaryl, optionally halogen-, cyano- (including in the alkyl moiety), nitro-, alkyl-, haloalkyl-, cycloalkyl- (which may be substituted), alkoxy-, haloalkoxy-, alkylthio-, haloalkylthio-, alkylsulphinyl-, alkylsulphonyl-, haloalkylsulphinyl-, haloalkylsulphonyl-, amino-, alkylamino-, dialkylamino-, alkylcarbonylamino-, alkoxycarbonylamino-, alkoxyalkyl-, haloalkoxyalkyl-, alkenyl-, alkynyl-, cycloalkylalkyl-, alkylcarbonyl-, alkoxycarbonyl-or aminocarbonyl-substituted hetarylalkyl,
R² is hydrogen or alkyl,
Q is nitrogen or C-R³ in which
R³ is a radical from the group of hydrogen, alkyl, haloalkyl, OH, alkoxy, haloalkoxy, SH, alkylsulphanyl, alkylsulphinyl, alkylsulphonyl, NH₂, alkylamino and dialkylamino,
V is a radical from the group of oxygen, sulphur and NR⁴ and
R⁴ is a radical from the group of hydrogen, cyano, alkyl, haloalkyl and cycloalkyl, with the proviso that compounds in which R¹ is phenyl or 4-chlorophenyl and, at the same time, Q is C, R³ is methyl, V is O, G¹ is N or C-A¹ and A¹ and A² are each H are excluded.

4. Composition, **characterized by** a content of at least one compound of the formula (I) according to Claim 1 and customary extenders and/or surfactants.

5. Composition, **characterized by** a content of at least one compound of the formula (Ia) according to Claim 2 and customary extenders and/or surfactants.

6. Composition, **characterized by** a content of at least one compound of the formula (Ib) according to Claim 3 and customary extenders and/or surfactants.

7. Non-therapeutic use of compounds of the formula (Ia) according to Claim 2 or of compositions according to Claim 5 for controlling pests.

8. Non-therapeutic use of compounds of the formula (Ib) according to Claim 3 or of compositions according to Claim 6 for controlling pests.

## Revendications

1. Utilisation non thérapeutique de composés de formule (I) 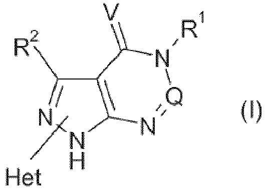 dans laquelle
Het représente un radical de la série 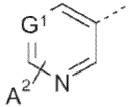 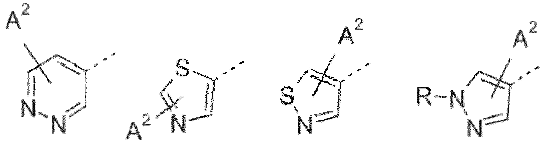 dans laquelle la ligne en pointillés représente la liaison vers un des atomes d'azote dans le cycle pyrazole, et
G¹ représente N ou C-A¹,
A¹ représente hydrogène, halogène, cyano, nitro, alkyle, halogénoalkyle, alcoxy, halogénoalcoxy, ou cycloalkyle ou cycloalcényle chacun éventuellement substitué,
A² représente hydrogène, halogène, cyano, nitro, alkyle, halogénoalkyle, alcoxy, halogénoalcoxy, ou cycloalkyle ou cycloalcényle chacun éventuellement substitué,
R représente hydrogène, alkyle, halogénoalkyle ou cycloalkyle éventuellement substitué,
R¹ représente un radical de la série constituée par hydrogène, alkyle, halogénoalkyle, cyanoalkyle, alcényle, halogénoalcényle, alcynyle, halogénoalcynyle, alcoxy, halogénoalcoxy, alcoxyalkyle éventuellement substitué par halogène, bis(alcoxy)alkyle éventuellement substitué par halogène, alkylsulfanylalkyle éventuellement substitué par halogène, alkylcarbonylalkyle éventuellement substitué par halogène, alkylsulfinylalkyle éventuellement substitué par halogène, alkylsulfonylalkyle éventuellement substitué par halogène, dialkylaminosulfanylalkyle, dialkylaminosulfinylalkyle, dialkylaminosulfonylalkyle, alcoxycarbonylalkyle éventuellement substitué par halogène, alcynyloxy éventuellement substitué par halogène, alcynyloxycarbonyle éventuellement substitué par halogène, dialkylaminocarbonyle, N-alkyl-N-cycloalkylaminocarbonyle, dialkylaminocarbonylalkyle, N-alkyl-N-cycloalkylaminocarbonylalkyle, hétérocyclylcarbonylalkyle, alkylsulfanyle, halogénoalkylsulfanyle, alkylsulfinyle, halogénoalkylsulfinyle, alkylsulfonyle, halogénoalkylsulfonyle, cycloalkyle éventuellement substitué par halogène, cyano, nitro, alkyle, halogénoalkyle, alcoxy, halogénoalcoxy, alcoxycarbonyle, halogénoalcoxycarbonyle ou hétaryle (qui est éventuellement lui-même substitué par alkyle ou halogène), cycloalkylcarbonyle éventuellement substitué par halogène, cyano, alkyle, halogénoalkyle, alcoxy, halogénoalcoxy, alcoxycarbonyle, halogénalcoxycarbonyle ou hétaryle (qui est éventuellement lui-même substitué par alkyle ou halogène), cycloalkylalkyle éventuellement substitué par halogène, cyano, nitro, alkyle, halogénoalkyle, alcoxy, halogénoalcoxy, alcoxycarbonyle, halogénoalcoxycarbonyle ou hétaryle (qui est éventuellement lui-même substitué par alkyle ou halogène), hétérocyclyle éventuellement substitué, hétérocyclylalkyle éventuellement substitué par halogène, cyano (également dans la partie alkyle), nitro, alkyle, halogénoalkyle, cycloalkyle (qui peut être substitué), alcoxy, halogénoalcoxy, alkylthio, haloalkylthio, alkylsulfinyle, alkylsulfonyle, haloalkylsulfinyle, haloalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle, aryle éventuellement substitué par halogène, cyano, nitro, alkyle, halogénoalkyle, cycloalkyle (qui peut être substitué), alcoxy ou halogénoalcoxy, arylalkyle éventuellement substitué par halogène, cyano, alkyle, halogénoalkyle, cycloalkyle (qui peut être substitué), alcoxy ou halogénoalcoxy, hétaryle éventuellement substitué par halogène, cyano (également dans la partie alkyle), nitro, alkyle, halogénoalkyle, cycloalkyle (qui peut être substitué), alcoxy, halogénoalcoxy, alkylthio, haloalkylthio, alkylsulfinyle, alkylsulfonyle, haloalkylsulfinyle, haloalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle, hétarylalkyle éventuellement substitué par halogène, cyano (également dans la partie alkyle), nitro, alkyle, halogénoalkyle, cycloalkyle (qui peut être substitué), alcoxy, halogénoalcoxy, alkylthio, haloalkylthio, alkylsulfinyle, alkylsulfonyle, haloalkylsulfinyle, haloalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle,
R² représente hydrogène ou alkyle,
Q représente azote ou C-R³,
R³ représentant un radical de la série constituée par hydrogène, alkyle, halogénoalkyle, OH, alcoxy, halogénoalcoxy, SH, alkylsulfanyle, alkylsulfinyle, alkylsulfonyle, NH₂, alkylamino et dialkylamino,
V représente un radical de la série constituée par oxygène, soufre et NR⁴, et
R⁴ représente un radical de la série constituée par hydrogène, cyano, alkyle, halogénoalkyle et cycloalkyle,
pour lutter contre des animaux nuisibles.

2. Composés de formule (Ia) 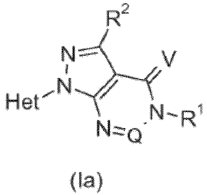 dans laquelle
Het représente un radical de la série 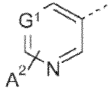 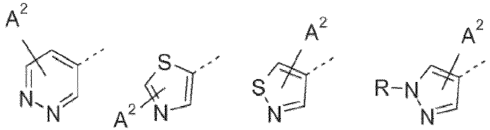 dans laquelle la ligne en pointillés représente la liaison vers l'atome d'azote dans le cycle pyrazole, et
G¹ représente N ou C-A¹,
A¹ représente hydrogène, halogène, cyano, nitro, alkyle, halogénoalkyle, alcoxy, halogénoalcoxy, ou cycloalkyle ou cycloalcényle éventuellement substitué,
A² représente hydrogène, halogène, cyano, nitro, alkyle, halogénoalkyle, alcoxy, halogénoalcoxy, ou cycloalkyle ou cycloalcényle éventuellement substitué,
R représente hydrogène, alkyle, halogénoalkyle ou cycloalkyle éventuellement substitué,
R¹ représente un radical de la série constituée par alkyle, halogénoalkyle, cyanoalkyle, alcényle, halogénoalcényle, alcynyle, halogénoalcynyle, alcoxy, halogénoalcoxy, alcoxyalkyle éventuellement substitué par halogène, bis(alcoxy)alkyle éventuellement substitué par halogène, alkylthioalkyle éventuellement substitué par halogène, alkylcarbonylalkyle éventuellement substitué par halogène, alkylsulfinylalkyle éventuellement substitué par halogène, alkylsulfonylalkyle éventuellement substitué par halogène, dialkylaminosulfanylalkyle, dialkylaminosulfinylalkyle, dialkylaminosulfonylalkyle, cycloalkyle éventuellement substitué par halogène, cyano, nitro, alkyle, halogénoalkyle, alcoxy, halogénoalcoxy, alcoxycarbonyle, halogénoalcoxycarbonyle ou hétaryle (qui est éventuellement lui-même substitué par alkyle ou halogène), cycloalkylalkyle éventuellement substitué par halogène, cyano, nitro, alkyle, halogénoalkyle, alcoxy, halogénoalcoxy, alcoxycarbonyle, halogénoalcoxycarbonyle ou hétaryle (qui est éventuellement lui-même substitué par alkyle ou halogène), hétérocyclyle éventuellement substitué, hétérocyclylalkyle éventuellement substitué par halogène, cyano (également dans la partie alkyle), nitro, alkyle, halogénoalkyle, cycloalkyle (qui peut être substitué), alcoxy, halogénoalcoxy, alkylthio, haloalkylthio, alkylsulfinyle, alkylsulfonyle, haloalkylsulfinyle, haloalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle, aryle éventuellement substitué par halogène, cyano, nitro, alkyle, halogénoalkyle, cycloalkyle (qui peut être substitué), alcoxy ou halogénoalcoxy, arylalkyle éventuellement substitué par halogène, cyano, alkyle, halogénoalkyle, cycloalkyle (qui peut être substitué), alcoxy ou halogénoalcoxy, hétaryle éventuellement substitué par halogène, cyano (également dans la partie alkyle), nitro, alkyle, halogénoalkyle, cycloalkyle (qui peut être substitué), alcoxy, halogénoalcoxy, alkylthio, haloalkylthio, alkylsulfinyle, alkylsulfonyle, haloalkylsulfinyle, haloalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle, hétarylalkyle éventuellement substitué par halogène, cyano (également dans la partie alkyle), nitro, alkyle, halogénoalkyle, cycloalkyle (qui peut être substitué), alcoxy, halogénoalcoxy, alkylthio, haloalkylthio, alkylsulfinyle, alkylsulfonyle, haloalkylsulfinyle, haloalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle,
R² représente hydrogène ou alkyle,
Q représente azote ou C-R³,
R³ représentant un radical de la série constituée par hydrogène, alkyle, halogénoalkyle, OH, alcoxy, halogénoalcoxy, SH, alkylsulfanyle, alkylsulfinyle, alkylsulfonyle, NH₂, alkylamino et dialkylamino,
V représente un radical de la série constituée par oxygène, soufre et NR⁴, et
R⁴ représente un radical de la série constituée par hydrogène, cyano, alkyle, halogénoalkyle et cycloalkyle,
à condition que les composés dans lesquels R¹ représente 4-chlorophényle et simultanément Q représente C, R³ représente méthyle, V représente O, G¹ représente N ou C-A¹, et A¹ et A² représentent H soient exclus, et que les composés dans lesquels R¹ représente 3-chlorophényle ou cycloalkyle éventuellement substitué et simultanément Q représente C-R³, R³ représente hydrogène et V représente O soient également exclus.

3. Composés de formule (Ib) 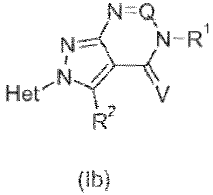 dans laquelle
Het représente un radical de la série 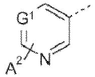 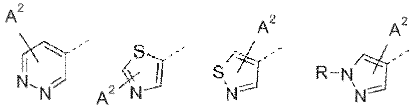 dans laquelle la ligne en pointillés représente la liaison vers l'atome d'azote dans le cycle pyrazole, et
G¹ représente N ou C-A¹,
A¹ représente hydrogène, halogène, cyano, nitro, alkyle, halogénoalkyle, alcoxy, halogénoalcoxy, ou cycloalkyle ou cycloalcényle chacun éventuellement substitué,
A² représente hydrogène, halogène, cyano, nitro, alkyle, halogénoalkyle, alcoxy, halogénoalcoxy, ou cycloalkyle ou cycloalcényle chacun éventuellement substitué,
R représente hydrogène, alkyle, halogénoalkyle ou cycloalkyle éventuellement substitué,
R¹ représente un radical de la série constituée par alkyle, halogénoalkyle, cyanoalkyle, alcényle, halogénoalcényle, alcynyle, halogénoalcynyle, alcoxy, halogénoalcoxy, alcoxyalkyle éventuellement substitué par halogène, bis(alcoxy)alkyle éventuellement substitué par halogène, alkylthioalkyle éventuellement substitué par halogène, alkylcarbonylalkyle éventuellement substitué par halogène, alkylsulfinylalkyle éventuellement substitué par halogène, alkylsulfonylalkyle éventuellement substitué par halogène, dialkylaminosulfanylalkyle, dialkylaminosulfinylalkyle, dialkylaminosulfonylalkyle, cycloalkyle éventuellement substitué par halogène, cyano, nitro, alkyle, halogénoalkyle, alcoxy, halogénoalcoxy, alcoxycarbonyle, halogénoalcoxycarbonyle ou hétaryle (qui est éventuellement lui-même substitué par alkyle ou halogène), cycloalkylalkyle éventuellement substitué par halogène, cyano, nitro, alkyle, halogénoalkyle, alcoxy, halogénoalcoxy, alcoxycarbonyle, halogénoalcoxycarbonyle ou hétaryle (qui est éventuellement lui-même substitué par alkyle ou halogène), hétérocyclyle éventuellement substitué, hétérocyclylalkyle éventuellement substitué par halogène, cyano (également dans la partie alkyle), nitro, alkyle, halogénoalkyle, cycloalkyle (qui peut être substitué), alcoxy, halogénoalcoxy, alkylthio, haloalkylthio, alkylsulfinyle, alkylsulfonyle, haloalkylsulfinyle, haloalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle, aryle éventuellement substitué par halogène, cyano, nitro, alkyle, halogénoalkyle, cycloalkyle (qui peut être substitué), alcoxy ou halogénoalcoxy, arylalkyle éventuellement substitué par halogène, cyano, alkyle, halogénoalkyle, cycloalkyle (qui peut être substitué), alcoxy ou halogénoalcoxy, hétaryle éventuellement substitué par halogène, cyano (également dans la partie alkyle), nitro, alkyle, halogénoalkyle, cycloalkyle (qui peut être substitué), alcoxy, halogénoalcoxy, alkylthio, haloalkylthio, alkylsulfinyle, alkylsulfonyle, haloalkylsulfinyle, haloalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle, hétarylalkyle éventuellement substitué par halogène, cyano (également dans la partie alkyle), nitro, alkyle, halogénoalkyle, cycloalkyle (qui peut être substitué), alcoxy, halogénoalcoxy, alkylthio, haloalkylthio, alkylsulfinyle, alkylsulfonyle, haloalkylsulfinyle, haloalkylsulfonyle, amino, alkylamino, dialkylamino, alkylcarbonylamino, alcoxycarbonylamino, alcoxyalkyle, halogénoalcoxyalkyle, alcényle, alcynyle, cycloalkylalkyle, alkylcarbonyle, alcoxycarbonyle ou aminocarbonyle,
R² représente hydrogène ou alkyle,
Q représente azote ou C-R³,
R³ représentant un radical de la série constituée par hydrogène, alkyle, halogénoalkyle, OH, alcoxy, halogénoalcoxy, SH, alkylsulfanyle, alkylsulfinyle, alkylsulfonyle, NH₂, alkylamino et dialkylamino,
V représente un radical de la série constituée par oxygène, soufre et NR⁴, et
R⁴ représente un radical de la série constituée par hydrogène, cyano, alkyle, halogénoalkyle et cycloalkyle,
à condition que les composés dans lesquels R¹ représente phényle ou 4-chlorophényle et simultanément Q représente C, R³ représente méthyle, V représente O, G¹ représente N ou C-A¹, et A¹ et A² représentent H soient exclus.

4. Agent, **caractérisé par** une teneur en au moins un composé de formule (I) selon la revendication 1 et des extendeurs et/ou substances tensioactives usuels.

5. Agent, **caractérisé par** une teneur en au moins un composé de formule (Ia) selon la revendication 2 et des extendeurs et/ou substances tensioactives usuels.

6. Agent, **caractérisé par** une teneur en au moins un composé de formule (Ib) selon la revendication 3 et des extendeurs et/ou substances tensioactives usuels.

7. Utilisation non thérapeutique de composés de formule (Ia) selon la revendication 2 ou d'agents selon la revendication 5 pour lutter contre des nuisibles.

8. Utilisation non thérapeutique de composés de formule (Ib) selon la revendication 3 ou d'agents selon la revendication 6 pour lutter contre des nuisibles.
